# EUROPEAN PATENT APPLICATION

(11) **EP 4 755 913 A1**
(43) Date of publication of application: **10.06.2026**
(21) Application number: 24848061.8
(22) Date of filing: 22.07.2024
(51) Int. Cl.: C07K 16/28, C07K 16/46, A61K 39/395, A61P 35/00

(54) **EGFR/C-MET BISPECIFIC ANTIBODY AND USE THEREOF**

(30) Priority: 02.08.2023 CN 202310966671
(71) Applicant: Abiotech Pharmaceutical Co., Ltd, Changzhi, Shanxi 046011 (CN)
(72) Inventor: LI, Xianzhong, Beijing 100176 (CN); GAN, Zhixue, Beijing 100176 (CN); ZHANG, Qingjun, Beijing 100176 (CN); HOU, Xiaoshuang, Beijing 100176 (CN); WANG, Jianan, Beijing 100176 (CN); GAO, Yonghong, Beijing 100176 (CN); WANG, Haijun, Beijing 100176 (CN); BAI, Xianhong, Beijing 100176 (CN)
(74) Representative: Elzaburu S.L.P.
(86) International application number: PCT/CN2024/106631
(87) International publication number: WO 2025/026102

(57) **Abstract**

A bispecific antibody targeting EGFR and c-Met. By means of modifying the amino acid sequence of an Fc segment of a binding arm, the heterodimeric interaction between an EGFR binding arm and a c-Met binding arm of the bispecific antibody is stronger than the interaction of the respective homodimers, such that a heterodimer can be assembled *in vitro.* The bispecific antibody is more effective in inhibiting the proliferation of EGFR and c-Met double positive tumor cells than a combination of an EGFR monoclonal antibody and a c-Met monoclonal antibody. The bispecific antibody mediates antibody-dependent cytotoxicity (ADCC), promotes target protein internalization, and inhibits the cell proliferation of the strain with acquired resistance to osimertinib. The prepared bispecific antibody is simple to prepare and stable in structure, a pure bispecific antibody is obtained by means of *in-vitro* assembly, and the bispecific antibody is expected to better meet clinical treatment needs, and therefore the bispecific antibody has good market prospects.

## Description

The present application claims priority of the patent application No. 202310966671.2 filed in China on August 2, 2023, the content of which is incorporated herein by reference in its entirety for all purposes.

### TECHNICAL FIELD

The present invention relates to an anti-EGFR/c-Met bispecific antibody or an antigen binding fragment thereof, and the use thereof as an anticancer drug.

### BACKGROUND

In the field of treating EGFR-positive tumors, TKI resistance remains an important issue affecting the efficacy of EGFR-targeted therapy. In recent years, bispecific antibodies targeting EGFR and c-Met have become an important solution direction for acquired resistance to EGFR-TKI, and have the potential for use in the first-line treatment of non-small cell lung cancer (NSCLC). Based on the crosstalk and direct interaction existing between EGFR and c-Met signaling pathways, the two anti-tumor mechanisms corresponding to the EGFR and c-Met targeting bispecific antibody can also interact to produce a synergistic effect, which can not only enhance the anti-tumor effect but also reduce the dosage of antibody, thereby lowering the risk for adverse reactions.

Currently, the first approved representative drug targeting the EGFR/c-MET target worldwide is Johnson & Johnson's bispecific antibody amivantamab (JNJ-372), which was approved in the United States in May, 2021 for the treatment of non-small cell lung cancer patients with EGFR exon 20 insertion mutations who have progressed during or after platinum-based chemotherapy, providing a new strategy for NSCLC treatment. According to the results of the CHRYSALIS Phase I study of amivantamab for progressed platinum-based chemotherapy-treated non-small cell lung cancer with EGFR exon 20 insertion mutations, the incidence of infusion-related reactions (IRRs) is relatively high. Besides amivantamab, pharmaceutical companies both domestic and international, such as Merus, Genor Biopharma, EpimAb Biotherapeutics, and Hansoh Pharma, are also developing EGFR/c-Met target antibodies. In general, the currently filed EGFR/c-Met bispecific antibodies focus on therapeutic efficacy against tumors (and/or TKI-resistant patients), while their safety profiles are not yet clear or require improvement.

### SUMMARY

The present invention provides EGFR/c-Met bispecific antibody molecules that combine the advantages of desired dual-target selectivity for EGFR and c-Met, potent anti-tumor efficacy, and a promising safety profile. They are capable of addressing the clinical need for treating TKI-resistant cancers while maintaining a balance between efficacy and safety.

The development of the EGFR/c-Met bispecific antibody of the present invention aims not only to treat patients who have developed resistance to existing therapies such as EGFR or c-Met monoclonal antibodies, but also to enhance tumor selectivity, improve therapeutic efficacy, and ensure favorable safety. Furthermore, it considers the manufacturability requirements for the assembly of the EGFR- and c-Met-binding arms. This antibody is expected to become a next-generation therapeutic that addresses the suboptimal safety profiles of currently available dual-targeting treatments against EGFR and c-Met, while also offering advantages in binding arm assembly.

EGFR and c-Met are co-expressed in many cancer types. Antibody molecules targeting these two targets ("bispecific antibody molecules") offer the prospect of broad clinical benefit across multiple cancer indications.

In the first aspect, the present invention provides a bispecific antibody comprising an antigen-binding arm A that specifically binds to human epidermal growth factor receptor (EGFR) and antagonizes the binding of epidermal growth factor (EGF) to EGFR, and an antigen-binding arm B that specifically binds to human proto-oncogene receptor tyrosine kinase (c-Met) and antagonizes the binding of hepatocyte growth factor (HGF) to c-Met; wherein the binding arm A comprises light chain A (LCA) and heavy chain A (HCA), and the binding arm B comprises light chain B (LCB) and heavy chain B (HCB).

In some embodiments, the LCA comprises the amino acid sequences shown in SEQ ID NO: 1.

In some embodiments, the HCA comprises the amino acid sequences shown in SEQ ID NO:2.

In some embodiments, the LCB comprises the amino acid sequences shown in SEQ ID NO:3.

In some embodiments, the HCB comprises the amino acid sequences shown in SEQ ID NO:4.

In some embodiments, the LCA, HCA, LCB and HCB comprise the amino acid sequences shown in SEQ ID NOs: 1, 2, 3 and 4, respectively.

In some embodiments, the binding arm A is a half-antibody structure of an IgG-type anti-EGFR antibody, and the binding arm B is a half-antibody structure of an IgG-type anti-c-Met antibody.

In the second aspect, the present invention provides a bispecific antibody, which comprises an antigen-binding arm A specifically targeting EGFR and an antigen-binding arm B specifically targeting c-Met, wherein the binding arm A comprises a light chain variable region A (VLA) and a heavy chain variable region A (VHA), and the binding arm B comprises a light chain variable region B (VLB) and a heavy chain variable region B (VHB), wherein the VHA and VLA of the binding arm A are derived from an IgG polypeptide A (IgG (A)) comprising a homodimeric structure of light chain variable region A (VLA) and heavy chain variable region A (VHA), and the VHB and VLB of the binding arm B are derived from an IgG polypeptide B (IgG(B)) comprising a homodimeric structure of light chain variable region B (VLB) and heavy chain variable region B (VHB).

In some embodiments, the VHA comprises VHA CDR1, VHA CDR2, and VHA CDR3 of the amino acid sequences shown in SEQ ID NOs: 19, 20, and 21, respectively.

In some embodiments, the VLA comprises VLA CDR1, VLA CDR2, and VLA CDR3 of the amino acid sequences shown in SEQ ID NOs: 22, 23, and 24, respectively.

In some embodiments, the VHB comprises VHB CDR1, VHB CDR2, and VHB CDR3 of the amino acid sequences shown in SEQ ID NOs: 25, 26, and 27, respectively.

In some embodiments, the VLB comprises VLB CDR1, VLB CDR2, and VLB CDR3 of the amino acid sequences shown in SEQ ID NOs: 28, 29, and 30, respectively.

In some embodiments, the VHA comprises comprise the amino acid sequences shown in SEQ ID NO:5.

In some embodiments, the VLA comprises comprise the amino acid sequences shown in SEQ ID NO:6.

In some embodiments, the VHB comprises comprise the amino acid sequences shown in SEQ ID NO:7.

In some embodiments, the VLB comprises comprise the amino acid sequences shown in SEQ ID NO:8.

In some embodiments, the VHA, VLA, VHB and VLB comprise the amino acid sequences shown in SEQ ID NOs: 5, 6, 7 and 8, respectively.

In some embodiments, the binding arm A further comprises a heavy chain constant region A (CHA) and a light chain constant region A (CLA), and the binding arm B further comprises a heavy chain constant region B (CHB) and a light chain constant region B (CLB), wherein the CHA and the CHB are both IgG1 isotype heavy chain constant regions.

In some embodiments, the CHA comprises an Fc(A) region, the Fc(A) region comprises a CH3(A) region; the CHB comprises an Fc(B) region, the Fc(B) region comprises a CH3(B) region.

In some embodiments, the heterodimeric interaction between the CH3(A) region and the CH3(B) region is stronger than each of the homodimeric interaction of the CH3(A) region and the CH3(B) region.

In some embodiments, the binding arm A further comprises a heavy chain constant region A (CHA) and a light chain constant region A (CLA), and the binding arm B further comprises a heavy chain constant region B (CHB) and a light chain constant region B (CLB), wherein the CHA and the CHB are both IgG1 isotype heavy chain constant regions.

In some embodiments, the CH3(A) of antigen binding arm A and the CH3(B) of antigen binding arm B both comprise amino acid mutations that enhance the interaction that promotes the formation of heterodimer.

In some embodiments, the CH3(A) comprises at least one amino acid substitution, the CH3(B) comprises at least one amino acid substitution, and when numbering the residues according to the EU scheme, the substitution in CH3(A) and the substitution in CH3(B) occur at different positions of amino acid residue.

In some embodiments, the CH3(A) comprises an amino acid mutation as shown in F405L, and the CH3 (B) comprises an amino acid mutation as shown in K409R.

In some embodiments, both the Fc(A) and the Fc(B) regions comprise amino acid mutations that reduce immunogenicity.

In some embodiments, both the Fc (A) and Fc(B) regions comprise a combination of amino acid site mutations consisting of K214R, D356E and L358M.

In some embodiments, the CHA, CLA, CHB and CLB comprise the amino acid sequences as shown SEQ ID NOs: 11, 12, 13 and 14, respectively.

In the third aspect, the present invention provides a composition of encoding nucleic acid comprising polynucleotide sequences encoding the HCA, HCB, LCA, and LCB of the bispecific antibody according to the aforementioned aspects, as shown in SEQ ID NOs: 15, 16, 17 and 18 respectively.

In the fourth aspect, the present invention provides a preparation method for the aforementioned anti-EGFR/c-Met bispecific antibody based on Fab exchange technology.

In the fifth aspect, the present invention provides a pharmaceutical composition comprising the bispecific antibody or antigen-binding fragment thereof of the aforementioned aspects, and a pharmaceutically acceptable carrier or diluent.

In the sixth aspect, the present invention provides the bispecific antibody molecule specifically binding to EGFR and c-Met as defined herein in its entirety, or an antigen-binding fragment thereof, and a pharmaceutical composition comprising the bispecific antibody molecule or antigen-binding fragment thereof, for use in the manufacture of a medicament for a method of treating a disease, such as treating cancer, in the human or animal body.

In the seventh aspect, the present invention provides a method of treating cancer, comprising administering the antibody molecule or pharmaceutical composition as defined in the invention.

In some embodiments, the cancer is selected frompancreatic cancer, colorectal cancer, non-small cell lung cancer (NSCLC), and squamous head and neck cancer (SQHN). In some exemplary embodiments, the cancer to be treated is non-small cell lung cancer (NSCLC).

The present disclosure comprises combinations of the described aspects and preferred features, unless such combination is clearly impermissible or expressly avoided.

It should be understood that, within the scope of the present invention, the various technical features described above in the present invention and those specifically described below (e.g., in the Examples) can be combined with each other to form new or preferred technical solutions. Due to space limitations, they are not listed here.

The EGFR/c-Met bispecific antibody of the present disclosure has the following functions:
(1) It can specifically bind to human c-Met protein and EGFR protein, and the binding of binding arm B to c-Met is significantly stronger than that of binding arm A to EGFR, with a difference in binding activity exceeding 20-fold between the two arms;
(2) It can bind to CHOK1-EGFR cells overexpressing human EGFR and CHOK1-HGFR cells overexpressing human c-Met respectively, and the binding activity of the binding arm B to CHOK1-HGFR cells is significantly higher than that of the binding arm A to CHOK1-EGFR cells;
(3) It can specifically bind to double-positive tumor cells;
(4) It can antagonize the binding of the ligand EGF to the target protein human EGFR and the binding of the ligand HGF to the target protein human c-Met respectively;
(5) It has affinity for Fc receptor proteins at a level comparable to that of amivantamab;
(6) It can inhibit the autophosphorylation of EGFR mediated by the ligand EGF and the autophosphorylation of c-Met mediated by the ligand HGF respectively;
(7) It can mediate the internalization and degradation of EGFR and c-Met target proteins respectively, and the mediated dual-target internalization has a synergistic effect;
(8) It mediates antibody-dependent cellular cytotoxicity (ADCC) against CHOK1-EGFR target cells, CHOK1-cMet target cells, and EGFR and c-Met double-positive tumor cells respectively, and the ADCC mediated against double-positive tumor cells primarily depends on the EGFR antibody arm;
(9) It can inhibit the proliferation of various human tumor cells *in vitro;* its inhibitory efficacy on tumor cell proliferation *in vitro* is stronger than the combination of anti-EGFR monoclonal antibody and anti-c-Met monoclonal antibody, having a synergistic effect;
(10) It can inhibit the proliferation of tumor cells with acquired resistance to the third-generation TKIs (e.g., osimertinib) *in vitro;*
(11) It can inhibit tumor growth *in vivo* in a mouse xenograft model of HGF autocrine human tumor cells.

The positive and progressive effects of the anti-EGFR/c-Met bispecific antibody of the present invention are as follows: based on its selective and specific binding to EGFR and c-Met double-positive tumor cells, its inhibitory efficacy on human tumor cell proliferation *in vitro* is stronger than the combination of anti-EGFR monoclonal antibody and anti-c-Met monoclonal antibody, having a synergistic effect; its functional characteristic of inhibiting HGF autocrine human tumor growth *in vivo* is expected to provide a new option for tumor immunotargeted therapy suitable for tumor patients with acquired resistance to Osimertinib, and is suitable for large-scale production in the market of tumor treatment.

### BRIEF DESCRIPTION OF THE DRAWINGS

Examples and experiments illustrating the principles of the present disclosure can be discussed with reference to the accompanying drawings, wherein:
FIG. 1 ELISA test of the dose-dependent binding curves of BT461-DB targeting binding to cMet or EGFR protein. A, ELISA test of the dose-dependent binding curve of the anti-EGFR/cMet antibody to human cMet protein. B, ELISA test of the dose-dependent binding curve of the anti-EGFR/cMet antibody to human EGFR protein. The x-axis represents antibody concentration (logarithmic value), and the y-axis represents the binding signal intensity of the antibody.
FIG. 2 FACS test of the dose-dependent binding curves of BT461-DB targeting binding to cell surface EGFR and cMet. A, FACS test of the dose-dependent binding curve of the anti-EGFR/cMet antibody to CHO-K1 cells overexpressing human c-Met. B, FACS test of the dose-dependent binding curve of the anti-EGFR/cMet antibody to CHO-K1 cells overexpressing human EGFR. The x-axis represents antibody concentration (logarithmic value), and the y-axis represents the binding signal intensity of the antibody.
FIG. 3 FACS test of the antibody concentration gradient binding curve of the anti-EGFR/cMet antibody to EGFR, HGFR double-positive MKN45 cells. Left, binding curve; right, EC50 of the binding curve. The x-axis represents antibody concentration (logarithmic value), and the y-axis represents the binding signal intensity of the antibody.
FIG. 4 ELISA test of the dose-dependent binding curves for BT461-DB blocking the binding activity of ligands HGF and EGF to HGFR and EGFR target proteins. A, ELISA test of the dose-dependent binding curve of the anti-EGFR/cMet antibody blocking HGF binding to human HGFR protein. B, ELISA test of the dose-dependent binding curve of the anti-EGFR/cMet antibody blocking EGF binding to human EGFR protein. The x-axis represents antibody concentration (logarithmic value), and the y-axis represents the binding signal intensity of the ligand HGF.
FIG. 5 Binding and dissociation curves of BT461-DB. A, Binding and dissociation curve of BT461-KIH with EGFR-HIS protein. B, Binding and dissociation curve of BT461-DB with EGFR-HIS protein. C, Binding and dissociation curve of BT461-KIH with HGFR-HIS protein. D, Binding and dissociation curve of BT461-DB with HGFR-HIS protein.
FIG. 6 Western-blot detection of BT461-DB inhibition of autophosphorylation of target proteins EGFR and c-Met.
FIG. 7 Detection of cellular internalization levels induced by the BT461-DB antibody. Left, after treatment with gradient concentrations of antibody, FACS test of the internalization of the antibody of the present invention. Right, EC50 and TOP (maximum internalization level) of the gradient concentration antibody - internalization level curve. The x-axis represents antibody concentration (logarithmic value), and the y-axis represents the internalization level of the fluorescently labeled antibody.
FIG. 8 Test results of ADCC level mediated by BT461-DB against target cells evaluated by ADCC reporter system. A, *In vitro* ADCC effect (antibody-dependent) against CHOK1 cells overexpressing EGFR. B, *In vitro* ADCC effect (antibody-dependent) against CHOK1 cells overexpressing cMet
FIG. 9 Test results of ADCC level mediated by BT461-DB against HCC827 tumor cells evaluated by ADCC reporter system. Left, ADCC effect level curve. B, EC50 and TOP value (maximum ADCC effect level) of the curve.
FIG. 10 Antibody BT461-DB inhibits the proliferation of tumor cell line HCC827-HGF *in vitro. A,* The *in vitro* efficacy of BT461-DB is comparable to that of BT461-KIH. B, The *in vitro* efficacy of BT461-DB shows a synergistic effect.
FIG. 11 Experimental results of resistance verification for cells with acquired resistance to osimertinib.
FIG. 12 Proliferation curves of antibody BT461-DB inhibiting cells with acquired resistance to osimertinib *in vitro.* A, BT461-DB significantly inhibits the proliferation of cells with acquired resistance to osimertinib. B, The *in vitro* efficacy of BT461-DB against resistant cells shows a synergistic effect.
FIG. 13 Experimental results of antibody BT461-DB inhibiting the growth of HCC827-HGF xenograft tumors. A, Tumor volume growth curve. B, Statistical table of tumor inhibition rate.
FIG. 14 PEE12.4 plasmid.
FIG. 15 PEE6.4 plasmid.

### DETAILED EMBODIMENTS

Through inventive efforts, the researchers of the present invention have obtained an anti-EGFR/c-Met bispecific antibody that can solve the TKI resistance problem in the field of EGFR-positive cancer treatment and achieve high-purity assembly. Compared with the combination of EGFR monoclonal antibody and c-Met monoclonal antibody, the bispecific antibody of the present invention has significantly higher affinity for c-Met than for EGFR binding, can antagonize the binding of ligands HGF and EGF to target proteins EGFR and c-Met; can significantly inhibit intracellular p-c-Met (Y1234/1235) phosphorylation levels while slightly inhibiting p-EGFR (Y1173) phosphorylation levels, thereby inhibiting the c-Met and EGFR signaling pathways; the Fc has relatively high affinity for receptor proteins, can mediate ADCC against EGFR-positive cells, c-Met-positive cells, and EGFR and c-Met double-positive tumor cells respectively, promotes EGFR and c-Met target protein internalization, and the internalization of EGFR and c-Met dual targets has a synergistic effect; can inhibit proliferation of HGFR-positive cell, and the degree of inhibition is stronger than the combination of anti-EGFR monoclonal antibody and anti-c-Met monoclonal antibody, showing synergistic effect.

Unless otherwise defined, scientific and technical terms related to the present invention shall have the meanings commonly understood by one of ordinary skill in the art. Furthermore, unless the context requires otherwise, singular terms shall include the plural and plural terms shall include the singular. Generally, the nomenclature and techniques used in connection with cell and tissue culture, molecular biology, and protein and oligonucleotide or polynucleotide chemistry and hybridization described herein are those well known and commonly used in the art. Standard techniques are used for recombinant DNA, oligonucleotide synthesis, and tissue culture and transformation (e.g., electroporation, transient transfection). Enzymatic reactions and purification techniques are performed according to the manufacturer's instructions, as commonly accomplished in the art, or as described herein. The aforementioned techniques and procedures are generally performed according to conventional methods well known in the art and as described in various general and more specific references that are cited and discussed throughout this specification. The nomenclature used in connection with, and the laboratory procedures and techniques described herein for, analytical chemistry, synthetic organic chemistry, and medicinal and pharmaceutical chemistry are those well known and commonly used in the art. Standard techniques are used for chemical syntheses, chemical analyses, pharmaceutical preparation, formulation, delivery, and treatment of patients.

Aspects and examples of the present disclosure will be discussed below with reference to the accompanying drawings. Other aspects and disclosures will be apparent to those skilled in the art. All documents mentioned herein are incorporated by reference.

### Epidermal growth factor receptor (EGFR)

Human EGFR (also known as protooncogene c-ErbB-1) is a transmembrane protein that is a receptor for members of the epidermal growth factor family (EGF family). Binding of EGFR to ligand induces receptor dimerization and autophosphorylation of several tyrosine residues (Y992, Y1045, Y1068, Y1148, and Y1173) in the C-terminal regulatory region of EGFR.

Abnormal EGFR expression activity has been linked to many diseases, including neurological disorders and many cancers.

In this specification, "EGFR" may refer to EGFR from any species and includes EGFR isoforms, fragments, variants or homologues from any species.

In an exemplary embodiment, the EGFR comprises the amino acid sequence shown in SEQ ID NO:32.

### Tyrosine protein kinase (c-Met)

Human c-Met, also known as receptor tyrosine kinase, or hepatocyte growth factor receptor (HGFR), binding of which to its ligand HGF induces autophosphorylation of several tyrosine residues (Y1234/Y1235) on the intracellular domain of c-Met, providing docking sites for downstream signaling molecules and activating multiple signaling cascades. Overexpression of c-Met has been observed in many human tumors and is often associated with metastatic phenotype and poor prognosis. Examples of cancers in which high levels of c-Met expression have been observed include non-small cell lung cancer, pancreatic cancer, colorectal cancer, head and neck squamous cell carcinoma, breast cancer, and esophageal gastric cancer. Co-expression of EGFR and c-Met is frequently observed in these cancers.

In this specification, "c-Met" can be c-Met from any species and includes c-Met isoforms, fragments, variants or homologues from any species.

In an exemplary embodiment, the HGFR comprises the amino acid sequence shown in SEQ ID NO:31.

As used herein, the term "Fab arm" refers to a heavy chain-light chain pair. The variable regions of the heavy and light chains of immunoglobulin molecules comprise binding domains that interact with antigens. The constant region of an antibody (Ab) mediates immunoglobulin binding to host tissues or factors, including various cells of the immune system (such as effector cells) and components of the complement system such as Clq, the first component of the classical pathway of complement activation.

A "full-length antibody" as used herein refers to an antibody that comprises all heavy and light chain constant domains and variable domains normally found in antibodies of that isotype.

As used herein, "isotype" refers to an immunoglobulin class encoded by heavy chain constant region genes (e.g., IgG1, IgG2, IgG, or IgG4).

As used herein, the term "Fc region" refers to an antibody region comprising at least a hinge region, a CH2 domain, and a CH3 domain.

The term "heterodimer interaction between CH3(A) region and CH3(B) region" refers to the interaction between CH3(A) region and CH3(B) region in CH3(A)/CH3(B) heterodimer proteins.

The term "tumor cell protein" refers to a protein located on the cell surface of tumor cells. In the present invention, tumor cell proteins are such as EGFR and c-Met.

As used herein, the term "effector cell" refers to an immune cell involved in the effector phase of an immune response as opposed to the recognition phase and activation phase of the immune response. Exemplary immune cells include cells of bone marrow or lymphoid origin, such as lymphocytes (such as B cells and T cells, including cytolytic T cells (CTLs)), killer cells, natural killer cells, macrophages, monocytes, eosinophils, polymorphonuclear cells such as neutrophils, granulocytes, mast cells, and basophils. Some effector cells express specific Fc receptors and perform specific immune functions.

In some embodiments, effector cells are capable of inducing antibody-dependent cytotoxicity (ADCC), such as natural killer cells, capable of inducing ADCC.

In some embodiments, the effector cell can phagocytize the target antigen or target cell.

### Composition of matter

The present invention provides bispecific antibodies that specifically bind to EGFR and c-Met, polynucleotides encoding bispecific antibodies of the present invention, vectors, host cells, and methods of preparing and using the same.

"Antigen-binding arm" describes the molecular portion of a bispecific antibody that binds to all or part of the corresponding antigen.

The bispecific antibody specifically binding EGFR and c-Met of the present invention comprises an antigen binding arm A specifically binding EGFR and an antigen binding arm B specifically binding c-Met, the affinity of the bispecific antibody for c-Met is significantly higher than that for EGFR, and the affinity difference is greater than or equal to 20 times.

The combinations of amino acid sequence numbers of fragments comprised in binding arm A and binding arm B of exemplary embodiments of the bispecific antibody of the present invention are shown in Table 1, the light chain, heavy chain, variable region and CDR sequences of EGFR-specific binding arm A are shown in Table 2 and Table 3 of Example 1, respectively, and the amino acid sequences of light chain, heavy chain, variable region and CDR sequences of c-Met-specific binding arm B are shown in Table 4 and Table 5 of Example 1, respectively.

### EGFR-specific binding arm A

Binding arm A of the bispecific antibody of the present invention binds to EGFR with high affinity and inhibits EGFR signaling and may provide beneficial effects in terms of specificity and reduced EGFR receptor phosphorylation levels compared to small molecule EGFR inhibitors and anti-EGFR monoclonal antibodies. Binding arm A of the present invention is monovalent, thus preventing the desired receptor clustering and activation that other divalent molecules may be present.

In some embodiments, binding arm A of the bispecific antibody of the present invention that specifically binds to EGFR comprises light chain A(LCA) and heavy chain A(HCA).

In some embodiments, the LCA of binding arm A preferably comprises the amino acid sequence as shown in SEQ ID NO: 1.

In some embodiments, the HCA of binding arm A preferably comprises the amino acid sequence as shown in SEQ ID NO:2.

In some embodiments, the LCA and HCA of binding arm A preferably comprise amino acid sequences as shown in SEQ ID NO:1 and SEQ ID NO:2, respectively.

In some embodiments, the LCA and HCA are derived from IgG polypeptide A (IgG (A)) having a homodimeric structure consisting of light chain A (LCA) and heavy chain A(HCA).

In some embodiments, the LCA and HCA are derived from IgG polypeptide A (IgG (A)) having a homodimeric structure consisting of light chain A (LCA) and heavy chain A(HCA) as shown in SEQ ID NO:1 and SEQ ID NO:2, respectively.

In some embodiments, the binding arm A comprises a light chain variable region A (VLA) and a heavy chain variable region A (VHA); wherein the binding arm A is derived from an IgG polypeptide A (IgG (A)) having a homodimeric structure consisting of a light chain variable region A (VLA) and a heavy chain variable region A (VHA).

In some embodiments, the binding arm A is a half antibody of a homodimeric IgG antibody.

In some embodiments, the VLA and the VHA comprise VHA CDRs and VLA CDRs, respectively, as in Table 3 in Example 1.

In some embodiments, the binding arm A that specifically binds to EGFR has VHA CDR1 of the amino acid sequence as shown in SEQ ID NO:19.

In some embodiments, the binding arm A that specifically binds to EGFR has VHA CDR2 of the amino acid sequence as shown in SEQ ID NO:20.

In some embodiments, the binding arm A that specifically binds to EGFR has VHA CDR 3 of the amino acid sequence as shown in SEQ ID NO:21.

In some embodiments, the binding arm A that specifically binds to EGFR has VLA CDR1 of the amino acid sequence as shown in SEQ ID NO:22.

In some embodiments, the binding arm A that specifically binds to EGFR has VLA CDR2 of the amino acid sequence as shown in SEQ ID NO:23.

In some embodiments, the binding arm A that specifically binds to EGFR has VLA CDR3 of the amino acid sequence as shown in SEQ ID NO:24.

The amino acid sequence of the binding arm A of the bispecific antibody embodiment of the present invention and the amino acid sequence of the parent antibody are shown in Example 1.

In some embodiments, the heavy chain variable region A(VHA) preferably comprises VHA CDR1, VHA CDR2, and VHA CDR3 of the amino acid sequences as shown in SEQ ID NOs:19, 20, and 21, respectively, and the light chain variable region A(VLA) preferably comprises VLA CDR1, VLA CDR2, and VLA CDR3 of the amino acid sequences as shown in SEQ ID NOs:22, 23, and 24, respectively.

In some embodiments, the heavy chain variable region A (VHA) comprises the amino acid sequence as shown in SEQ ID NO:5.

In some embodiments, the light chain variable region A (VLA) comprises the amino acid sequence as shown in SEQ ID NO:6.

Wherein, the VHA and VLA comprise amino acid sequences shown in SEQ ID NOs:5 and 6, respectively.

In some embodiments, the binding arm A is derived from a homodimeric IgG polypeptide A (IgG (A)) consisting of a VHA comprising the amino acid sequence of SEQ ID NO:5 and a VLA comprising the amino acid sequence of SEQ ID NO:6.

In some embodiments, the binding arm A further comprises heavy chain constant region A (CHA) and light chain constant region A (CLA).

In some embodiments, the CHA is an isotype of IgG1, IgG2, IgG3, or IgG4.

In some embodiments, the CHA is an IgG1 isotype heavy chain constant region.

In some embodiments, the antigen binding arm A comprises an Fc(A) region comprising a CH3(A) region.

In some embodiments, the CH3(A) region comprises amino acid mutations that reduce heavy chain mismatch rates.

In some embodiments, the CH3(A) region comprises an amino acid mutation as shown in F405L.

In some embodiments, the Fc(A) region comprises subtype reversion mutations that reduce immunogenicity.

In some embodiments, the Fc(A) region comprises amino acid mutations, e.g., as shown in K214R, D356E and L358M.

In some embodiments, the Fc(A) comprises the amino acid sequence as shown in SEQ ID NO:9.

In some embodiments, the CHA comprises the amino acid sequence as shown in SEQ ID NO:11.

In some embodiments, the LCA is a κ type or λ type light chain.

In some embodiments, the LCA is a κ type light chain.

In some embodiments, the CLA comprises the amino acid sequence as shown in SEQ ID NO: 12.

### c-Met-specific binding arm B

The binding arm B of the bispecific antibody of the present invention binds to c-Met with high affinity and inhibits c-Met signaling and may provide beneficial effects in terms of specificity and reduced c-Met receptor phosphorylation levels compared to small molecule c-Met inhibitors and anti-c-Met monoclonal antibodies. The binding arm B of the present invention is monovalent, thus preventing the desired receptor clustering and activation that other divalent molecules may be present.

In some embodiments, binding arm B of the bispecific antibody of the present invention that specifically binds to c-Met comprises light chain B (LCB) and heavy chain B (HCB).

In some embodiments, the LCB of binding arm A preferably comprises the amino acid sequence as shown in SEQ ID NO:3.

In some embodiments, the HCB of binding arm A preferably comprises the amino acid sequence as shown in SEQ ID NO:4.

In some embodiments, the light chain B (LCB) and heavy chain B (HCB) comprise amino acid sequences as shown in SEQ ID NO:3 and SEQ ID NO:4, respectively.

In some embodiments, the binding arm B is derived from IgG polypeptide B (IgG (B)) comprising a homodimeric structure consisting of light chain B (LCB) and heavy chain B (HCB).

In some embodiments, the binding arm B is a half-antibody of a light chain B (LCB) and heavy chain B (HCB) homodimeric IgG antibody comprising the amino acid sequences as shown in SEQ ID NO:3 and SEQ ID NO:4, respectively.

In some embodiments, the binding arm B comprises a light chain variable region B (VLB) and a heavy chain variable region B (VHB); wherein the binding arm B is derived from an IgG polypeptide B(IgG(B)) comprising a homodimeric structure consisting of a light chain variable region B (VLB) and a heavy chain variable region B(VHB).

In some embodiments, the VHB comprises VHB CDR1, VHB CDR2, and VHB CDR3, and the VLB comprises VLB CDR1, VLB CDR2, and VLB CDR3.

In some embodiments, the binding arm B that specifically binds to c-Met has VHB CDR1 of the amino acid sequence as shown in SEQ ID NO:25.

In some embodiments, the binding arm B that specifically binds to c-Met has VHB CDR2 of the amino acid sequence as shown in SEQ ID NO:26.

In some embodiments, the binding arm B that specifically binds to c-Met has VHB CDR3 of the amino acid sequence as shown in SEQ ID NO:27.

In some embodiments, the binding arm B that specifically binds to c-Met has VLB CDR1 of the amino acid sequence as shown in SEQ ID NO:28.

In someembodiments, the binding arm B that specifically binds to c-Met hasVLBCDR2 of the amino acid sequence as shown in SEQ ID NO:29.

In some embodiments, the binding arm B that specifically binds to c-Met has VLB CDR 3 of the amino acid sequence as shown in SEQ ID NO: 30.

In some embodiments, the heavy chain variable region B (VHB) comprises VHB CDR1, VHB CDR2, and VHB CDR3 of the amino acid sequences as shown in SEQ ID NOs:25, 26, and 27, respectively.

Insome embodiments, the light chain variable region B(VLB) comprises VLB CDR1, VLB CDR2, and VLB CDR3 of the amino acid sequences as shown in SEQ ID NOs:28, 29, and 30, respectively.

In some embodiments, the c-Met binding arm B comprises VHB CDR1, VHB CDR2, and VHB CDR 3 of the amino acid sequences as shown in SEQ ID NOs:25, 26, and 27, respectively, and VLB

CDR1, VLB CDR2, and VLB CDR3 of the amino acid sequences as shown in SEQ ID NOs:28, 29, and 30.

In some embodiments, the heavy chain variable region B (VHB) comprises the amino acid sequence as shown in SEQ ID NO:7.

In some embodiments, the light chain variable region B (VLB) comprises the amino acid sequence shown as in SEQ ID NO:8.

In some embodiments, the VHB and VLB comprise amino acid sequences as shown in SEQ ID NOs:7 and 8, respectively.

In some embodiments, the binding arm B is derived from a homodimeric IgG polypeptide B (IgG (B)) consisting of a VHB comprising the amino acid sequence of SEQ ID NO:7 and a VLB comprising the amino acid sequence of SEQ ID NO:8.

In some embodiments, the binding arm B further comprises heavy chain constant region B (CHB) and light chain constant region B (CLB).

In some embodiments, the CHB an isotype of IgG1, IgG2, IgG3, or IgG4.

In some embodiments, the CHB is an IgG1 isotype heavy chain constant region.

The CHB is an IgG1 isotype heavy chain constant region.

In some embodiments, the CHB comprises an Fc(B) region comprising a CH3(B) region.

In some embodiments, the CH3(B) region comprises amino acid mutations that reduce heavy chain mismatch rates.

In some embodiments, the CH3(B) region comprises an amino acid mutation as shown in K409R.

In some embodiments, the CH3(B) region comprises IgG subtype amino acid reversion mutations that reduce immunogenicity.

In some embodiments, the Fc(B) region comprises a combination of amino acid mutations as shown in K214R, D356E, and L358M.

In some embodiments, the Fc(B) comprises the amino acid sequence as shown in SEQ ID NO:10.

In some embodiments, the CHB comprises the amino acid sequence as shown in SEQ ID NO:13.

In some embodiments, the CLB is one of κ type and λ type light chain constant regions.

In some embodiments, the CLB is a κ light chain constant region.

In some embodiments, the CLB comprises the amino acid sequence as shown in SEQ ID NO: 14.

The amino acid sequence of the binding arm B of the bispecific antibody embodiment of the present invention and the amino acid sequence of the parent antibody are shown in Example 1.

### Structure of bispecific antibodies specifically binds to EGFR and c-Met

In a further aspect, the present invention relates to EGFR/c-Met bispecific heterodimeric proteins obtained or obtainable by the methods of the present invention.

In an embodiment of the method of the present invention, binding arm A and binding arm A of the bispecific molecule are both Fab structures, Fab(A) arm binds to EGFR-specific tumor cells, such as tumor cell surface protein EGFR, and Fab arm (B) recognizes c-Met-specific tumor cells, such as tumor cell surface protein c-Met.

In some embodiments, wherein binding arm A that binds to EGFR is an antigen binding arm comprising a VH/VL pair or a light chain/heavy chain pair specific for EGFR and binding arm B that binds to c-Met is an antigen binding arm comprising a VH/VL pair or a light chain/heavy chain pair specific for c-Met.

In addition, EGFR/c-Met bispecific heterodimer proteins of the present invention are asymmetric molecules, molecules with different characteristics on each Fab arm (e.g. EGFR-specific Fab(A) or c-Met-specific Fab(B)) or each CH3 domain (e.g. CH3(A) or CH3(B)) or molecules with different modifications throughout the molecule, e.g. molecules with amino acid substitutions for reducing heavy chain mismatches and reducing immunogenicity. Such asymmetric molecules may be produced in any suitable combination. This is further illustrated below by some non-limiting examples.

The bispecific antibody specifically binding EGFR and c-Met of the present invention comprises an antigen-binding arm A specifically binding EGFR and an antigen-binding arm B specifically binding c-Met, wherein the binding arm A is a half-antibody structure polypeptide A derived from a homodimeric IgG antibody with EGFR binding specificity comprising a variant Fc(A) region comprising a CH3(A) region; and the binding arm B is a half-antibody structure polypeptide B derived from a homodimeric IgG antibody with c-Met binding specificity comprising a variant Fc(B) region, wherein the Fc(B) region comprises a CH3(B) region; wherein the CH3(A) region and CH3(B) region differ in sequence and such that heterodimer interaction between the CH3(A) region and CH3(B) region is stronger than interaction between CH3 of homodimers of the CH3(A) region and CH3(B) region respectively.

In some embodiments, the EGFR-specific binding arm A comprises LCA and HCA as shown in SEQ ID NO:1 and 2, respectively, and the c-Met-specific binding arm B comprises LCB and HCB as shown in SEQ ID NO:3 and 4, respectively. The heavy chain of the binding arm A (HCA) and the heavy chain of the binding arm B (HCB) interact to form a heterodimer structure.

In some embodiments, the LCA and the HCA comprise an EGFR-specific light chain variable region (VLA) and a heavy chain variable region (VHA), respectively, and the LCB and the HCB comprise a c-Met-specific light chain variable region (VLB) and a heavy chain variable region ((VHB), respectively.

In some embodiments, the VHA comprises VHA CDR1, VHA CDR2 and VHA CDR3 of the amino acid sequences as shown in SEQ ID NOs:19, 20 and 21, respectively.

In some embodiments, the VLA comprises VLA CDR1, VLA CDR2, and VLA CDR3 of the amino acid sequences as shown in SEQ ID NOs:22, 23, and 24, respectively.

In some embodiments, the VHB comprises VHB CDR1, VHB CDR2, and VHB CDR3 of the amino acid sequences as shown in SEQ ID NOs:25, 26, and 27, respectively.

In some embodiments, the VLB comprises VLA CDR1, VLA CDR2, and VLA CDR3 of the amino acid sequences as shown in SEQ ID NOs:28, 29, and 30, respectively.

In some embodiments, the VLA comprises VLA CDR1, VLA CDR2, and VLA CDR3 the of amino acid sequences as shown in SEQ ID NOs:19, 20, and 21, respectively, the VHA comprises VHA CDR1, VHA CDR2, and VHA CDR3 of the amino acid sequences as shown in SEQ ID NOs:22, 23, and 24, respectively, the VLB comprises VLA CDR1, VLA CDR2, and VLA CDR3 of the amino acid sequences as shown in SEQ ID NOs:25, 26, and 27, respectively, and the VHB comprises VHB CDR1, VHB CDR2, and VHB CDR3 of the amino acid sequences as shown in SEQ ID NOs:28, 29, and 30, respectively.

In some embodiments, binding arm A with the EGFR-specific half-antibody structure comprises VHA and VLA as shown in SEQ ID NOs:5, 6, respectively.

In some embodiments, binding arm B with the c-Met-specific half-antibody structure comprises VLB and VHB as shown in SEQ ID NOs:7 and 8, respectively.

The light chain and heavy chain of binding arm A and binding arm B of the bispecific antibodies of the present invention disclosed herein further comprise light chain constant regions and heavy chain constant regions, respectively.

In some other embodiments, each Fab arm present in the bispecific molecule is derived from a different IgG subclass.

Preferably, both LCA and HCA of the binding arm A and LCB and HCB of the binding arm B of the bispecific antibody according to the present invention disclosed herein comprise constant regions of a full-length human IgG antibody.

In some embodiments, the binding arm A and the binding arm B are full-length IgG antibodies, preferably one of full-length IgG1, IgG2, IgG3 or IgG4 antibodies.

In some preferred embodiments the full-length IgG antibody is an antibody of the human IgG1 isotype.

In another embodiment of the heterodimeric protein of the present invention, the Fc region of polypeptide A (Fc(A)) is an isotype selected from IgG1, IgG2, IgG3 and IgG4 (except for the specified mutations) and the Fc region of polypeptide B (Fc(B)) is an isotype selected from IgG1, IgG2, IgG3 and IgG4 (except for the specified mutations).

In a further embodiment of the heterodimeric protein of the present invention, the Fc region of both the polypeptide A and the polypeptide B are IgG1 isotype.

In some embodiments, the binding arm of the bispecific antibody molecule described herein comprises a heavy chain constant region (CH) of IgG1 isotype, such as CHA region of binding arm A and CHB region of binding arm B.

For example, as described in more detail below, asymmetric bispecific antibody molecules have different CHA and CHB.

In some embodiments, the CHA comprises Fc(A) and the CHB comprises Fc(B).

In some embodiments, the Fc(A) comprises CH3(A) and the Fc(B) comprises CH3(B).

In some embodiments, heavy chain constant regions such as CHA and CHB of bispecific antibody molecules described herein may comprise one or more amino acid modifications. In some embodiments, the amino acid modification is amino acid substitution.

In a further aspect, the present invention relates to an EGFR/c-Met bispecific heterodimeric protein comprising a polypeptide A comprising an immunoglobulin Fc(A) region comprising a CH3(A) region; and a polypeptide B comprising an immunoglobulin Fc(B) region comprising a CH3(B) region, wherein the CH3(A) region and CH3(B) region differ in sequence and such that heterodimer interaction between the CH3(A) region and CH3(B) region is stronger than homodimer interaction of each of the CH3(A) regions and CH3(B) regions.

In some preferred embodiments, EGFR/c-Met bispecific heterodimeric proteins of the present invention are prepared using CH3 engineered Fab exchange technology platforms customary in the antibody industry that provide modified heavy chains the that preferentially form heterodimers after co-expression of the the modified the binding arm A half antibody and binding arm B half antibody the in cloned cells, with the stronger interaction between heavy chain HCA and HCB, particularly Fc(A) and Fc(B), than between homodimers.

In some embodiments, the CH3(A) of antigen binding arm A and the CH3(B) of antigen binding arm B both comprise mutations that enhance and promote the formation of heterodimer.

In some embodiments, the Fc(A) and the Fc(B) each comprise an amino acid mutation that prevents heavy chain mismatch.

The bispecific antibodies of the present invention disclosed herein are preferably used in humans. A preferred embodimentof the bispecific antibody of the present invention is a human or humanized antibody. The bispecific antibody of the present invention as disclosed herein is preferably a heterodimeric bispecific antibody formed by stronger interaction through the CH3 site of the heavy chain between the binding arm of the half-antibody structure targeting EGFR and the binding arm of the half-antibody structure targeting c-Met than between the respective homodimers.

In some embodiments, the CH3(A) region has an amino acid other than Phe at position 405, such as an amino acid other than Phe, Arg or Gly at position 405; in some embodiments, the CH3(B) region has an amino acid other than Lys, Leu or Met at position 409.

In some embodiments, wherein the CH3(A) comprises an amino acid mutation as shown in F405L. In some embodiments, the CH3(B) comprises an amino acid mutation as shown in K409R.

In some embodiments, the CH3(A) and the CH3(B) comprise amino acid mutations F405L and K409R, respectively, preventing heavy chain mismatch.

In some embodiments, both the Fc(A) and Fc(B) regions further comprise combinations of amino acid site mutations that reduce immunogenicity.

In some embodiments, both the CH3A and the CH3B comprise a combination of amino acid site mutations consisting of amino acid mutations K214R, D356E and L358M that reduce immunogenicity.

In a preferred embodiment, the binding arm A and the binding arm B may form a heterodimer by interaction between CHA and CHB, more specifically, between the CH3(A) and the CH3(B).

In some preferred embodiments, Fc(A) of binding armA comprises an Fc(A) fragment derived from constant region CHA of heavy chain A as shown in SEQ ID NO:3 and Fc(B) of binding armB comprises an Fc(B) fragment derived from constant region CHB of heavy chainB as shown in SEQ ID NO:4.

In some preferred embodiments, Fc(A) of binding armA comprises an amino acid sequence as shown in SEQ ID NO:9 and Fc(B) of binding armB comprises an amino acid sequence as shown in SEQ ID NO:10.

In some embodiments, the CHA and the CHB are heavy chain constant regions of IgG1 isotype.

In some embodiments, the CHA region comprises CH3(A) of the amino acid sequence shown in SEQ ID NO:33.

In some embodiments, the CHB region comprises CH3(B) of the amino acid sequence shown in SEQ ID NO:34.

In some embodiments, the CHA region comprises the amino acid sequence shown in SEQ ID NO:11.

In some embodiments, the CHB region comprises the amino acid sequence shown in SEQ ID NO:13.

In some embodiments, the CH3(A) and the CH3(B) comprise amino acid sequences as shown in SEQ ID NO:33 and as shown in SEQ ID NO:34, respectively.

In some embodiments, the CHA and the CHB comprise amino acid sequences as shown in SEQ ID NO:11 and as shown in SEQ ID NO:13, respectively.

In some embodiments, bispecific antibody molecules described herein comprise light chain constant regions, such as CLA comprised by antigen binding arm A and CLB comprised by antigen binding arm B.

In some embodiments, the CLA and CLB are selected from one of κ type light chain constant regions or λ type light chain constant regions.

In some embodiments, the CLA and CLB are both κ type light chain constant regions.

In some embodiments, the CLA region comprises the κ CLA region of the amino acid sequence shown in SEQ ID NO:12.

In some embodiments, the CLB region comprises the κ CLB region of the amino acid sequence shown in SEQ ID NO:14.

In some other embodiments of the heterodimeric protein of the present invention, the polypeptide A may be the full length heavy chain of an antibody, preferably a human antibody.

In some other embodiments of the heterodimeric protein of the present invention, the polypeptide B may be the full length heavy chain of an antibody, preferably a human antibody.

In some other embodiments of the heterodimeric protein of the present invention, both polypeptide A and polypeptide B are full-length heavy chains of two antibodies, preferably two human antibodies that bind to EGFR and c-Met, respectively, and thus the resulting heterodimeric protein is a bispecific antibody.

In some embodiments, the resulting heterodimeric protein comprises, in addition to heavy chains, two full-length light chains derived from EGFR-specific antibody and derived from a c-Met-specific antibody, respectively.

In another embodiment of the heterodimeric protein of the present invention, the increased intensity of the heterodimer interaction of the EGFR/c-Met bispecific as compared to the respective EGFR homodimer interaction and c-Met homodimer interaction is due to CH3 modification and not due to introduction of covalent bonds, cysteine residues or charged residues.

In another embodiment of the EGFR/c-Met heterodimeric protein of the present invention, the heterodimeric interaction between the polypeptide A and polypeptide B in the heterodimeric protein is such that Fab arm interchange does not occur at a final concentration of 75mM 2-MEA under the conditions described in Example 1.

In the method for preparing the bispecific antibody of the present invention, the probability of greatly reducing and preventing light and heavy chain mismatches is generally achieved by modifying the constant region of the heavy chain such that heterodimerization between the half-antibodies of the two specific binding arms formed by interaction of the heavy chain, particularly the CH3 portion of the heavy chain, between the binding arms of the bispecific antibody of the present invention is stronger than homodimerization between the half-antibody of each origin and the same half-antibody, and is more favorable for heterodimerization( i.e., heavy chain dimerization in combination with another heavy chain/light chain).

In some preferred embodiments, ADCC activity of antibodies can also be improved by techniques known to those skilled in the art.

Bispecific antibodies of the present invention as disclosed herein may have good ADCC properties by heavy chain modification through heavy chain constant region amino acid mutations.

In another embodiment, an amino acid mutation improve binding to an Fcγ- receptor or FcRn is comprised on one of the two Fab arms of the bispecific molecule.

In another embodiment, an amino acid mutation improve binding to Clq is comprised on one of the two Fab arms of the bispecific molecule.

In an exemplary embodiment of the antibody of the present invention, the combination of amino acid sequence numbers of fragments comprised in the binding arm A and the binding arm B is summarized in Table 1.

### Functional Properties of Bispecific Antibodies of the present invention

The EGFR/c-Met bispecific antibody of the present invention has the following functions:
(1) It can specifically bind to human c-Met protein and EGFR protein *in vitro,* and in ELISA assays, the EC50 for binding to human c-Met protein is significantly lower than the EC50 for binding to EGFR protein, with a difference exceeding 20-fold.
(2) It exhibits extremely high affinity for c-Met-HIS and high affinity for EGFR-HIS respectively;
(3) It can specifically bind to cells highly expressing human c-Met protein, cells highly expressing EGFR protein, and double-positive tumor cells expressing both c-Met protein and EGFR protein;
(3) It can block the binding of the ligand HGF to the receptor c-Met and block the binding of the ligand EGF to the receptor EGFR;
(4) The Fc has affinity for receptor proteins comparable to that of amivantamab;
(5) It can significantly inhibit the level of autophosphorylation of p-c-Met (Y1234/1235) mediated by the ligand HGF and slightly inhibit the level of autophosphorylation of p-EGFR (Yl173) mediated by the ligand EGF respectively within tumor cells;
(6) It can mediate the internalization and degradation of both EGFR and c-Met target proteins, and the level of internalization mediated by the anti-c-Met antibody arm is higher than the level of internalization mediated by the anti-EGFR antibody arm;
(7) It can mediate ADCC effects against cells highly expressing EGFR, cells highly expressing c-Met, and double-positive tumor cells highly expressing both EGFR and c-Met respectively; wherein the mediated ADCC primarily depends on the EGFR antibody arm;
(8) It can inhibit the proliferation of tumor cells *in vitro,* and the inhibition hass a synergistic effect compared to the combination of EGFR monoclonal antibody and c-Met monoclonal antibody;
(9) It can inhibit the proliferation of TKI-resistant tumor cells with acquired resistance *in vitro;* the inhibitory efficacy is stronger than the combination of anti-EGFR monoclonal antibody and anti-c-Met monoclonal antibody, and the inhibitory efficacy has a synergistic effect compared to the EGFR monoclonal antibody and the c-Met monoclonal antibody;
(10) It can have anti-tumor activity in mouse models of tumor cells.

### Nucleic acids, vectors, host cells

In a further aspect, the present invention provides a composition comprising the encoding nucleic acids for the binding arm A and the encoding nucleic acids for the binding arm B of the EGFR/c-Met heterodimeric bispecific antibody molecule as described in the preceding aspects herein.

In one embodiment, the present invention provides a composition of encoding nucleic acids comprising nucleic acid 1# encoding the binding arm A that specifically binds to EGFR and nucleic acid 2# encoding the binding arm B that specifically binds to c-Met, wherein the nucleic acid 1# is a fusion comprising encoding nucleic acid 3# for the heavy chain A of binding arm A and encoding nucleic acid 4# for the light chain A of binding arm A, and the nucleic acid 2# is a fusion comprising encoding nucleic acid 5# for the heavy chain B of binding arm B and encoding nucleic acid 6# for the light chain B of binding arm B, each respectively encoded.

In some embodiments, the heavy chain A (HCA) encoded by the encoding nucleic acid 3# comprises the CH3 mutation F405L which promotes the interaction between the EGFR binding arm and the c-Met binding arm to avoid heavy chain mispairing.

In some embodiments, the heavy chain B (HCB) encoded by the encoding nucleic acid 5# comprises the CH3 mutation K409R which promotes the interaction between the EGFR binding arm and the c-Met binding arm to avoid heavy chain mispairing.

In some embodiments, the Fc(A) of the heavy chain A encoded by the encoding nucleic acid 5# comprises the amino acid mutation combination K214R, D356E, L358M which reduces immunogenicity.

In some embodiments, the Fc(B) of the heavy chain B encoded by the encoding nucleic acid 7# comprises the amino acid mutation combination K214R, D356E, L358M which reduces immunogenicity.

In some embodiments, the nucleic acid molecules are purified or isolated from other nucleic acids or naturally occurring biological materials. A person skilled in the art of antibodies can prepare such nucleic acid molecules using methods well known in the art.

Described herein are these encoding nucleic acid 4# for the light chain A (LCA) and encoding nucleic acid 3# for the heavy chain A (HCA) of the EGFR-specific binding arm A.

Described herein are these encoding nucleic acid 6# for the light chain B (LCB) and encoding nucleic acid 5# for the heavy chain B (HCB) of the EGFR-specific binding arm B.

In some embodiments, the composition of encoding nucleic acids of this aspect can comprise:
(i) a nucleic acid sequence encoding the LCA domain as shown in SEQ ID NO: 1, and a nucleic acid sequence encoding the HCA domain as shown in SEQ ID NO: 2;
(ii) a nucleic acid sequence encoding the LCB domain as shown in SEQ ID NO: 3, and a nucleic acid sequence encoding the HCB domain as shown in SEQ ID NO: 4.

In another aspect, the present invention relates to an expression vector comprising the encoding nucleic acid construct for the EGFR-specific homodimer specified above and the encoding nucleic acid construct for the c-Met-specific homodimer.

In one aspect, the present invention also provides a composition comprising nucleic acid molecules encoding the binding arm A with half antibody structure or the binding arm B with half antibody structure of the bispecific antibody molecule described herein, or a composition of vectors respectively encoding nucleic acid molecules for the antibody molecule binding arm A or the antibody binding arm B.

The isolated nucleic acid molecules can be used to express the antibody molecules of the present disclosure or their binding. Nucleic acids are typically provided in the form of recombinant vectors for expression. Suitable vectors can be selected or constructed, comprising appropriate regulatory sequences including promoter sequences, terminator fragments, polyadenylation sequences, enhancer sequences, marker genes and other suitable sequences. Preferably, the vector comprises appropriate regulatory sequences to drive the expression of the nucleic acid in a host cell. The vector can be a plasmid, a virus, for example a plasmid, phage or phagemid, as appropriate.

In some embodiments, the vector is a pHR vector.

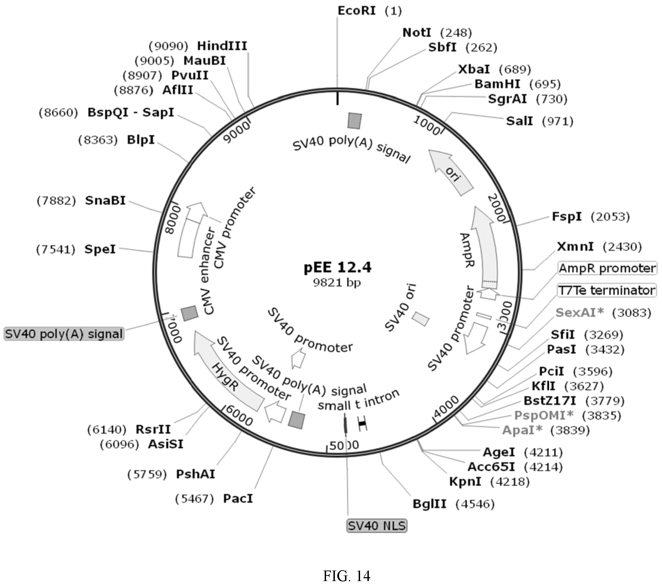

In some embodiments, the pHR vector is PEE12.4, as shown in FIG. 14.

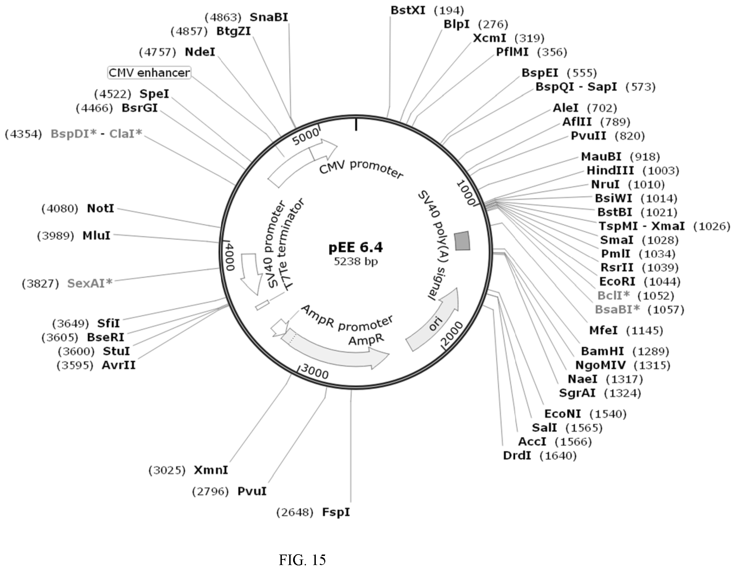

In some embodiments, the pHR vector is PEE6.4, as shown in FIG. 15.

In another aspect, the present invention relates to a host cell comprising the first and second nucleic acid constructs specified above.

### Preparation method

In another aspect, the present invention provides an *in vitro* method for producing EGFR/c-Met heterodimer bispecific antibodies, the method comprising the following steps:
a) providing an EGFR-specific homodimeric protein A comprising an immunoglobulin Fc(A) region, the Fc(A) region comprising a CH3(A) region,
b) providing a c-Met-specific homodimeric protein B comprising an immunoglobulin Fc(B) region, the Fc(B) region comprising a CH3(B) region,
   wherein the CH3(A) region and CH3(B) region differ in sequence and such that heterodimer interaction between the CH3(A) region and CH3(B) region is stronger than interaction between CH3 of homodimers of the CH3(A) region and CH3(B) region respectively,
c) incubating the EGFR-specific protein A together with the c-Met-specific protein B under reducing conditions sufficient to allow cysteines in the hinge region to undergo disulfide bond isomerization, and
d) obtaining the EGFR/c-Met bispecific heterodimeric protein.

In some embodiments, the homodimeric protein A and the homodimeric protein B, besides comprising the Fc region, also comprise one or more or all other regions of an antibody, namely CH1 region, VH region, CL region and/or VL region. Therefore, in some embodiments, the homodimeric protein A is a full-length antibody. In another embodiment, the homodimeric protein B is a full-length antibody.

In one embodiment, the Fc region of the homodimeric protein A is of an isotype selected from IgG1, IgG2, IgG3, and IgG4, and the Fc region of the homodimeric protein B is of an isotype selected from IgG1, IgG2, IgG3, and IgG4. In a preferred embodiment, the Fc regions of both the homodimeric protein A and the homodimeric protein B are of the IgG1 isotype.

In some embodiments, the homodimeric protein A and B provided in steps a) and b) are purified.

As mentioned above, the CH3(A) region and CH3(B) region of the homodimeric starting proteins differ in sequence and such that heterodimer interaction between the CH3(A) region and CH3(B) region is stronger than interaction between CH3 of homodimers of the CH3(A) region and CH3(B) region respectively.

In some embodiments, the increased strength of the heterodimeric interaction compared to the various homodimeric interactions is due to CH3 modifications, not due to the introduction of covalent bonds, cysteine residues, or charged residues.

In some embodiments, the product of the present invention is highly stable and does not undergo Fab arm exchange under mild reducing conditions *in vitro* or importantly *in vivo* after administration to humans *in vivo.* Therefore, in one embodiment, the heterodimeric interaction between the two of protein A and protein B in the resulting heterodimeric protein makes Fab arm exchange cannot occur under the conditions described in Example 1 at a final concentration of 75 mM 2-MEA.

In some embodiments, a stable heterodimeric protein can be obtained in high yield using the method of the present invention based on two homodimeric starting proteins comprising only a few relatively conservative asymmetric mutations in the CH3 region.

Therefore, in some embodiments, the sequence of the CH3(A) region and the sequence of the CH3(B) region comprise amino acid substitutions at positions that are not identical.

In some embodiments, the amino acids are natural amino acids.

In some embodiments, compared to the wild-type CH region, the homodimeric protein A comprises no more than 1 amino acid substitution in the CH3(A) region, and the homodimeric protein B comprises no more than 1 amino acid substitution in the CH3(B) region.

In some embodiments, the homodimeric protein A comprises an amino acid substitution at a position selected from comprising amino acid 405, and the homodimeric protein B comprises an amino acid substitution at a position selected from comprising amino acid 409, wherein the homodimeric protein A and the homodimeric protein B are not substituted at the same position.

In some embodiments, the homodimeric protein A comprises an amino acid substitution at position 405, and the homodimeric protein B comprises an amino acid substitution at position 409.

In some embodiments, the homodimeric protein A comprises an amino acid other than Phe at position 405, and the homodimeric protein B comprises an amino acid other than Lys, Leu, or Met at position 409.

In some embodiments, the homodimeric protein A comprises an amino acid at position 405 that is not Phe, Arg, or Gly.

In one such embodiment, the homodimeric protein B comprises an amino acid other than Lys, Leu, or Met at position 409, and the homodimeric protein A comprises an amino acid other than Phe at position 405.

In another embodiment, the homodimeric protein B comprises an amino acid other than Lys, Leu, or Met at position 409, and the homodimeric protein A comprises an amino acid other than Phe, Arg, or Gly at position 405.

In even another embodiment, the homodimeric protein A comprises Leu at position 405, and the second homodimeric protein B comprises Arg at position 409.

In addition to the amino acid substitutions specified above, the homodimeric protein A and B may comprises other amino acid substitutions, deletions, or insertions relative to the wild-type Fc sequence.

In another embodiment, the homodimeric protein B and the homodimeric protein A comprise the amino acid mutation combination consisting of K214R, D356E, and L358M.

In another embodiment, the CH3(A) region, besides comprising the specified mutations, also comprises the sequence as shown in SEQ ID NO:33.

In another embodiment, the CH3(B) region, besides comprising the specified mutations, also comprises the sequence as shown in SEQ ID NO:34.

As mentioned above, step c) of the method of the invention comprises incubating the first protein together with the second protein under reducing conditions sufficient to allow cysteines in the hinge region to undergo disulfide bond isomerization. Examples of suitable conditions are given herein. The minimum requirement for cysteines in the hinge region to undergo disulfide bond isomerization may vary depending on the homodimeric starting proteins, particularly the exact sequence in the hinge region. Importantly, the respective homodimeric interactions of the CH3(A) and CH3(B) regions are weak enough to allow cysteines in the hinge region to undergo disulfide bond isomerization under given conditions.

In some embodiments, the reducing conditions of step c) comprise adding a reducing agent, the reducing agent comprising but not limited to: 2-mercaptoethylamine (2-MEA), dithiothreitol (DTT), dithioerythritol (DTE), glutathione, tris(2-carboxhyl)phosphine (TCEP), L-cysteine and β-mercaptoethanol, preferably a reducing agent selected from: 2-mercaptoethylamine, dithiothreitol and tris(2-carboxhyl)phosphine.

In some preferred embodiments, the reducing conditions of step c) comprise adding the reducing agent 2-mercaptoethylamine (2-MEA).

In another embodiment, step c) comprises incubating at a temperature of at least 31°C for at least 5 minutes in the presence of at least 75 mM 2-mercaptoethylamine. The incubation can be performed at a pH from 5 to 8, such as pH 7.4.

In another embodiment, step d) comprises, for example, restoring conditions to non-reducing or less reducing by removing the reducing agent, such as by desalting.

In some preferred embodiments, step d) comprises, for example, restoring conditions to non-reducing or less reducing at pH 11.0 by removing the reducing agent, such as by desalting.

In some embodiments, the method of the present invention yields an antibody product in which more than 80%, e.g. more than 90%, e.g. more than 95%, e.g. more than 99% of the antibody molecules are the desired bispecific antibody.

The post-production nature of preparing EGFR/c-Met bispecific antibodies by Fab exchange under reducing conditions (e.g., by adding 2-MEA) as disclosed herein makes it a very suitable strategy for (high-throughput) screening of EGFR/c-Met bispecific antibodies. Furthermore, the *in vitro* method can be performed in a library, which allows greater control, higher flexibility, and yield over EGFR/c-Met heterodimeric proteins compared to co-expression, while screening can be performed in the final therapeutic form, eliminating the need for engineering after lead selection.

In another aspect, the preparation method of the EGFR/c-Met bispecific heterodimeric protein of the present invention can employ a "matrix" screening, i.e., generating a large number of combinations of EGFR/c-Met binding specificities based on two groups of antibodies, one group of anti-EGFR antibodies having an identical CH3(A) region and another group of anti-c-Met antibodies having an identical CH3(B) region, wherein the CH3(A) and CH3(B) region differ in sequence and such that heterodimer interaction between the CH3(A) and CH3(B) region is stronger than homodimer interaction of each of the CH3(A) regions and CH3(B) regions.

Therefore, in some embodiments, the present invention relates to a method of selecting a heterodimeric protein with EGFR/c-Met bispecificity, the method comprising the following steps:
a) providing a first group of EGFR-specific homodimeric proteins A comprising an Fc(A) region, wherein the homodimeric proteins A have an identical CH3(A) region,
b) providing a second group of c-Met-specific homodimeric proteins B comprising an Fc(B) region, wherein the homodimeric proteins B have an identical CH3(B) region, wherein the CH3(A) region and CH3(B) region differ in sequence and such that heterodimer interaction between the CH3(A) region and CH3(B) region is stronger than homodimer interaction of each of the CH3(A) regions and CH3(B) regions,
c) incubating the combination of the first group of EGFR-specific homodimeric proteins A and the second group of c-Met-specific homodimeric proteins B under reducing conditions sufficient to allow cysteines in the hinge region to undergo disulfide bond isomerization, thereby generating a group of bispecific antibodies,
d) optionally restoring the condition to non-reducing,
e) testing the resulting group of heterodimeric proteins for given desired properties, EGFR/c-Met bispecificity, etc., and
f) selecting a heterodimeric protein with desired EGFR/c-Met bispecific properties.

In some embodiments, the present invention relates to a method of selecting a desired EGFR/c-Met bispecific antibody, the method comprising the following steps:
a) providing a first group of homodimeric antibodies comprising a variable region with EGFR binding specificity, wherein the antibodies of the first group comprise a CH3(A) region,
b) providing a second group of homodimeric antibodies comprising a variable region with c-Met binding specificity, wherein the antibodies of the second group comprise a CH3(B) region, wherein the CH3(A) region and CH3(B) region differ in sequence and such that heterodimer interaction between the CH3(A) region and CH3(B) is stronger than homodimer interaction of each of the CH3(A) regions and CH3(B),
c) incubating the combination of the first group of antibodies and the second group of antibodies under reducing conditions sufficient to allow cysteines in the hinge region to undergo disulfide bond isomerization, thereby generating a group of EGFR/c-Met bispecific antibodies,
d) optionally restoring the condition to non-reducing,
e) testing the resulting group of bispecific antibodies for given desired properties, and
f) selecting a bispecific antibody with desired EGFR/c-Met bispecificity.

The screening strategy comprises two groups of antibody vectors comprising a range of specificities, where one group is cloned into a main chain capable of participating in Fab arm exchange with the main chain of the second group under reducing conditions (e.g., by adding 2-MEA). For example, clone the first group into an IgG1-F405L main chain, and clone the second group into an IgG1-K409R main chain (see also Example 1 for other possible backbone combinations).

Each member of the two groups of antibody vectors is then expressed on a small scale individually. For example, all antibody vectors are transiently transfected into CHO-K1 cells and expressed in culture.

Then, the expressed antibodies from the two groups are mixed pair-wise in an equimolar ratio in a matrix-like manner. For example, all individual antibodies are purified by small-scale Protein A chromatography, and antibody concentrations are measured by absorbance at 280 nm. Alternatively, other suitable (small-scale) purification methods or methods known in the art for determining protein concentration may be used.

In another embodiment, the purification step may be omitted if the expression medium does not affect downstream applications. Subsequently, antibody concentrations are normalized so that appropriate volumes comprise equimolar amounts of anti-EGFR antibody and anti-c-Met antibody.

For example, mix a group of anti-EGFR antibodies in the F405L backbone (e.g., BT461-DB-EGFR) each with an anti-cMet antibody in the K409R backbone (e.g., BT461-DB-cMet) at a ratio of 1:1.1; add an appropriate amount of reducing agent to the mixture of the antibodies and incubate at a permissible temperature for a suitable period of time. For example, to a mixture comprising a mg of antibody A (F405L) and 1.1a mg of antibody B (K409R), add 0.25b ml of 300 mM 2-MEA to form an assembly reaction system with a volume of b, resulting in a final concentration of 75 mM 2-MEA (final concentration 75 mM 2-MEA) and incubate at 31°C for 5 hours. Then, remove the reducing agent from the mixture (now comprising bispecific antibodies) to promote disulfide bond oxidation and avoid interference from the reducing agent during screening assays. For example, remove 2-MEA by buffer exchange using a desalting plate for the two-specificity bispecific antibody mixture. Alternatively, other suitable methods known in the art for removing reducing agents may be used.

The bispecific antibodies are then characterized biochemically or functionally to identify EGFR/c-Met bispecific antibody candidates of the present invention.

In some embodiments, a person skilled in the art of antibodies can also obtain the bispecific heterodimeric protein of the present invention by co-expressing constructs encoding an EGFR-specific polypeptide A and a c-Met-specific polypeptide B in a single cell.

Therefore, in another aspect, a method for preparing the heterodimeric protein of the present invention by co-expression in a single cell comprises the following steps:
a) providing a first nucleic acid construct encoding a polypeptide A comprising an immunoglobulin Fc(A) region, the Fc(A) region comprising a CH3(A) region,
b) providing a second nucleic acid construct encoding a second polypeptide B comprising an immunoglobulin Fc(B) region, the Fc(B) region comprising a CH3(B) region, wherein the CH3(A) region and CH3(B) region differ in sequence and such that heterodimer interaction between the CH3(A) region and CH3(B) is stronger than homodimer interaction of each of the CH3(A) regions and CH3(B),
c) co-expressing the first and second nucleic acid constructs in a host cell, and
d) obtaining the heterodimeric protein from the cell culture.

In one such embodiment, the homodimeric protein B comprises an amino acid other than Lys, Leu, or Met at position 409, and the homodimeric protein A comprises an amino acid other than Phe at position 405.

In another embodiment, the the homodimeric protein A comprises an amino acid other than Phe, Arg, or Gly at position 405, and homodimeric protein B comprises an amino acid other than Lys, Leu, or Met at position 409.

In another embodiment, the first homodimeric protein B comprises Arg at position 409, and the homodimeric protein A comprises Leu at position 405.

Suitable expression vectors (including promoters, enhancers, etc.) and suitable host cells for preparing antibodies are well known in the art. Examples of host cells include yeast, bacteria, and mammalian cells such as CHO or HEK cells.

In other embodiments, the co-expression method of the present invention includes any other features described under the above *in vitro* method.

In some exemplary embodiments, the preparation of the anti-EGFR/c-Met bispecific antibody molecule of the present invention is achieved by constructing recombinant expression vector 1 simultaneously expressing the light chain and heavy chain of the anti-EGFR antibody and recombinant expression vector 2 simultaneously expressing the light chain and heavy chain of the anti-c-Met antibody, respectively, then transiently transfecting the two recombinant expression vectors 1 and 2 into host cells separately, culturing the host cells to produce a fermentation culture product of the bispecific antibody of the present invention, then purifying the protein product prepared by the recombinant host cells from the culture product and obtaining the bispecific EGFR/c-Met antibody of the present invention through *in vitro* assembly and purification. The resulting bispecific antibody's binding arm A can have the characteristics as described above for the EGFR-binding arm, and the binding arm B of the bispecific molecule can have the characteristics as described above for the c-Met-binding arm.

In some exemplary embodiments, the construction of expression vectors for the EGFR/c-Met bispecific antibody of the present invention is based on Genmab's DuoBody platform technology, utilizing genetic engineering techniques to design mutations for the heavy chain (HCA) of the anti-EGFR antibody (F405L, K214R, D356E, L358M) and for the heavy chain (HCB) of the anti-c-Met antibody (K409R, K214R, D356E, L358M), synthesizing encoding nucleic acids 3# and 5# for HCA and HCB, and encoding nucleic acids 4# and 6# for LCA and LCB. Then, the encoding nucleic acids for the LCA of the anti-c-Met or anti-EGFR antibody and the HCA comprising the aforementioned amino acid mutations, and the encoding nucleic acids for the LCB of the anti-c-Met antibody and the HCB comprising the aforementioned amino acid mutations are respectively constructed into 4 expression vectors, resulting in 4 plasmids respectively expressing the light and heavy chains of the anti-c-Met or anti-EGFR antibody. Then, the plasmids expressing the light and heavy chains of the same antigen-binding specificity are subjected to SalI/NotI digestion and ligation, constructing the two nucleotide sequences of the nucleic acid encoding the same EGFR antigen-binding specific LCA and HCA into the same expression vector, and constructing the two nucleotide sequences of the nucleic acid encoding the same c-Met antigen-binding specific LCB and HCB into the same expression vector, respectively obtaining recombinant plasmids that commonly carry the encoding nucleic acid fusion for the anti-c-Met antibody light and heavy chains and recombinant plasmids that commonly express the encoding nucleic acid fusion for the anti-EGFR antibody light and heavy chains.

In some exemplary embodiments, the preparation method of the bispecific antibody of the present invention comprises the steps:
1. Based on Genmab's DuoBody platform technology, utilizing genetic engineering techniques to design and synthesize encoding nucleic acid 3# for the heavy chain A (HCA) of the EGFR antibody comprising amino acid mutations (F405L, K214R, D356E, L358M), and encoding nucleic acid 5# for the heavy chain B (HCB) of the anti-c-Met antibody comprising amino acid mutations (K409R, K214R, D356E, L358M); simultaneously designing encoding nucleic acid 4# for the light chain A (LCA) of the anti-EGFR antibody comprising an unmutated κ-type light chain constant region and VLA, and encoding nucleic acid 6# for the light chain B (LCB) of the c-Met antibody.
3. Construct 3# and 5# into expression plasmid vectors A1 and A2, respectively, and construct 4# and 6# into expression plasmids B1 and B2, respectively, to obtain recombinant expression plasmid B1-LCA and B1-LCB1- expressing the light chains of the anti-EGFR antibody and the anti-c-Met antibody respectively, and recombinant expression plasmid A1-HCA expressing the heavy chain A comprising the aforementioned CH3A mutations and Fc(A) mutations, and recombinant expression plasmid A1-HCB expressing the heavy chain B comprising the aforementioned CH3B mutations and Fc(B) mutations.
4. Perform SalI/NotI enzymes digestion and ligation (NEB) on the recombinant expression plasmids A1 and B1 for light and heavy chains, and A2 and B2, constructing the two nucleotide sequences encoding LCA and HCA into the same expression vector, and constructing the two nucleotide sequences encoding LCB and HCB into the same expression vector A1, respectively obtaining recombinant expression plasmid C1 carrying encoding nucleic acid fusion 1# co-expressing the light and heavy chains of the anti-EGFR antibody and recombinant expression plasmid C2 carrying encoding nucleic acid fusion 2# co-expressing the light and heavy chains of the anti-c-Met antibody.
5. Transiently transfect plasmid C1 encoding the anti-EGFR antibody and plasmid C2 encoding the anti-c-Met antibody protein into host cells separately, obtaining recombinant host cell A expressing HCA and LCA of the anti-EGFR binding arm and recombinant host cell B expressing HCB and LCB of the anti-c-Met binding arm.
6. Collect the cell supernatant and optionally isolate and purify the binding arm A and binding arm B of the anti-EGFR/c-Met bispecific antibody of the present invention produced thereby.
*7. In vitro* assembly and purification.

In some embodiments, expression plasmids A1 and A2 for the light chains of the EGFR binding arm and the c-Met binding arm of the anti-EGFR/c-Met bispecific antibody of the present invention are preferably PEE6.4, as shown in FIG. 15.

In some embodiments, expression plasmids B1 and B2 for the heavy chains of the EGFR binding arm and the c-Met binding arm of the anti-EGFR/c-Met bispecific antibody of the present invention are preferably PEE12.4, as shown in FIG. 14.

In some embodiments, the encoding nucleic acids for the light chain and heavy chain of the EGFR binding arm of the anti-EGFR/c-Met bispecific antibody of the present invention are jointly constructed into a PEE expression vector (pHR plasmid) to obtain recombinant pHR expression vector C1 expressing EGFR-specific IgG(A), and the encoding nucleic acids for the light chain and heavy chain of the c-Met binding arm are jointly constructed into a PEE expression vector (pHR plasmid) to obtain recombinant expression plasmid C2 expressing c-Met-specific IgG(B).

The nucleic acid molecules or vectors described herein can be introduced into host cells. Techniques for introducing nucleic acids or vectors into host cells are well known in the art and any suitable technique may be employed.

A range of host cells suitable for producing recombinant antibody molecules is known in the art and includes bacterial, yeast, insect or mammalian host cells. Preferred host cells are mammalian cells, such as CHO, NSO or HEK cells.

In some embodiments, the host cell is a CHO cell.

In some embodiments, the host cell is a CHO-K1 cell.

In some exemplary preferred embodiments, the host cell is a CHO-K1 cell with the FUT8 gene knocked out.

In some embodiments, FUT8 knockout cells may also be used during the production of the bispecific antibody of the present invention to perform glycoengineering on one or both of the homodimeric proteins to reduce fucose, thereby enhancing ADCC.

Knocking out the FUT8 gene in host cells is a genetic engineering means to engineer a reduction in antibody fucose levels. In mammalian cells, fucose binds to the amino glucose on the antibody Fc end via an α-1,6 glycosidic bond, which is catalyzed by the α-1,6-fucosyltransferase encoded by the FUT8 gene. Knocking out the FUT8 gene will completely eliminate fucose on the antibody Fc end, thereby enhancing the ADCC effect of the binding arms constituting the bispecific antibody of the present invention.

The inventors further screened the obtained clones through functional activity, resulting in the bispecific antibody molecules of the present invention which specifically bind EGFR-positive tumor cells and/or specifically bind c-Met-positive tumor cells, and which exhibit significant differences in binding to tumor cells versus non-tumor tissues.

In this manner, the inventors identified antibodies capable of distinguishing tumor from non-tumor tissues. The bispecific antibody of the present invention possesses the expected characteristics necessary for therapeutic applications, namely specific recognition of EGFR and c-Met targets present on cancer cell surfaces and effective triggering of internalization upon binding to these targets, and the binding arm A and binding arm B have significantly different affinities, with the c-Met-specific binding arm B having significantly higher affinity for c-Met than the EGFR-specific binding arm A for EGFR, while conferring tumor-killing capability and safety, and its structure can provide over 99% bispecific antibody monomer purity in *in vitro* assembly, for example BT461-DB.

In some exemplary embodiments, the anti-EGFR/c-Met bispecific antibody with a DuoBody structure of the present invention (e.g., BT461-DB) demonstrates high binding affinity (in the nanomolar range) for c-Met expressed on the cell surface of cancer cells. Furthermore, this anti-EGFR/c-Met bispecific antibody shows a high ability to trigger internalization of the antibody complex, as shown in Example 11. Exemplarily, the present invention yields an EGFR/c-Met bispecific antibody named BT-461-DB.

Methods for culturing host cells are well known in the art. The method may further include a step of isolating and/or purifying the EGFR/c-Met bispecific antibody molecule. Techniques for purifying recombinantly obtained EGFR/c-Met bispecific antibody molecules are well known in the art and include, for example, HPLC, FPLC, or affinity chromatography, such as affinity chromatography.

In some embodiments, purification can be performed using an affinity tag on the antibody molecule. The method may also include formulating the antibody molecule as a pharmaceutical composition, optionally together with pharmaceutically acceptable excipients or other substances as described below.

In some embodiments, the homodimeric protein A and B provided in steps a) and b) are purified.

In some preferred embodiments, the preparation of the anti-EGFR/c-Met bispecific antibody molecule with a DuoBody structure of the present invention comprises *in vitro* assembly and purification steps.

In some preferred embodiments, the assembly is performed according to the Fab exchange technology of the duobody platform, mixing the affinity chromatography-purified half-antibody of anti-EGFR (exemplary such as BT461-DB-EGFR) and the half-antibody of anti-c-Met (exemplary such as BT461-DB-c-Met) *in vitro* for assembly. Add reducing agent for reduction in a water bath, desalt to remove the reducing agent and exchange buffer, then let stand at room temperature for oxidation.

In some preferred embodiments, the purification employs a cation exchange chromatography method.

In some preferred embodiments, the assembly system comprises anti-EGFR antibody (exemplary such as BT461-DB-EGFR) and anti-cMet antibody (exemplary such as BT461-DB-c-Met) at approximately a 1:1 ratio.

In some preferred embodiments, the reducing agent is 2-MEA.

In some preferred embodiments, the reducing agent 2-MEA is at a concentration of 50~100 mmol/L.

In some preferred embodiments, the reduction is performed in a water bath.

In some preferred embodiments, the desalting employs G25 chromatography technology.

### Biological functional properties of the EGFR/c-Met antibody molecules

The EGFR/c-Met bispecific antibody molecule described herein (exemplified as BT461 DB) can be evaluated and validated by reference to certain functional properties.

### Specific binding to EGFR protein and c-Met protein in vitro

In some embodiments, the anti-EGFR/c-Met bispecific antibody of the present invention is capable of specifically binding to EGFR protein and specifically binding to c-Met protein in vitro.

In some embodiments, the bispecific antibody of the present invention is capable of binding to human EGFR with high affinity and specifically binding to human c-Met with very high affinity.

In some exemplary embodiments, the present invention evaluates the level of extracellular binding to EGFR and binding to c-Met of the EGFR/c-Met bispecific antibody by measuring the *in vitro* binding EC50 value to human EGFR and c-Met via ELISA test.

In some exemplary embodiments, the binding arm A of the anti-EGFR/c-Met bispecific antibody of the present invention, such as BT461-DB, can bind to EGFR with an ELISA binding EC50 of approximately 1260-1450 ng/mL, preferably 1290-1350 ng/mL.

In some exemplary embodiments, the anti-EGFR/c-Met bispecific antibody of the present invention (e.g., BT461-DB binding arm B can bind to c-Met with an ELISA binding EC50 of approximately 2-20 ng/mL, preferably 5-15 ng/mL, more preferably 7-10 ng/mL.

The method for determining the ELISA binding EC50 can be found in the method shown in Example 2.

### EGFR and/or Met binding to cell surface

In some embodiments, the bispecific antibody of the present invention is capable of binding to cells overexpressing EGFR and cells overexpressing c-Met, respectively.

In some embodiments, the level of binding of the EGFR/c-Met bispecific antibody to cell surface-expressed EGFR and cell surface-expressed c-Met is evaluated by determining the binding EC50 value via FACS method.

In some embodiments, the bispecific antibody of the present invention, such as BT461-DB, can bind to CHO-K1 cells overexpressing EGFR with a FACS binding EC50 of approximately 1610-1820 ng/mL.

In some embodiments, the bispecific antibody of the present invention, such as BT461-DB, can bind to CHO-K1 cells overexpressing c-Met with a FACS binding EC50 of approximately 245-285 ng/mL.

The method for determining the *in vitro* binding EC50 via FACS can be found in Example 3.

In some embodiments, the bispecific antibody of the present invention, such as BT461-DB, can specifically bind to EGFR/c-Met double-positive tumor cells.

In some exemplary embodiments, the BT461-DB of the present invention can bind to double-positive tumor cells overexpressing EGFR and c-Met with a FACS binding EC50 of approximately 780-910 ng/mL.

In some exemplary embodiments, the double-positive tumor cells overexpressing EGFR and c-Met are MKN45 cells, as shown in Example 4.

In some embodiments, the BT461-DB of the present invention is capable of antagonizing the binding of ligand HGF to the target protein c-Met.

In some exemplary embodiments, such as BT461, the level of antagonism of the binding of ligands HGF, EGF to membrane proteins EGFR, c-MET by the EGFR/c-Met bispecific antibody is evaluated by the antagonistic IC50 value determined by ELISA method. In some embodiments, in the specific binding example of double-positive tumor cells, the ELISA blocking IC50 value of the EGFR/c-Met bispecific antibody of the present invention antagonizing HGF-c-Met is not less than 1000 ng/mL, not less than 830 ng/mL, or preferably not less than 730 ng/mL, more preferably not less than 680 ng/mL, most preferably not less than 630 ng/mL, more preferably not less than 610 ng/mL, more preferably not less than 550 ng/mL, most preferably not less than 460 ng/mL.

In some exemplary embodiments, for example when measured using HGF-HIS, the bispecific antibody BT461 of the present invention can inhibit HGF binding to c-Met with a FACS antagonistic IC50 value of approximately 425-500 ng/mL. The method for determining the antagonistic IC50 via FACS can be found in the method shown in Example 5.

### Affinity for target proteins

In some embodiments, the bispecific binding arm A and binding arm B of the present invention have a high level of affinity for EGFR and c-Met antigens, respectively.

In some embodiments, bio-layer interferometry (BLI) can be used to detect the affinity of the bispecific antibody of the present invention for binding to EGFR and c-Met target proteins.

In some embodiments, in BLI monitoring, binding arm A in the bispecific antibody of the present invention binds EGFR with an affinity of KD(M) equal to or 1.05E-7, or preferably less than 8.45E-8, more preferably less than 6.95E-8, even more preferably less than 4.65E-8, even more preferably less than 3.35E-8, most preferably less than 2.15E-8.

In some embodiments, BLI affinity determination results show that the bispecific antibody of the present invention, such as BT461-DB, at a concentration of 5 µg/mL, binds to human EGFR with a dissociation constant KD(M) equal to or less than 2.262E-08, or preferably equal to or less than 2.52E-08, or more preferably equal to or less than 2.42E-08, or even more preferably equal to or less than 2.35E-08, or most preferably less than 2.20E-08.

In some embodiments, in BLI detection, binding arm B in the bispecific antibody of the present invention binds to human c-Met with an affinity of KD(M) equal to or less than 2.45E-9, or less than 2.25E-9, or preferably less than 1.95E-9, or preferably less than 1.75E-9, or preferably less than 1.55E-9, or more preferably less than 1.40E-9, or even more preferably less than 9.96E-10, or even more preferably less than 8.85E-10, or most preferably less than 7.62E-10.

In some embodiments, the bispecific antibody BT461 of the present invention binds to human c-Met with a dissociation constant (KD) of KD(M) equal to or less than 1.20E-09, equal to or less than 8.12E-10, equal to or less than 7.82E-10, less than 7.62E-10.

An exemplary BLI detection method is as shown in Example 6.

In some embodiments, the EGFR binding arm of the exemplary bispecific antibody BT461-DB molecule of the present invention binds EGFR with a KD(M) that is significantly higher than the KD(M) with which the c-Met binding arm binds c-Met.

In some embodiments, the ratio of the affinity of the c-Met binding arm for c-Met to the affinity of the EGFR binding arm for EGFR in the exemplary bispecific antibody BT461-DB molecule of the present invention exceeds nearly 20-fold.

### Affinity of Fc for receptor proteins

In some embodiments, the Fc of the exemplary bispecific antibody BT461-DB molecule of the present invention has high binding affinity to receptor proteins comparable to that of amivantamab.

In some embodiments, the receptor proteins include FcγRI (CD64), FcγRIIIA (CD16a-F176), FcγRIIIA (CD16a-V176), FcγRIIIB (CD16b), FcγRIIA (CD32a), FcγRIIB/C (CD32b/c), Fc Rn and C1q.

In some embodiments, BLI method is used to determine the binding affinity of Fc for receptor proteins.

Bispecific antibodies can also serve as mediators for redirecting effector mechanisms to disease-related tissues such as tumors.

In some embodiments, the binding affinity KD(M) of the Fc of BT461-DB of the present invention for receptor protein FcγRIIIA (CD16a-V176) as measured by BLI is approximately not higher than 2.62E-8.

In some embodiments, the binding affinity KD(M) of the Fc of BT461-DB of the present invention for receptor protein FcγRIIIA (CD16a-F176) as measured by BLI is approximately not higher than 3.26E-7.

In some embodiments, the binding affinity KD(M) of the Fc of BT461-DB of the present invention for receptor protein FcγRIIIB (CD16b) as measured by BLI is approximately not higher than 1.416E-5.

In some embodiments, the binding affinity KD(M) of the Fc of BT461-DB of the present invention for receptor protein FcγRIIA (CD32a) as measured by BLI is approximately not higher than 1.50E-6.

In some embodiments, the binding affinity KD(M) of the Fc of BT461-DB of the present invention for receptor protein FcγRIIB/C (CD32b/c) as measured by BLI is approximately not higher than 1.63E-6.

In some embodiments, the binding affinity KD (M) of the Fc of BT461-DB of the present invention for receptor protein FcγRI (CD64) as measured by BLI is approximately not higher than 8.95E-9.

In some embodiments, the binding affinity KD(M) of the Fc of BT461-DB of the present invention for receptor protein FcRn as measured by BLI is approximately not higher than 2.25E-7.

In some embodiments, the binding affinity KD(M) of the Fc of BT461-DB of the present invention for receptor protein C1q as measured by BLI is approximately not higher than 1.09E-8.

### Inhibition of EGFR autophosphorylation and c-Met autophosphorylation

In some embodiments, the BT461 of the present invention is capable of inhibiting EGFR autophosphorylation and c-Met autophosphorylation.

In some embodiments, the change in EGFR and c-Met autophosphorylation levels can be determined by Western blotting to investigate whether the anti-c-Met/EGFR bispecific antibody can inhibit the c-Met and EGFR signaling pathways.

Exemplarily, the results of chemiluminescence system development after Western blot analysis show that under conditions of 100 ng/mL EGF-mFC + 100 ng/mL HGF-his, after treating MKN45 cells with 250 µg/mL IgG1, antibody amivantamab, BT461-KIH, and BT-461 DB for a period, BT461-KIH, BT461-DB, and amivantamab can significantly inhibit p-c-Met (Y1234/1235) phosphorylation levels and slightly inhibit p-EGFR (Y1173) phosphorylation levels.

### Mediating the internalization degradation of target protein

In some embodiments, the exemplary antibody BT461-DB with a DuoBody structure of the present invention is capable of mediating and promoting the internalization and degradation of target proteins EGFR and c-Met.

In some embodiments, the internalization level mediated by the anti-c-Met antibody arm of the exemplary antibody BT461-DB of the present invention is higher than that mediated by the anti-EGFR antibody arm. In some embodiments, the level of target protein internalization mediated by the anti-c-Met binding arm and anti-EGFR binding arm of the exemplary antibody BT461-DB of the present invention can be evaluated by incubating the bispecific antibody of the present invention with a fluorescently labeled secondary antibody and then detecting with flow cytometry, using the obtained EC50 (ng/mL) value (from PE fluorescence excitation and detection) for assessment.

In some embodiments, the exemplary antibody BT461-DB of the present invention has a stronger ability to promote target protein internalization than the anti-EGFR binding arm. In some embodiments, the exemplary antibody BT461-DB of the present invention has a slightly weaker ability to promote target protein internalization compared to the anti-c-Met binding arm.

In some embodiments, the EC50 for promoting target protein internalization by the exemplary antibody BT461-DB of the present invention is approximately not higher than 700 ng/mL, or preferably approximately not higher than 650 ng/mL, or most preferably approximately not higher than 590 ng/mL.

In some embodiments, the EC50 for promoting target protein internalization by the exemplary antibody BT461-DB-c-Met binding arm of the present invention is approximately not higher than 420 ng/mL, or preferably approximately not higher than 350 ng/mL, or most preferably approximately not higher than 268 ng/mL.

In some embodiments, the EC50 for promoting target protein EGFR internalization by the exemplary antibody BT461-DB-EGFR binding arm of the present invention is approximately not higher than 680 ng/mL, or preferably approximately not higher than 650 ng/mL, or most preferably approximately not higher than 590 ng/mL.

### Mediating the antibody-dependent cellular cytotoxicity (ADCC)

In some embodiments, the EGFR/c-Met bispecific antibody of the present invention, such as BT461-DB, is capable of mediating antibody-dependent cellular cytotoxicity (ADCC).

In some embodiments, the present invention uses the ADCC Reporter Bioassay method to detect the ADCC biological activity of the antibody of the present invention.

In some embodiments, the EC50 value for ADCC mediated by the EGFR/c-Met bispecific antibody of the present invention, such as BT461-DB, against CHOK1-EGFR target cells is approximately not higher than 300 ng/mL, or approximately not higher than 280 ng/mL, or approximately not higher than 260 ng/mL, or approximately not higher than 208 ng/mL, or approximately not higher than 188 ng/mL.

In some embodiments, the EC50 value for ADCC mediated by the EGFR/c-Met bispecific antibody of the present invention, such as BT461-DB, against CHOK1-c-Met target cells is approximately not higher than 1290 ng/mL, or approximately not higher than 260 ng/mL, or approximately not higher than 238 ng/mL, or approximately not higher than 208 ng/mL, or approximately not higher than 188 ng/mL, or approximately not higher than 168 ng/mL.

In some embodiments, the EC50 value for ADCC mediated by the EGFR/c-Met bispecific antibody of the present invention, such as BT461-DB, against EGFR and c-Met double-positive tumor cells is approximately not higher than 156 ng/mL, preferably approximately not higher than 124.48 ng/mL, more preferably approximately not higher than 89.48 ng/mL, even more preferably approximately not higher than 72.18 ng/mL, most preferably approximately not higher than 52.18 ng/mL.

In some embodiments, the level of ADCC mediated by the EGFR/c-Met bispecific antibody of the present invention against double-positive tumor cells is comparable between the two binding arms.

### In vitro inhibition of tumor cell proliferation

In some embodiments, the example of the EGFR/c-Met bispecific antibody of the present invention can inhibit tumor cell proliferation *in vitro.*

In some embodiments, the EGFR/c-Met bispecific antibody of the present invention, such as BT461-DB, can inhibit the proliferation of EGFR-positive tumor cells and/or c-Met-positive tumor cells *in vitro.*

In some embodiments, the EGFR/c-Met bispecific antibody of the present invention, such as BT461-DB, can inhibit the proliferation of tumor cells expressing HGF *in vitro.*

In some exemplary embodiments, the EGFR/c-Met bispecific antibody of the present invention can inhibit the proliferation of human pancreatic cancer cells *in vitro.*

In some exemplary embodiments, the EGFR/c-Met bispecific antibody of the present invention can inhibit the proliferation of human non-small cell lung cancer EGFR mutant cells *in vitro.*

In some embodiments, the tumor cell line is HCC827-HGF.

In some embodiments, for example, the effect of the antibody of the present invention on the proliferation of these cell lines can be detected using the CellCounting-Lite^{®}3D kit, and the synergistic inhibitory level of the EGFR/c-Met bispecific antibody of the present invention on tumor cell proliferation can be evaluated by comparing the level of decrease in tumor cell viability induced by a mixture comprising the EGFR/c-Met bispecific antibody (e.g., BT461-DB and BT461-KIH) and two monospecific antibodies (one binding EGFR and the other binding c-Met).

In some exemplary embodiments, under the condition of tumor cell proliferation inhibition by the bispecific molecule of the present invention, the cell viability of tumor cells is comparable to the measured value under amivantamab inhibition, and the post-inhibition tumor cell viability with BT461-DB is comparable to that with BT461-KIH. The ability of both BT461-DB and BT461-KIH to inhibit tumor cell activity is stronger than the combination of their related EGFR monoclonal antibody and anti-c-Met monoclonal antibody (e.g., Nimotuzumab & LY358-B12), indicating a synergistic effect of BT461-DB in inhibiting tumors *in vitro.*

### In vitro inhibition of proliferation of cell strain with acquired resistance to TKI

In some embodiments, the bispecific molecule of the present invention, such as BT461-DB, can significantly inhibit the proliferation of TKI-resistant tumor cells.

In some embodiments, the bispecific molecule of the present invention, such as BT461-DB, can significantly inhibit the proliferation of TKI-resistant tumor cells expressing HGF.

In some embodiments, the bispecific molecule of the present invention, such as BT461-DB, can significantly inhibit the proliferation of tumor cells HCC827-HGF-TKI resistant cells at multiple gradient concentrations.

In some embodiments, the bispecific molecule of the present invention, such as BT461-DB, can significantly inhibit the proliferation of tumor cells HCC827-HGF-osimertinib resistant cells at multiple gradient concentrations, and its inhibitory level on HCC827-HGF cell proliferation is comparable to that of BT461-KIH and amivantamab.

In some embodiments, the bispecific molecule of the present invention, such as BT461-DB, can significantly inhibit the proliferation of tumor cells HCC827-HGF-osimertinib resistant cells in the concentration range of 28-550 µg/mL, and its inhibitory level on HCC827-HGF cell proliferation is comparable to that of BT461-KIH and amivantamab.

In some embodiments, the efficacy of the antibody BT461-DB of the present invention in inhibiting the proliferation of tumor cells HCC827-HGF-osimertinib resistant cells is stronger than the c-Met binding arm of BT461-DB, and stronger than the combination of anti-EGFR monoclonal antibody and anti-c-Met monoclonal antibody, indicating that the anti-EGFR/c-Met dual-target antibody of the present invention has a synergistic effect in inhibiting the proliferation of osimertinib-resistant tumor cells *in vitro.*

### Having therapeutic effect of inhibiting tumor growth in vivo

In some embodiments, the bispecific antibody of the present invention as disclosed herein can inhibit the growth of EGFR-positive tumors and c-Met-positive tumors *in vivo.*

In some exemplary embodiments, the exemplary antibody BT461-DB of the present invention has efficacy in inhibiting tumor growth in a mouse xenograft model of human non-small cell lung cancer cells.

In some exemplary embodiments, the administration period for antibody BT461-DB in a mouse xenograft model of HCC827-HGF cells is preferably 21 days.

In some exemplary embodiments, antibody BT461 has efficacy in inhibiting tumor growth in an NSG mouse xenograft model of human pancreatic cancer cells.

The EGFR/c-Met bispecific antibody BT461-DB of the present invention and BT461-KIH have efficacy in inhibiting tumor growth at administering doses of 1.5~8 mg/kg.

In some exemplary embodiments, at an administering dose of 2 mg/kg, the TGI (tumor growth inhibition ratio) of BT461-DB is 67.7%.

In some exemplary embodiments, at an administering dose of 5 mg/kg, the TGI (tumor growth inhibition ratio) of BT461-DB is 122.9%.

### Pharmaceutical composition

The present invention also provides a pharmaceutical composition comprising the bispecific antibody of the present invention as disclosed herein and a pharmaceutically acceptable excipient, for use in treating a subject having, or at risk of developing, an EGFR-positive, c-Met-positive, or EGFR/c-Met-positive tumor.

In some embodiments, the pharmaceutically acceptable excipient includes, but is not limited to, non-toxic solid, semi-solid, or liquid fillers, diluents, encapsulating materials, or any type of formulation auxiliary.

In some embodiments, appropriate fluidity can be maintained by, for example, using coatings (e.g., lecithin), by maintaining the desired particle size in the case of dispersions, and by using surfactants, stabilizers, cryoprotectants, or antioxidants.

In some embodiments, the pharmaceutical composition comprises a composition of the EGFR/c-Met bispecific antibody as provided by the present invention, such as BT461-DB, and further comprises one or more antioxidants that will reduce oxidation of the antibody or antigen-binding fragment, this reduction in oxidation will prevent oxidation of the antibody or antigen-binding fragment, extend its shelf life and/or improve its efficacy, prevent or reduce loss of binding affinity, thereby improving antibody stability and maximizing shelf life. In certain embodiments, the present invention provides a composition comprising one or more antibodies disclosed herein and one or more antioxidants such as methionine. In certain embodiments, the antioxidant is methionine.

In some embodiments, the carrier can be a solvent or dispersion medium comprising, for example, water, ethanol, polyols (e.g., glycerol, propylene glycol, and liquid polyethylene glycol, etc.), or suitable mixtures thereof.

In some embodiments, the pharmaceutical composition is prepared by dissolving or dispersing an effective amount of the antibody of the present invention in a pharmaceutically acceptable carrier or aqueous medium.

In some embodiments, the pharmaceutical forms of the pharmaceutical composition suitable for injectable use include sterile aqueous solutions or dispersions; and sterile powders for the extemporaneous preparation of sterile injectable solutions or dispersions.

In some embodiments, the pharmaceutical composition is a dry, particularly lyophilized, composition which, upon addition of sterile water or physiological saline, allows for the production of an injectable solution.

In some embodiments, the pharmaceutically acceptable carrier may comprise, for example, aqueous vehicles such as sodium chloride injection, Ringer's injection, isosmotic dextrose injection, sterile water injection, or dextrose and lactated Ringer's injection.

Injectable pharmaceutical compositions can be prepared in any conventional form, such as liquid solutions, suspensions, or solid forms suitable for producing liquid solutions, suspensions, or emulsions. Injectable formulations may comprise: (1) sterile and/or pyrogen-free solutions ready for immediate injection; (2) sterile dry soluble products that are combined with a solvent only immediately prior to use, such as lyophilized powder; (3) sterile suspensions ready for immediate injection; (4) sterile dry insoluble products that are combined with a vehicle only immediately prior to use; (5) as well as sterile and/or pyrogen-free emulsions. The solutions may be aqueous or non-aqueous.

In certain embodiments, unit dosage parenteral formulations may be packaged in ampoules, vials, or syringes with needles. All formulations for parenteral administration should be sterile and pyrogen-free, as is known and practiced in the art. In certain embodiments, sterile lyophilized powder is prepared by dissolving the antibody or antigen-binding fragment as disclosed in the present invention in a suitable solvent.

In some embodiments, the solvent may comprise a buffer, such as citrate, sodium phosphate or potassium phosphate, or other such buffers known to those skilled in the art. In some embodiments, the buffer is approximately neutral pH. The sterile filtered solution is then added and lyophilized under standard conditions known to those skilled in the art to obtain the desired formulation. In some embodiments, the resulting solution will be distributed into vials for lyophilization. The lyophilized powder can be stored under appropriate conditions, such as storage at about 4°C to room temperature. The lyophilized powder is reconstituted with Water for Injection to obtain a formulation for parenteral administration. In some embodiments, for reconstitution, sterile and/or pyrogen-free water or other suitable liquid carrier is added to the lyophilized powder. The precise amount depends on the selected therapy to be administered and can be determined empirically.

In some embodiments, the pharmaceutical composition of the present invention further comprises one or more additional therapeutic means or therapeutic action groups for co-administration in addition to the anti-EGFR/c-Met bispecific antibody described in the present invention, for example, another therapeutic agent, such as a chemotherapeutic agent or an anticancer drug combination.

### Drug use in combination

In some embodiments, the antibody disclosed in the present invention may be administered alone or in combination with one or more additional treatment means or pharmaceutical agents. For example, the antibody disclosed in the present invention may be administered in combination with another therapeutic agent, for instance, a chemotherapeutic agent or an anti-cancer drug.

In some embodiments, the present invention provides a combination drug use treatment method comprising administering to a subject in need thereof a therapeutic composition comprising any aforementioned anti-EGFR/c-Met bispecific antibody and one or more additional therapeutically active ingredients.

In certain embodiments, the anti-EGFR/c-Met bispecific antibody disclosed in the present invention can be administered simultaneously with the one or more additional therapeutic agents, and the antibody or antigen-binding fragment and the additional therapeutic agent may be administered as part of the same pharmaceutical composition. In certain embodiments, administration of the antibody or antigen-binding fragment disclosed in the present invention before or after another agent is also considered administration "in combination" with the agent, even if the anti-EGFR/c-Met bispecific antibody of the present invention and the other agent are administered via different routes. Where possible, the additional therapeutic agents administered in combination with the antibody disclosed in the present invention are administered according to the schedule listed in the product information sheet of the additional therapeutic agent or a protocol well-known in the art.

The present invention comprises, in a pharmaceutical composition, the aforementioned anti-EGFR/c-Met antibody or antigen-binding fragment thereof and an anti-FGFR2 antibody ADC co-formulated with one or more additional therapeutically active ingredients as described elsewhere in the present invention.

Although antibody molecules can be administered alone, the anti-EGFR/c-Met bispecific antibody molecule of the present invention is typically administered in the form of a pharmaceutical composition, which can comprise at least one component other than the anti-EGFR/c-Met bispecific antibody molecule of the present invention. Therefore, another aspect of the present disclosure also provides methods including formulating the antibody molecule into a pharmaceutical composition.

In some embodiments, the anti-EGFR/c-Met bispecific antibody or antigen-binding fragment thereof of the present invention can be co-formulated and/or administered in combination with one or more additional targeted specific therapeutic antibody components selected from the group consisting of: such as another FGFR2 antagonist, an antagonist of another EGFR family member such as ErbB3 or ErbB4, an antagonist anti-ErbB2, such as of ErbB3 or ErbB4, MET antagonist, EGFR antagonist, IGF1R antagonist, IGF1R antagonist, DGFR-α inhibitor, PDGFR-β inhibitor or small molecule kinase inhibitor, VEGF-inhibitor, DLL4 antagonist.

In some embodiments, the additional therapeutically active ingredient is a second specificity antibody.

In some embodiments, the second specificity antibody includes but is not limited to MSLN antagonist (for example, anti-MSLN antibody), CA9 antagonist (for example, anti-CA9 antibody), uroplakin antagonist (for example, uroplakin antagonist). LGR5 antagonist (for example, anti-LGR5 antibody), monovalent CD20 antagonist (for example, monovalent anti-CD20 antibody such as rituximab), CD20xCD3 bispecific antibody, PD-1 blocker (for example anti-PD-1 antibody such as pembrolizumab or nivolumab) and the like.

In some embodiments, other therapeutic agents that can be advantageously administered in combination with the antibody provided by the present invention also include, for example, tamoxifen, aromatase inhibitors and cytokine inhibitors, including small molecule cytokine inhibitors and antibodies that bind to cytokines such as including but not limited to IL-1, IL-2, IL-3, IL-4, IL-5, IL-6 or their respective receptors.

In some embodiments, additional therapeutic agents that can be combined with any anti-FGFR2 antibody or antigen-binding fragment thereof described in the present invention (including anti-FGFR2 antibody ADC) for treatment include one or more chemotherapeutic agents.

In some embodiments, examples of chemotherapeutic agents that can be combined with any anti-EGFR/c-Met bispecific antibody or antigen-binding fragment thereof described in the present invention, for example BT461-DB, for treatment include alkylating agents, ethylenimines, nitrosoureas, antibiotics, antimetabolites, purine analogs, pyrimidine analogs, alkyl sulfonates, folate supplements, aldophosphamide glycosides, taxanes, platinum analogs, and pharmaceutically acceptable salts, acids or derivatives of any of the aforementioned.

In some embodiments, the anti-FGFR2 antibody or antigen-binding fragment thereof of the present invention, including anti-FGFR2 antibody ADC, can also be administered in combination and/or co-formulated with antiviral agents, antibiotics, analgesics, corticosteroids, steroids, oxygen, antioxidants, COX inhibitors, cardioprotective agents, metal chelators, IFN-γ and/or NSAIDs.

### For treating diseases related to EGFR and c-Met overexpression

The EGFR/c-Met bispecific antibody of the present invention or an ADC comprising the same can be used for modulating, treating, alleviating, helping to prevent the occurrence of cell, tissue, organ, tumor or human disease or specific lesion or reducing its symptoms.

In some embodiments, the method for administering the drug comprises administering to a patient an effective amount of a drug comprising the EGFR/c-Met bispecific antibody of the present invention, such that the growth or metastasis of a tumor or cancer cell expressing EGFR and/or c-Met is inhibited.

In some embodiments, the method for administering the drug comprises administering to a patient in need thereof a therapeutically effective amount of the EGFR/c-Met bispecific antibody of the present invention for a time sufficient to treat cancer.

In some embodiments, the cancer resists treatment with one or more EGFR inhibitors or has acquired resistance to treatment with one or more EGFR inhibitors.

In some embodiments, the patient may be treated with the anti-EGFR antibody Nimotuzumab, or has acquired resistance to the treatment.

Detection of elevated c-Met expression levels or increased c-Met activity, such as caused by elevated levels of circulating HGF, activating mutations of the c-Met gene, or c-Met gene amplification, can identify patients with resistance to EGFR inhibitors.

In some embodiments, wherein the cancer is associated with EGFR mutation, residue deletion, EGFR amplification or c-Met amplification or mutation.

The EGFR/c-Met bispecific antibody of the present invention can be used to treat any disease or disorder characterized by abnormal activation or production of EGFR, c-Met, EGF, soluble EGFR, soluble c-Met, or other EGFR ligands or HGF, or disorders related to EGFR or c-Met expression, which may or may not involve malignancy or cancer, wherein abnormal activation and/or production of EGFR, c-Met, EGF or other EGFR ligands or HGF occurs in cells or tissues of a subject suffering from or predisposed to the disease or disorder.

The EGFR/c-Met bispecific antibody of the present invention, comprising binding arm A and binding arm B, can be used to treat tumors, comprising cancers and benign tumors.

In some embodiments, exemplary cancers suitable for treatment by the EGFR/c-Met bispecific antibody of the present invention comprise cancers that overexpress EGFR and/or c-Met and are cancers associated with increased EGFR activity and/or expression levels.

In some embodiments, the types of the increased EGFR activity and/or expression levels comprise but are not limited to EGFR activating mutations, EGFR gene amplification, or ligand-mediated EGFR activation.

In some embodiments, the causes of the increased c-Met activity and/or expression levels comprise but are not limited to c-Met activating mutations, c-Met gene amplification, or HGF-mediated c-Met activation, or mutant KRAS.

In some embodiments, EGFR activating mutations that may be associated with cancer cause an increase in at least one biological activity related to EGFR, the increase in biological activity comprising but not limited to increased tyrosine kinase activity, formation of receptor homodimers and heterodimers, and enhanced ligand binding.

In some embodiments, c-Met activating mutations that may be associated with cancer cause an increase in at least one biological activity related to c-Met, the increase in raised c-Met-related biological activities comprising but not limited to tyrosine kinase activity, formation of receptor homodimers and heterodimers, and enhanced ligand binding, etc.

In another embodiment of the present invention, an experimental mouse model has an NSCLC tumor or tumor metastasis with an activating EGFR mutation or EGFR gene amplification, and a method for administering the EGFR/c-Met bispecific antibody of the present invention to treat the tumor disease model mouse comprises administering to the model mouse a therapeutically effective amount of the EGFR/c-Met bispecific antibody of the present invention.

Another aspect of the present invention provides a method for treating a cancer patient, the method comprising administering to a patient in need thereof a therapeutically effective amount of the EGFR/c-

Met bispecific antibody of the present invention for a time sufficient to treat cancer, wherein the cancer is associated with EGFR mutation, EGFR amplification or c-Met amplification.

In some embodiments, the cancer is associated with wild-type EGFR and wild-type c-Met.

In some embodiments, the cancer is associated with wild-type EGFR and amplification of c-Met.

In some embodiments, the patient suffers from NSCLC associated with amplification of EGFR and wild-type c-Met.

In some embodiments, the patient suffers from NSCLC associated with EGFR amplification and c-Met amplification.

In some embodiments, the patient is treated with the EGFR/c-Met bispecific antibody of the present invention or an ADC thereof. The BT461-MMAE, BT461-Dxd and BT461-exatecan of the present invention show efficacy in *in vivo* tumor animal models when the tumor is associated with L858R, T790M, del(E746, A750) EGFR, EGFR amplification, wild-type c-Met and/or c-Met amplification.

In one embodiment, the EGFR/c-Met bispecific antibody of the present invention or an ADC thereof can be used in the manufacture of a medicament for treating non-small cell lung cancer (NSCLC).

In some embodiments, the cells of NSCLC have an epithelial phenotype.

In some embodiments, the NSCLC has acquired resistance to an EGFR monoclonal antibody.

In one embodiment, the EGFR monoclonal antibody is Nimotuzumab.

The present invention also provides use of the EGFR/c-Met bispecific antibody of the present invention or an ADC thereof for the manufacture of a medicament for treating a subject suffering from a tumor or at risk of suffering from the tumor, wherein the method for administering the medicament comprises administering to a subject in need thereof a therapeutically effective amount of the antibody or pharmaceutical composition as disclosed herein, wherein the tumor is preferably an EGFR or c-Met single-positive or EGFR/c-Met double-positive tumor.

Before starting the treatment, the method preferably further comprises determining whether the subject suffers from such an EGFR, c-Met or EGFR/c-Met positive tumor.

The bispecific antibody of the present invention can be applied to a wide range of cancers. It is suitable for exemplary cancers treated by the bispecific molecule of the present invention such as the EGFR/c-Met bispecific antibody of the present invention.

In some exemplary embodiments, the cancer is non-small cell lung cancer.

In some exemplary embodiments, the cancer is gastric cancer.

In some exemplary embodiments, the cancer is pancreatic cancer.

### Application in the manufacture of medicaments

In one aspect, the present invention provides use of an anti-EGFR/c-Met bispecific antibody or a pharmaceutical composition comprising the same in the manufacture of a medicament for treating cancer or tumor.

One aspect of the present invention is to provide use of the bispecific antibody of the present invention for the manufacture of a medicament for inhibiting the growth or proliferation of cells expressing EGFR and/or c-Met, wherein the method for administering the medicament comprises contacting a patient's cells with the EGFR/c-Met bispecific antibody of the present invention.

In some preferred embodiments, the tumor, preferably EGFR/c-Met positive cancer, preferably a patient population having an EGFR monoclonal antibody resistant phenotype or tumor/cancer.

In some preferred embodiments, the subject is preferably a subject suitable for antibody therapy using an EGFR-specific antibody such as Nimotuzumab.

Co-expression of EGFR and c-Met is associated with many cancer types and bispecific antibody molecules targeting these two molecules, conjugates comprising such bispecific antibody molecules provide broad clinical opportunities across multiple indications. Therefore, the antibody molecules or antibody-drug conjugates described herein can be used in therapeutic applications, particularly for the treatment of cancer.

The bispecific antibody molecules or pharmaceutical compositions described herein can be used in methods for treating human EGFR and/or c-Met positive-related tumors or cancers.

Relevant aspects of the present disclosure provide:
(i) The bispecific antibody molecule described herein, for use as a medicament;
(ii) The bispecific antibody molecule described herein for use in a method of treating a disease or condition;
(iii) Use of the bispecific antibody molecule described herein in the manufacture of a medicament for treating a disease or condition; and,
(iv) A method of treating a disease or condition in an individual, wherein the method comprises administering to the individual a therapeutically effective amount of the antibody molecule or conjugate described herein.

In some embodiments, treatment can be any treatment or therapy that achieves certain desired therapeutic effects, such as inhibiting or delaying the progression of a condition, and comprises treatment reducing the progression rate, halting the progression rate, improving the condition. The condition of the condition, cure or alleviation (whether partial or complete), prevention, amelioration, delay, reduction or arrest of one or more symptoms and/or signs of the condition, or prolonging the survival of the individual or patient, beyond the lifespan expected in the absence.

In some embodiments, the described treatment methods can comprise administering to the individual at least one additional therapeutic agent in addition to the bispecific antibody molecule of the present invention.

In some embodiments, the anti-EGFR/c-Met bispecific antibody molecule of the present invention can be administered to an individual alone or in combination with one or more other treatments, wherein the additional treatment can be administered to the individual simultaneously, sequentially, or separately from the administration of the antibody molecule or conjugate. When the additional treatment is administered simultaneously with the antibody molecule or conjugate, the antibody molecule and the additional treatment can be administered to the individual as a combined preparation. For example, the additional therapy can be a known therapy or therapeutic agent for the disease to be treated.

In preferred examples, the antibody molecule described herein or a pharmaceutical composition comprising the same can be used in a method for treating cancer.

Cancer can be characterized by abnormal proliferation of malignant cancer cells.

In some embodiments, the tumor is a tumor of abnormal proliferation of EGFR-positive malignant cancer cells or c-Met-positive malignant cancer cells.

In some embodiments, the tumor is a tumor of abnormal proliferation of c-Met-positive malignant cancer cells.

In some embodiments, the tumor is EGFR/c-Met double-positive tumor cells.

EGFR-positive tumors are typically tumors with EGFR activating mutations. EGFR activating mutations are EGFR mutations that lead to activation of the EGF/EGFR signaling pathway. EGFR activating mutations may be important for the cancerous state of the tumor. One reason why such tumors can resist EGFR-targeted therapy is through activation of the HGF/c-Met signaling pathway. The tumor can also be an HGF-related tumor. Activation of the c-Met/HGF signaling pathway is one way in which EGFR-positive tumors can escape treatment with EGFR-targeted therapy. The antibody of the present invention is particularly suitable for treating HGF-related tumors or HGF-dependent tumors, wherein activation of the c-Met/HGF signaling pathway is associated with the presence or excess of HGF. Compared to HGF/c-Met signaling-negative tumor cells, positive tumor cells with simultaneous activation of the HGF/c-Met signaling pathway have a more superior selective growth advantage.

In some embodiments, cancers treated using the antibody molecule described herein can be selected from the group consisting of: lung cancer (e.g., non-small cell lung cancer (NSCLC)), pancreatic cancer, breast cancer, colorectal cancer, kidney cancer, gastric cancer, head and neck cancer, ovarian cancer or glioblastoma.

In one embodiment, the cancer to be treated is non-small cell lung cancer (NSCLC).

In one embodiment, the cancer is gastric cancer.

In some embodiments, the cancer is pancreatic cancer.

In some embodiments, the treatment can include inhibiting cancer growth, including complete cancer remission, and/or inhibiting cancer metastasis, as well as inhibiting cancer recurrence. Cancer growth generally refers to any of a variety of indicators signaling changes within the cancer towards a more developed form. Features disclosed in the foregoing description, or appended claims, or drawings, are expressed in their specific form or as means for performing the disclosed function, or as a method or process for obtaining the disclosed results (as the case may be). The present disclosure can be realized in its different forms using individual or in any combination of these features.

While the present disclosure has been described in connection with the above exemplary examples, many equivalent modifications and variations will be apparent to those skilled in the art when given the present disclosure. Therefore, the exemplary examples disclosed above are considered illustrative and not restrictive. Various changes can be made to the described examples without departing from the spirit and scope of the present disclosure.

To avoid any doubt, any theoretical explanations provided herein are provided to enhance the reader's understanding. The inventors do not wish to be bound by any of these theoretical explanations.

Any section headings used herein are for organizational purposes only and should not be construed as limiting the subject matter described.

Throughout this specification, including the appended claims, unless the context requires otherwise, the words "comprise" and " comprise" and variations such as "comprises", "comprising" and " comprising" will be understood to imply the inclusion of a stated integer or step or group of integers or steps, but not the exclusion of any other integer or step or group of integers or steps.

It must be noted that, as used in the specification and appended claims, the singular forms "a", "an", and "the" include plural referents unless the context clearly dictates otherwise. Ranges may be expressed herein as from "about" one particular value and/or to "about" another particular value. When expressing such a range, another example includes from the one particular value and/or to the other particular value. Similarly, when values are expressed as approximations by use of the antecedent "about," it will be understood that the particular value forms another example. The term "about" in relation to a numerical value is optional and means, e.g., +/-10%.

### Specific Examples

The present invention is further illustrated below by way of examples, without thereby limiting the present invention to the scope of the examples described.

Experimental methods whose detailed conditions are not specified in the following embodiments are performed under conventional conditions or as recommended by the manufacturer of the raw material or commodity, or as described in biotechnology textbooks such as molecular cloning, laboratory manuals, cold spring harbor laboratories, methods of contemporary molecular biology, cell biology, etc. Reagents whose specific source is not indicated are conventional reagents purchased through commercial channels.

The reagents and raw materials used in the present invention are commercially available.

### Example 1. Construction, Expression, Assembly and Purification of Anti-c-Met/EGFR Bispecific Antibody BT461-DB

### 1.1 Construction of BT461-DB

The construction of Anti-c-Met/EGFR Bispecific Antibody BT461-DB is based on Genmab's DuoBody platform technology. Mutations (F405L, K214R, D356E, L358M) have been performed on the heavy chain of anti-EGFR antibody and mutations (K409R, K214R, D356E, L358M) have been performed on the heavy chain of anti-c-Met antibody by genetic engineering technology. Next, the nucleotide sequences of the light chain and heavy chain of the anti-c-Met or anti-EGFR antibody are respectively constructed into the PEE6.4 and PEE12.4 expression vectors to obtain the light chain and heavy chain plasmids of the anti-c-Met or anti-EGFR antibody. Then, the light chain and heavy chain plasmids are subjected to SalI/NotI enzyme digestion and ligation (using NEB), and the two nucleotide sequences are constructed into the same PEE expression vector (pHR plasmid), resulting in a plasmid that co-expresses the light chain and heavy chain of the anti-c-Met or anti-EGFR antibody.

The amino acid sequence of binding arm A of the bispecific antibody embodiment of the present invention is shown in Example 1, which comprises the light chain, heavy chain, variable region, and CDR sequences listed in Table 2 and Table 3. The amino acid sequence of binding arm B is shown in Example 1, which comprises the light chain, heavy chain, variable region, and CDR sequences listed in Table 4 and Table 5.

The amino acid sequences of the heavy chain and light chain of the anti-EGFR antibody: as shown in Table 2, the sequence numbers for its heavy chain, light chain, and the amino acid sequences of the comprised segments are shown in Table 1.

The amino acid sequences of the heavy chain and light chain of the anti-c-MET antibody: as shown in Table 3, the sequence numbers for the amino acid sequences of each segment of its heavy chain and light chain are shown in Table 1.

**Table 1. Sequence numbers of the amino acids of the binding arm component fragments and ligands of BT461 with Duobody structure (abbreviated as BT461 DB)**

| Domain | SEQ ID NO: of the amino acid sequence of the functional fragment comprised in the anti-EGFR binding arm A | SEQ ID NO: of the amino acid sequence of the functional fragment comprised in the anti-c-Met binding arm B |
|---|---|---|
| VL | 6 | 8 |
| CL | 12 | 14 |
| VH | 5 | 7 |
| CH | 11 | 13 |
| Fc (comprises mutations) | 9 | 10 |
| LC | 1 | 3 |
| HC | 2 | 4 |
| Single strand of binding arm | 4 | 15 |
| VHCDR1 | 19 | 25 |
| VHCDR2 | 20 | 26 |
| VHCDR3 | 21 | 27 |
| VLCDR1 | 22 | 28 |
| VLCDR2 | 23 | 29 |
| VLCDR3 | 24 | 30 |

**Table 2. Amino acid sequence of binding arm A**

| Domain | Anti-EGFR Binding arm A (BT41-DB-EGFR) |
|---|---|
| VLA | (SEQ ID NO:6) |
| CLA | (SEQ ID NO:12) |
| VHA |  (SEQ ID NO:5) |
| CHA | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKRVE PKSCDKTHTCPPCPAPELLGGPSVFLFPPKPKDTLMISRTPEVTCVVVDV SHEDPEVKFNWYVDGVEVHNAKTKPREEQYNSTYRVVSVLTVLHQDW LNGKEYKCKVSNKALPAPIEKTISKAKGQPREPQVYTLPPSREEMTKNQ VSLTCLVKGFYPSDIAVEWESNGQPENNYKTTPPVLDSDGSFLLYSKLT VDKSRWQQGNVFSCSVMHEALHNHYTQKSLSLSPGK (SEQ ID NO:11) |
| Fc(A) | EPKSCDKTHTCPPCPAPELLGGPSVFLFPPKPKDTLMISRTPEVTCVVVD VSHEDPEVKFNWYVDGVEVHNAKTKPREEQYNSTYRVVSVLTVLHQD WLNGKEYKCKVSNKALPAPIEKTISKAKGQPREPQVYTLPPSREEMTKN QVSLTCLVKGFYPSDIAVEWESNGQPENNYKTTPPVLDSDGSFLLYSKL TVDKSRWQQGNVFSCSVMHEALHNHYTQKSLSLSPGK(SEQ ID NO:9) |
| CH3(A) | GQPREPQVYTLPPSREEMTKNQVSLTCLVKGFYPSDIAVEWESNGQPEN NYKTTPPVLDSDGSFLLYSKLTVDKSRWQQGNVFSCSVMHEALHNHYT QKSLSLSPGK(SEQ ID NO:31) |
| LCA | DIQMTQSPSSLSASVGDRVTITCRSSQNIVHSNGNTYLDWYQQTPGKAP KLLIYKVSNRFSGVPSRFSGSGSGTDFTFTISSLQPEDIATYYCFQYSHVP WTFGQGTKLQITRTVAAPSVFIFPPSDEQLKSGTASVVCLLNNFYPREAK VQWKVDNALQSGNSQESVTEQDSKDSTYSLSSTLTLSKADYEKHKVYA CEVTHQGLSSPVTKSFNRGEC(SEQ ID NO:1) |
| HCA | QVQLQQSGAEVKKPGSSVKVSCKASGYTFTNYYIYWVRQAPGQGLEWI GGINPTSGGSNFNEKFKTRVTITADESSTTAYMELSSLRSEDTAFYFCTR QGLWFDSDGRGFDFWGQGTTVTVSSASTKGPSVFPLAPSSKSTSGGTAA LGCLVKDYFPEPVTVSWNSGALTSGVHTFPAVLQSSGLYSLSSVVTVPSS SLGTQTYICNVNHKPSNTKVDKRVEPKSCDKTHTCPPCPAPELLGGPSVF LFPPKPKDTLMISRTPEVTCVVVDVSHEDPEVKFNWYVDGVEVHNAKT KPREEQYNSTYRVVSVLTVLHQDWLNGKEYKCKVSNKALPAPIEKTISK AKGQPREPQVYTLPPSREEMTKNQVSLTCLVKGFYPSDIAVEWESNGQP ENNYKTTPPVLDSDGSFLLYSKLTVDKSRWQQGNVFSCSVMHEALHNH YTQKSLSLSPGK (SEQ ID NO:2) |

**Table 3. Exemplary CDR sequences of binding arm A**

| CDR region | CDR sequence and SEQ ID NO: |
|---|---|
| VLACDR1 | RSSQNIVHSNGNTYLD(SEQ ID NO:22) |
| VLACDR2 | KVSNRFS (SEQ ID NO:23) |
| VLACDR3 | FQYSHVPWT(SEQ ID NO:24) |
| VHACDR1 | NYYIY (SEQ ID NO:19) |
| VHACDR2 | GINPTSGGSNFNEKFKT (SEQ ID NO:20) |
| VHACDR3 | QGLWFDSDGRGFDF (SEQ ID NO:21) |

**Table 4. Amino acid sequence of binding arm B**

| Domain | Anti-c-Met binding arm B (BT461-DB-c-Met) |
|---|---|
| VLB | (SEQ ID NO:8 ) |
| CLB | (S EQ ID NO:14 ) |
| VHB | (SEQ ID NO:7) |
| CHB | ASTKGPSVFPLAPSSKSTSGGTAALGCLVKDYFPEPVTVSWNSGALTSGVHTFPA VLQSSGLYSLSSVVTVPSSSLGTQTYICNVNHKPSNTKVDKRVEPKSCDKTHTCP PCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEK TISKAKGQPREPQVYTLPPSREEMTKNQVSLTCLVKGFYPSDIAVEWESNGQPEN NYKTTPPVLDSDGSFFLYSRLTVDKSRWQQGNVFSCSVMHEALHNHYTQKSLS LSPGK (SEQ ID NO:13) |
| Fc(B) | EPKSCDKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCK VSNKALPAPIEKTISKAKGQPREPQVYTLPPSREEMTKNQVSLTCLVKGFYPSDI AVEWESNGQPENNYKTTPPVLDSDGSFFLYSRLTVDKSRWQQGNVFSCSVMHE ALHNHYTQKSLSLSPGK(SEQ ID NO:10) |
| CH3(B) | GQPREPQVYTLPPSREEMTKNQVSLTCLVKGFYPSDIAVEWESNGQPENNYKTT PPVLDSDGSFFLYSRLTVDKSRWQQGNVFSCSVMHEALHNHYTQKSLSLSPGK( SEQ ID NO:32) |
| LCB | DIQMTQSPSSLSASVGDRVTITCSVSSSVSSIYLHWYQQKPGKAPKLLIYSTSNLA SGVPSRFSGSGSGTDFTLTISSLQPEDFATYYCQVYSGYPLTFGGGTKVEIKRTVA APSVFIFPPSDEQLKSGTASVVCLLNNFYPREAKVQWKVDNALQSGNSQESVTE QDSKDSTYSLSSTLTLSKADYEKHKVYACEVTHQGLSSPVTKSFNRGEC(SEQ ID NO:3) |
| HCB | QVQLVQSGAEVKKPGASVKVSCKASGYTFTDYYMHWVRQAPGQGLEWMGRV NPNRRGTTYNQKFEGRVTMTTDTSTSTAYMELRSLRSDDTAVYYCARANWLD YWGQGTTVTVSSASTKGPSVFPLAPSSKSTSGGTAALGCLVKDYFPEPVTVSWN SGALTSGVHTFPAVLQSSGLYSLSSVVTVPSSSLGTQTYICNVNHKPSNTKVDKR VEPKSCDKTHTCPPCPAPELLGGPSVFLFPPKPKDTLMISRTPEVTCVVVDVSHE DPEVKFNWYVDGVEVHNAKTKPREEQYNSTYRVVSVLTVLHQDWLNGKEYK CKVSNKALPAPIEKTISKAKGQPREPQVYTLPPSREEMTKNQVSLTCLVKGFYPS DIAVEWESNGQPENNYKTTPPVLDSDGSFFLYSRLTVDKSRWQQGNVFSCSVM HEALHNHYTQKSLSLSPGK (SEQ ID NO:4) |

**Table 5. Exemplary CDR sequences comprised in binding arm B**

| | Binding Arm B |
|---|---|
| VLBCDR1 | SVSSSVSSIYLH(SEQ ID NO:28) |
| VLBCDR2 | STSNLAS (SEQ ID NO:29) |
| VLBCDR3 | QVYSGYPLT (SEQ ID NO:30) |
| VHBCDR1 | DYYMH (SEQ ID NO:25) |
| VHBCDR2 | RVNPNRRGTTYNQKFEG (SEQ ID NO:26) |
| VHBCDR3 | ANWLDY (SEQ ID NO:27) |

The encoding nucleic acids of binding arm A and binding arm B are shown in Table 6.

**Table 6. Encoding nucleic acids of the binding arms**

| Fragment of the binding arm | Encoding nucleic acid sequence number and nucleotide sequence |
|---|---|
| LCA | GATATCCAGATGACTCAGAGTCCAAGTTCTCTGTCAGCCTCCGTGGGCGAT CGAGTGACCATTACTTGCAGATCATCCCAGAACATCGTCCACTCTAATGGG AATACCTACCTGGACTGGTATCAACAGACCCCAGGGAAGGCCCCAAAGCTC CTGATCTACAAGGTGTCCAACAGGTTTAGCGGTGTGCCAAGCAGATTCTCA GGGAGTGGTTCTGGCACTGATTTTACCTTCACTATCTCCTCCTTGCAGCCCG AAGACATCGCCACTTATTATTGTTTCCAGTATTCCCACGTGCCTTGGACCTT TGGCCAGGGGACTAAGCTGCAAATTACTCGAACAGTGGCTGCCCCTTCCGT CTTTATCTTCCCCCCTTCAGATGAGCAGCTGAAATCCGGAACTGCCAGTGTA GTGTGCCTGCTGAATAACTTCTACCCTAGAGAGGCAAAGGTCCAGTGGAAG GTGGACAATGCCCTTCAAAGCGGAAACTCTCAGGAATCAGTCACAGAGCA GGACTCCAAGGATAGTACCTATTCTCTTTCTTCAACACTGACTCTGAGTAAA GCCGATTACGAGAAGCACAAAGTCTATGCATGTGAAGTGACTCATCAGGG ACTGTCTAGTCCAGTTACTAAATCTTTTAACAGGGGGGAATGT (SEQ ID NO:16) |
| HCA | CAAGTCCAACTCCAGCAGAGTGGTGCCGAAGTGAAAAAGCCTGGCAGCTC TGTCAAAGTCTCCTGTAAGGCTTCAGGCTACACTTTCACCAACTACTACATC TACTGGGTGCGGCAGGCTCCTGGTCAGGGCTTGGAATGGATAGGCGGTATC AACCCTACAAGTGGAGGTTCAAACTTCAACGAGAAGTTCAAGACCCGCGTT ACAATCACTGCTGACGAGAGCAGCACCACAGCTTATATGGAACTCAGTTCC CTGCGGTCTGAAGACACAGCCTTCTATTTCTGCACACGGCAGGGGCTTTGG TTTGATAGTGACGGTCGAGGGTTTGACTTCTGGGGACAAGGCACTACTGTA ACAGTGTCTTCAGCCTCCACTAAAGGCCCATCCGTGTTCCCTCTGGCTCCAA GTAGCAAGTCCACTAGCGGAGGGACAGCTGCACTGGGTTGCCTTGTTAAGG ATTATTTCCCCGAACCCGTCACAGTCTCTTGGAATAGTGGCGCCCTCACTTC CGGCGTCCATACCTTCCCAGCTGTGCTGCAGTCCTCTGGGCTCTACAGCCTG AGCAGCGTCGTTACTGTCCCATCTAGCAGTCTGGGAACCCAAACATACATA TGTAACGTCAATCACAAGCCTTCTAACACAAAAGTGGACAAGAGGGTAGA GCCCAAGAGTTGTGACAAAACACATACATGTCCTCCATGCCCAGCTCCTGA ACTTCTTGGAGGCCCATCTGTTTTCCTGTTTCCCCCTAAGCCAAAAGACACC TTGATGATTTCTCGTACCCCCGAAGTCACCTGCGTTGTGGTGGACGTATCAC ACGAGGACCCCGAAGTCAAGTTCAACTGGTATGTGGATGGCGTCGAAGTCC ACAACGCTAAGACAAAACCCCGTGAGGAGCAGTACAACAGTACCTATAGG GTGGTCAGTGTGCTTACCGTGCTTCACCAAGATTGGCTTAATGGGAAGGAG TACAAATGCAAGGTGTCCAACAAAGCACTGCCAGCACCAATCGAGAAGAC TATCAGTAAAGCTAAGGGGCAGCCCAGGGAGCCTCAAGTGTATACCCTTCC CCCTAGCCGGGAGGAGATGACAAAGAACCAAGTGAGCTTGACATGCCTCG TGAAAGGATTTTATCCTTCCGACATAGCAGTGGAGTGGGAAAGTAATGGGC AGCCCGAAAACAACTATAAGACCACCCCACCTGTTTTGGATAGCGATGGCA GTTTTCTGCTTTACTCTAAGCTGACCGTTGATAAAAGTAGATGGCAGCAGG GCAACGTTTTCAGTTGCTCCGTGATGCATGAAGCACTCCACAATCACTATA CACAGAAATCCCTGAGCCTGTCTCCAGGCAAA(SEQ ID NO:15) |
| LCB | GATATTCAGATGACTCAGTCCCCAAGCTCCTTGTCCGCCTCCGTTGGTGACC GGGTCACCATCACATGCTCCGTATCCTCCTCCGTCTCCTCTATCTATTTGCA CTGGTATCAGCAGAAGCCCGGTAAAGCCCCAAAACTGCTCATCTATAGTAC AAGCAATCTGGCCAGTGGAGTGCCTTCTCGCTTTAGCGGCAGCGGCTCAGG AACCGATTTTACACTGACCATCTCCTCCCTGCAGCCCGAAGATTTCGCCACA TATTATTGTCAGGTGTACTCCGGATATCCACTTACCTTCGGTGGCGGGACAA AAGTGGAGATAAAACGGACTGTGGCCGCACCATCCGTTTTCATCTTTCCCC CCTCCGACGAACAACTCAAGTCCGGCACAGCTTCTGTGGTGTGCCTGCTGA ATAACTTCTACCCAAGGGAGGCCAAAGTCCAGTGGAAAGTGGACAACGCT CTGCAGAGCGGCAATTCTCAGGAATCTGTGACTGAGCAGGATTCAAAAGAT TCTACTTACAGTCTGTCCTCCACCCTGACACTCTCCAAGGCCGACTATGAGA AACACAAGGTGTACGCATGCGAGGTAACTCACCAGGGACTGTCCAGTCCTG TTACAAAGTCCTTTAATCGTGGCGAATGT (SEQ ID NO:18) |
| HCB | CAAGTGCAATTGGTGCAATCTGGCGCTGAAGTTAAAAAACCAGGAGCAAG TGTTAAAGTATCTTGTAAGGCCTCCGGCTATACCTTTACAGACTACTATATG CATTGGGTCAGACAAGCTCCAGGTCAGGGCCTGGAATGGATGGGAAGAGT GAACCCAAATCGGCGTGGAACCACATACAATCAGAAATTCGAGGGGAGAG TTACCATGACCACCGACACCTCAACCTCTACCGCTTATATGGAGCTGCGAA GTCTCCGGTCTGATGATACCGCTGTGTACTATTGCGCCAGAGCCAACTGGC TCGACTATTGGGGCCAGGGCACTACTGTCACAGTGAGCAGTGCCTCCACTA AGGGACCCTCCGTCTTTCCTCTGGCTCCTTCCTCCAAGTCAACATCAGGTGG CACAGCTGCTCTGGGATGTTTGGTGAAGGATTATTTCCCCGAGCCTGTTACC GTGAGTTGGAACTCAGGTGCTCTCACTTCTGGGGTGCATACATTTCCCGCCG TCCTGCAGTCTTCCGGATTGTATAGCCTGTCAAGTGTGGTCACCGTGCCCTC TAGTTCCCTGGGAACACAGACATACATTTGCAACGTGAATCACAAGCCCAG CAATACTAAGGTGGACAAGAGGGTTGAACCCAAGAGTTGCGATAAAACAC ATACTTGTCCTCCCTGTCCTGCACCAGAGCTGCTTGGGGGACCAAGTGTATT CCTTTTTCCCCCCAAGCCTAAGGACACTCTGATGATCAGCCGGACTCCTGA GGTGACCTGTGTAGTGGTAGACGTTTCACATGAGGATCCCGAAGTGAAGTT CAACTGGTATGTGGATGGGGTTGAAGTGCATAATGCAAAGACAAAACCAC GAGAGGAACAGTACAATTCCACTTATAGGGTCGTTTCAGTGCTGACCGTTC TGCACCAGGACTGGCTGAACGGTAAAGAGTATAAATGCAAGGTGTCCAAT AAGGCTCTGCCTGCTCCTATCGAAAAGACTATTAGCAAGGCTAAAGGGCAG CCTAGAGAACCACAGGTCTATACTCTCCCCCCCAGCCGTGAGGAGATGACT AAAAATCAGGTGAGCCTCACATGCCTGGTGAAGGGCTTCTACCCCTCAGAT ATCGCAGTGGAATGGGAAAGCAATGGACAGCCCGAGAACAACTACAAAAC CACACCTCCAGTTTTGGATTCTGACGGGTCTTTCTTTCTGTACAGCAGGTTG ACCGTGGATAAGAGCCGATGGCAGCAAGGGAACGTGTTTTCTTGCTCCGTG ATGCACGAGGCCCTGCACAATCACTACACACAAAAGTCCCTGTCCCTGAGC CCAGGCAAA (SEQ ID NO:17) |

### 1.2 Expression and Purification of Anti-EGFR Antibody (BT461-DB-EGFR) and Anti-c-Met Antibody (BT461-DB-c-Met)

According to the manufacturer's instructions, the pHR plasmid DNA encoding the anti-EGFR antibody and anti-c-Met antibody proteins was respectively transiently transfected into CHO-K1 cells with the FUT8 gene knocked out, using the ExpiFectamine CHO Transfection Kit (Gibco). The transfected cells were placed in OPM-CD TransCHO (OPM, P83059) expression medium and cultured in a shaker at 37°C, 125 rpm for 14 days, after which the cell supernatant was collected.

The obtained cell suspension (CHO-K1 cells, Chinese Hamster Ovary cells, ATCC, CCL-61) was centrifuged (5000 rpm, 30 mins), and the supernatant was filtered (using a 0.22 µm filter membrane) followed by affinity chromatography purification.

Affinity chromatography capture for the EGFR and c-Met arms: Mab Select Sure LX resin (cytiva) was used, with a retention time of 6 minutes. The fermentation supernatant was loaded onto the chromatography column at a loading capacity of 40 mg protein per milliliter of resin. The target protein was in an elution buffer at pH 3.8. The specific experimental step is shown in Table 7.

**Table 7**

| Step | Buffer | Column volume (CV) |
|---|---|---|
| Balance | 20 mM PB, 150 mM NaCl, pH7.4 | 5 |
| Load sample | Cell culture fluid | / |
| Balance | 20 mM PB, 150 mM NaCl, pH7.4 | 2 |
| Rinse 1 | 20 mM NaAC, 0.5 M NaCl, pH 5.0 | 5 |
| Rinse 2 | 20 mM NaAC, pH 5.0 | 4 |
| Elution | 20 mM NaAC, pH 3.8 | 6 |
| CIP | 0.2 M NaOH | 2 |
| Balance | 20 Mm PB, 150 Mm NaCl, pH7.4 | 2 |
| Storage | 20% ethanol | 2 |

The obtained antibody solution underwent buffer exchange (buffer replaced with PBS) and concentration using a 30 KD ultrafiltration tube.

The obtained protein was tested for concentration using Nanodrop, and tested for purity using HPLC-SEC.

### 1.3 In Vitro Assembly and Purification of BT461-DB

Assembly system: Using Fab exchange technology, the affinity chromatography-purified anti-EGFR antibody (BT461-DB-EGFR) and anti-c-Met antibody (BT461-DB-c-Met) were assembled at a ratio of 1:1.1 (for example, 8 mg of the EGFR antibody arm and 8.8 mg of the c-Met antibody arm) and added to a reducing agent, 2-MEA, at a final concentration of 75 mmol/L. Reduction was performed in a 31°C water bath for 5 hours.

The 2-MEA reducing agent was removed by G-25 desalting, and the buffer was exchanged to PBS. Oxidation was carried out by standing at room temperature for 48 hours. The oxidation buffer was: PBS (pH 7.110, Cond: 16.376 mS/cm).

The reduction reaction system configurations for the two single arms are shown in Table 8.

**Table 8**

| Sample and buffer | Amount added |
|---|---|
| EGFR | a mg |
| c-Met | 1.1a mg |
| 300mM 2-MEA | 0.25b ml final concentration 75mM |
| 1M Tris | Adjust the pH to 7.4 |
| 1 X PBS | Added to b ml of reaction system |

The above reaction system was placed in a 31°C water bath for 5 hours to fully reduce the disulfide bonds between the antibodies.

G25 chromatography has a molecular sieve effect, which can separate the large-molecule antibodies from the small-molecule reducing agent 2-MEA. The experimental step is shown in Table 9.

**Table 9**

| Step | Buffer | Retention time (min) | Column volume (CV) |
|---|---|---|---|
| Balance | 1 X PBS pH7.4 | 4 | 3-5 |
| Load sample | Sample of reaction system in step 2 | 4 | <0.3 |
| Balance | 1 X PBS pH7.4 | 4 | 3-5 |
| CIP | 0.2M NaOH | 4 | 3 |
| Storage | 20% ethanol | 4 | 3 |

After loading was completed, the sample was collected. The UV280 peak collection range was 100-100 mAU.

The purification system was: cation exchange chromatography, using the Boglong Diamond SP Mustang resin. The aggregates and fragments inherently present during the expression of the two antibodies, as well as the excess c-Met during the assembly process, resulted in the presence of product-related impurities in the sample after oxidation. There is a difference in the isoelectric points between the bispecific antibody and the product-related impurities, allowing for the removal of impurities via cation exchange. Mobile phase A: 20 mM NaAc pH 5.0; Mobile phase B: 20 mM NaAc, 1 M NaCl pH 5.0. The experimental step is shown in Table 10.

**Table 10**

| Step | Buffer | Retention time (min) | Column volume (CV) |
|---|---|---|---|
| Balance | 20mM NaAc pH5.0 | 3-6 | 3-5 |
| Load sample | G25 desalting and buffer-exchanged sample, the pH was adjusted to 5.0 using 3 M acetic acid, and was diluted with water for injection until the conductivity was less than 10 mS/cm. | 3-6 | NA |
| Balance | 20mM NaAc pH5.0 | 3-6 | 3-5 |
| Gradient elution | 0-50% B | 5 | 30 |
| CIP | 1 M NaOH | 3-6 | 3-5 |
| Storage | 20% ethanol | 3-6 | 3-5 |

During the elution process, the cation exchange UV280 peak collection range was 100-100 mAU. Collection tubes were changed when an inflection point appeared in the peak shape. The cation exchange elution samples were sent for testing of SEC, NR-CE, and CEX.

The test results showed that the SEC monomer purity of BT461-DB was 99.66%, with aggregates accounting for 0.27% and fragments accounting for 0.08%. The NR-CE-SDS (non-reduced capillary gel electrophoresis) monomer purity was 96.2%, meeting the requirements for subsequent studies.

### 1.3 In Vitro Assembly and Purification of BT461-DB

Assembly system: Using Fab exchange technology, the affinity chromatography-purified anti-EGFR antibody (BT461-DB-EGFR) and anti-c-Met antibody (BT461-DB-c-Met) were assembled at a ratio of 1:1.1 (for example, 8 mg of the EGFR antibody arm and 8.8 mg of the c-Met antibody arm) and added to a reducing agent, 2-MEA, at a final concentration of 75 mmol/L. Reduction was performed in a 31°C water bath for 5 hours.

The 2-MEA reducing agent was removed by G-25 desalting, and the buffer was exchanged to PBS. Oxidation was carried out by standing at room temperature for 48 hours. The oxidation buffer was: PBS (pH 7.110, Cond: 16.376 mS/cm).

The reduction reaction system configurations for the two single arms are shown in Table 11.

**Table 11**

| Sample and buffer | Amount added |
|---|---|
| EGFR | a mg |
| c-Met | 1.1a mg |
| 300mM 2-MEA | 0.25b ml final concentration 75mM |
| 1M Tris | Adjust the pH to 7.4 |
| 1 X PBS | Added to b ml of reaction system |

The above reaction system was placed in a 31°C water bath for 5 hours to fully reduce the disulfide bonds between the antibodies.

G25 chromatography has a molecular sieve effect, which can separate the large-molecule antibodies from the small-molecule reducing agent 2-MEA. The experimental step is shown in Table 12.

**Table 12**

| Step | Buffer | Retention time (min) | Column volume (CV) |
|---|---|---|---|
| Balance | 1 X PBS pH7.4 | 4 | 3-5 |
| Load sample | Sample of reaction system in step 2 | 4 | <0.3 |
| Balance | 1 X PBS pH7.4 | 4 | 3-5 |
| CIP | 0.2M NaOH | 4 | 3 |
| Storage | 20% ethanol | 4 | 3 |

After loading was completed, the sample was collected. The UV280 peak collection range was 100-100 mAU.

The purification system was: cation exchange chromatography, using the Boglong Diamond SP Mustang resin. The aggregates and fragments inherently present during the expression of the two antibodies, as well as the excess c-Met during the assembly process, resulted in the presence of product-related impurities in the sample after oxidation. There is a difference in the isoelectric points between the bispecific antibody and the product-related impurities, allowing for the removal of impurities via cation exchange. Mobile phase A: 20 mM NaAc pH 5.0; Mobile phase B: 20 mM NaAc, 1 M NaCl pH 5.0. The experimental step is shown in Table 13.

**Table 13**

| Step | Buffer | Retention time (min) | Column volume (CV) |
|---|---|---|---|
| Balance | 20mM NaAc pH5.0 | 3-6 | 3-5 |
| Load sample | G25 desalting and buffer-exchanged sample, the pH was adjusted to 5.0 using 3 M acetic acid, and was diluted with water for injection until the conductivity was less than 10 mS/cm. | 3-6 | NA |
| Balance | 20mM NaAc pH5.0 | 3-6 | 3-5 |
| Gradient elution | 0-50% B | 5 | 30 |
| CIP | 1 M NaOH | 3-6 | 3-5 |
| Storage | 20% ethanol | 3-6 | 3-5 |

During the elution process, the cation exchange UV280 peak collection range was 100-100 mAU. Collection tubes were changed when an inflection point appeared in the peak shape. The cation exchange elution samples were sent for testing of SEC, NR-CE, and CEX.

The test results showed that the SEC monomer purity of BT461-DB was 99.66%, with aggregates accounting for 0.27% and fragments accounting for 0.08%. The NR-CE-SDS (non-reduced capillary gel electrophoresis) monomer purity was 96.2%, meeting the requirements for subsequent studies.

### Example 2. Evaluation of the Binding Activity of BT461-DB to EGFR and c-Met Antigen Proteins 2.1 Targeted Binding to c-Met Protein

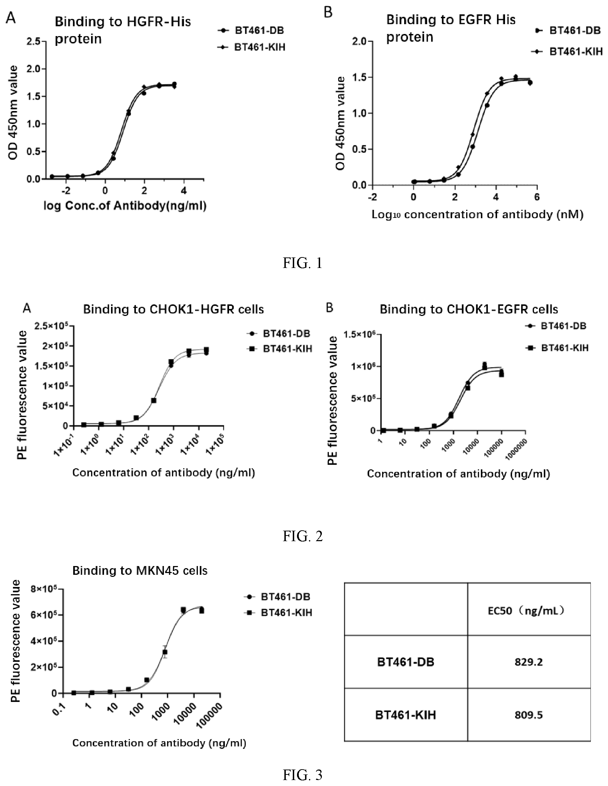

C-MET-HIS (Acrobiosystems, C-MET-H5227) was diluted to 2 µg/mL using coating solution (mix 8 ml of 0.2 mol/L Na2CO3 and 17 ml of 0.2 mol/L NaHCO3, then add 75 ml of distilled water, adjust pH to 9.6). A volume of 50 µL per well was added to the microplate wells and incubated at 37°C for 2 hours. The liquid in the wells was discarded. The wells were washed 5 times using a plate washer with 200 µL of washing solution (PBST, PBS dilution buffer added with 0.05% Tween-20) per wash. 200 µL of blocking solution (PBST + 5% skimmed milk powder) was added to each well and left overnight at 4°C. The liquid in the wells was discarded. The wells were washed 5 times using a plate washer (Tecan, model Hydrospeed) with 200 µL of washing solution per wash. 50 µL of BT461 and amivantamab (Johnson & Johnson, batch number MAS0001) antibodies at a concentration of 20 µg/mL, serially diluted 5-fold across 7 gradients, were added to each well, along with a blank control. Incubation was performed at 37°C for 60 min. The liquid in the wells was discarded. The wells were washed 5 times using a plate washer with 200 µL of washing solution per wash. 50 µL of the secondary antibody, Goat-anti-human-IgG-Fc-Secondary Antibody (Jackson, diluted 1:10000), was added to each well and incubated at 37°C for 60 min. The liquid in the wells was discarded. The wells were washed 5 times using a plate washer with 200 µL of washing solution per wash. 50 µL of chromogenic solution was added per well and incubated at 37°C for 15 min. 50 µL per well of 2 mol/L H2SO4 was added to stop the reaction, and the OD450 value was read on a microplate reader. The ELISA-binding activity analysis results are shown in Table 14 below and FIG. 1A.

### 2.2 Targeted Binding to EGFR Protein

EGFR-HIS (Acrobiosystems, EGR-H5222) was diluted to 2 µg/mL using coating solution (mix 8 ml of 0.2 mol/L Na2CO3 and 17 ml of 0.2 mol/L NaHCO3, then add 75 ml of distilled water, adjust pH to 9.6). A volume of 50 µL per well was added to the microplate wells and incubated at 37°C for 2 hours. The liquid in the wells was discarded. The wells were washed 5 times using a plate washer with 200 µL of washing solution (PBST, PBS dilution buffer added with 0.05% Tween-20) per wash. 200 µL of blocking solution (PBST + 5% skimmed milk powder) was added to each well and left overnight at 4°C. The liquid in the wells was discarded. The wells were washed 5 times using a plate washer (Tecan, model Hydrospeed) with 200 µL of washing solution per wash. 50 µL of the anti-c-Met/EGFR bispecific antibodies (BT461, amivantamab) at a concentration of 20 µg/mL, serially diluted 5-fold across 7 gradients, were added to each well, along with a blank control. Incubation was performed at 37°C for 60 min. The liquid in the wells was discarded. The wells were washed 5 times using a plate washer with 200 µL of washing solution per wash. 50 µL of the secondary antibody, Goat-anti-human-IgG-Fc-Secondary Antibody (Jackson, diluted 1:10000), was added to each well and incubated at 37°C for 60 min. The liquid in the wells was discarded. The wells were washed 5 times using a plate washer with 200 µL of washing solution per wash. 50 µL of chromogenic solution was added per well and incubated at 37°C for 15 min. 50 µL per well of 2 mol/L H2SO4 was added to stop the reaction, and the OD450 value was read on a microplate reader. The ELISA-binding activity analysis results are shown in Table 14 below and FIG. 1B.

**Table 14. Binding EC50 of the Bispecific Antibody of the Present Invention to c-Met and EGFR Proteins**

| EC50 (ng/mL) | BT461-DB | BT461-KIH |
|---|---|---|
| HGFR-his | 8.216 | 6.684 |
| EGFR-his | 1300 | 807.2 |

As can be seen from Table 14 and FIG. 1, the antibody BT461-DB can specifically bind to human c-Met protein and EGFR protein, and its binding activity is consistent with the BT461-KIH antibody.

### Example 3. Evaluation of the Binding Activity of BT461-DB to CHO-K1 Cells Overexpressing EGFR and c-Met Respectively

### 3.1 BT461 Targets and Binds to CHO-K1 Cells Overexpressing c-Met Membrane Protein

To test the specificity of the antibody of the present invention in binding to c-Met membrane protein on the cell surface, we stably overexpressed human c-Met membrane protein in CHO-K1 cells (Chinese Hamster Ovary cell subclone, ATCC #CCL-61) via plasmid electroporation and drug selection. The human c-Met overexpressing CHO-K1-c-Met cells were digested, counted, resuspended in a 96-well plate (3E5 cells added per well), and washed with PBS (twice); Blank control and experimental groups were set up on the 96-well plate. The antibodies BT461 and amivantamab (at a concentration of 20 µg/mL) were added to the wells of the experimental group and serially diluted 5-fold to create 7 concentration gradients; The 96-well plate was placed at 4°C to incubate for 30 mins, centrifuged (1000 rpm, 2 mins), the liquid in the plate wells was removed, and then washed with PBS (200 µL per wash, twice); PE-anti-human-IgG-Fc-Secondary-Antibody (Jackson, diluted at a ratio of 1:10000, 50 µL) was added to each well, incubated at 4°C for 30 mins, centrifuged (1000 rpm, 2 mins), the liquid was removed, and then washed with PBS (200 µL per wash, twice); PBS (200 µL per well) was added to each well, the cells were resuspended, and then read on a flow cytometer. The FACS-binding activity analysis results are shown in Table 15 and FIG. 2A.

3.2 Similarly, to test the specificity of the antibody of the present invention in binding to EGFR membrane protein on the cell surface, we stably overexpressed human EGFR membrane protein in CHO-K1 cells (Chinese Hamster Ovary cell subclone, ATCC #CCL-61) via plasmid electroporation and drug selection. The human EGFR overexpressing CHO-K1-EGFR cells were digested, counted, resuspended in a 96-well plate (3E5 cells added per well), and washed with PBS (twice); in the 96-well plate, blank control and experimental groups were set up, the anti-c-Met/EGFR antibodies BT461 and amivantamab (at a concentration of 20 µg/mL) were added to the wells of the experimental group and serially diluted 5-fold to create 7 concentration gradients; The 96-well plate was placed at 4°C to incubate for 30 mins, centrifuged (1000 rpm, 2 mins), the liquid in the plate wells was removed, and then washed with PBS (200 µL per wash, twice); PE-anti-human-IgG-Fc-Secondary-Antibody (Jackson, diluted at a ratio of 1:10000, 50 µL) was added to each well, incubated at 4°C for 30 mins, centrifuged (1000 rpm, 2 mins), the liquid was removed, and then washed with PBS (200 µL per wash, twice); PBS (200 µL per well) was added to each well, the cells were resuspended, and then read on a flow cytometer. The FACS-binding activity analysis results are shown in Table 15 and FIG. 2B.

**Table 15. Binding EC50 of BT461-DB to Cells Expressing c-Met and/or EGFR Membrane Protein**

| EC50 (ng/mL) | BT461-DB | BT461-KIH |
|---|---|---|
| CHOK1-HGFR | 266.2 | 270.4 |
| CHOK1-EGFR | 1725 | 1962 |

The results showed that the antibody BT461-DB of the present invention binds to EGFR or c-Met protein expressed on the surface of CHO-K1 cells with EC50 values of 1725 ng/mL and 266.2 ng/mL, respectively. Its binding activity is consistent with that of the antibody BT461-KIH (1962 ng/mL and 270.4 ng/mL, respectively).

### Example 4. Specific Binding of BT461-DB to EGFR/c-Met Double-Positive Tumor Cell MKN45 Cells

To further test the binding of the antibody of the present invention to tumor cells expressing both targets, we digested, counted, and resuspended EGFR and c-Met membrane proteins highly expressing MKN45 cells (Shangen Biotechnology, Cat No. SNL-173), in a 96-well plate (3E5 cells added per well), and washed with PBS (twice); in the 96-well plate, blank control and experimental groups were set up, the anti-c-Met/EGFR antibodies BT461 and amivantamab (at a concentration of 20 µg/mL) were added to the wells of the experimental group and serially diluted 5-fold to create 7 concentration gradients; The 96-well plate was placed at 4°C to incubate for 30 mins, centrifuged (1000 rpm, 2 mins), the liquid in the plate wells was removed, and then washed with PBS (200 µL per wash, twice); PE-anti-human-IgG-Fc-Secondary-Antibody (Jackson, diluted at a ratio of 1:10000, 50 µL) was added to each well, incubated at 4°C for 30 mins, centrifuged (1000 rpm, 2 mins), the liquid was removed, and then washed with PBS (200 µL per wash, twice); PBS (200 µL per well) was added to each well, the cells were resuspended, and then read on a flow cytometer. The FACS-binding activity analysis results are shown in Table 16 and FIG. 3.

The results showed that the binding activities of antibodies BT461-DB and BT461-KIH to MKN45 are essentially consistent, with EC50 values of 829.2 ng/mL and 809.5 ng/mL, respectively.

**Table 16**

| | EC50(ng/mL) |
|---|---|
| BT461-DB | 829.2 |
| BT461--KIH | 809.5 |

### Example 5. Antagonizing the Binding of Ligands HGF and EGF to Target Proteins EGFR and c-Met 5.1 Antagonizing the Binding of EGF to Protein EGFR

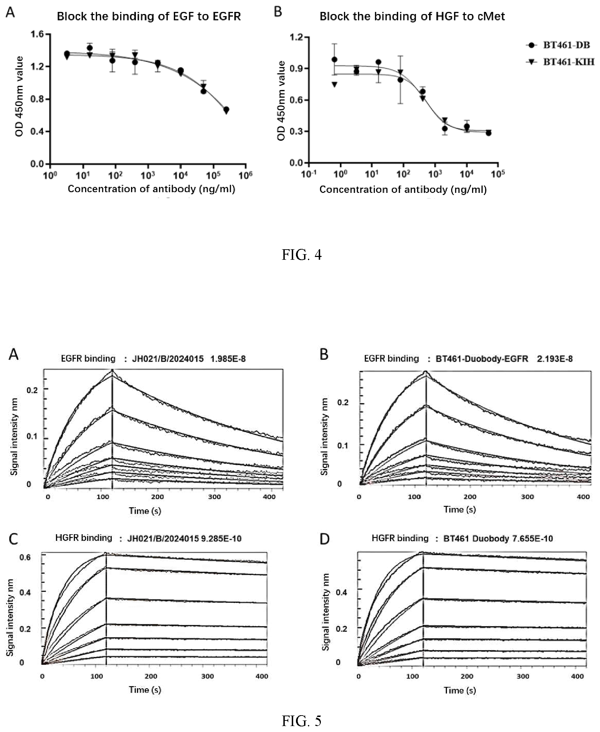

EGF-mFC (Acrobiosystems, Cat No. EGF-H525b) was diluted to 5 µg/mL using coating solution (mix 8 ml of 0.2 mol/L Na2CO3 and 17 ml of 0.2 mol/L NaHCO3, then add 75 ml of distilled water, adjust pH to 9.6) and coated onto the microplate wells at 50 µL per well. It was incubated at 37°C for 2 hrs. The liquid was removed and the wells were washed with washing solution (PBST, PBS dilution buffer added with 0.05% Tween-20) (200 µL per wash, 5 times); 200 µL of blocking solution was added to each well and left overnight at 4°C. The liquid was removed and the wells were washed with washing solution (200 µL per wash, 5 times); Blank control and experimental groups were set up on the microplate. EGFR-his (25 µL, 4 µg/mL) and the anti-c-Met/EGFR antibody were added to the wells of the experimental group and serially diluted 5-fold to create 7 concentration gradients. Incubation was performed at 37°C for 120 mins. The liquid was removed and the wells were washed with washing solution (200 µL per wash, 5 times); Goat-anti-HIS-Fc-Secondary-Antibody (Jackson, diluted at a ratio of 1:10000) was added to each well. The liquid was removed and the wells were washed with washing solution (200 µL per wash, 5 times); Chromogenic solution (50 µL per well) was added to each well and incubated at 37°C for 15 mins. Then, H2SO4 aqueous solution (50 µL per well, 2 mol/L) was added to stop the reaction, and the OD450 value was read on a microplate reader. The ELISA-blocking activity analysis results are shown in Table 17 and FIG. 4A.

### 5.2 Antagonizing the Binding of HGF to Protein c-Met

HGFR-hfc (Acrobiosystems, Cat No. MET-H5256) was diluted to 2 µg/mL using coating solution (mix 8 ml of 0.2 mol/L Na2CO3 and 17 ml of 0.2 mol/L NaHCO3, then add 75 ml of distilled water, adjust pH to 9.6) and coated onto the microplate wells at 50 µL per well. It was incubated at 37°C for 2 hrs. The liquid was removed and the wells were washed with washing solution (PBST, PBS dilution buffer added with 0.05% Tween-20) (200 µL per wash, 5 times); 200 µL of blocking solution was added to each well and left overnight at 4°C. The liquid was removed and the wells were washed with washing solution (200 µL per wash, 5 times); Blank control and experimental groups were set up on the microplate. 25 µL of HGF-HIS (Acrobiosystems, Cat No. HGF-H52H3) at a concentration of 200 ng/mL, and antibodies BT461-DB and BT461-KIH at a concentration of 500 µg/mL were added to the wells of the experimental group and serially diluted 5-fold to create 7 concentration gradients. Incubation was performed at 37°C for 120 mins. The liquid was removed and the wells were washed with washing solution (200 µL per wash, 5 times); Goat-anti-human-his-Secondary-Antibody (Jackson, Cat No.: 109-005-098, diluted at a ratio of 1:10000) was added to each well. The liquid was removed and the wells were washed with washing solution (200 µL per wash, 5 times); Chromogenic solution (50 µL per well) was added to each well and incubated at 37°C for 15 mins. Then, H₂SO₄ aqueous solution (50 µL per well, 2 mol/L) was added to stop the reaction, and the OD450 value was read on a microplate reader. The ELISA-blocking activity analysis results are shown in Table 17 and FIG. 4B.

**Table 17. IC50 for Blocking the Binding of HGF and EGF to Proteins EGFR and c-Met**

| IC50 (ng/mL) | BT461-DB | BT461-KIH |
|---|---|---|
| HGF | 456.4 | 543.3 |

As can be seen from FIG. 4A, both antibodies BT461-DB and BT461-KIH can block the binding of the ligand EGF to the receptor EGFR, and the blocking levels are consistent. As can be seen from Table 17 and FIG. 4B, both antibodies BT461-DB and BT461-KIH can block the binding of the ligand HGF to the receptor c-Met, and the blocking levels are essentially consistent. The IC50 values for antibodies BT461-DB and BT461-KIH are 456.4 ng/mL and 543.3 ng/mL, respectively.

### Example 6. Detection of the Affinity of Antibody BT461-DB for Antigens Based on Bio-Layer Interferometry (BLI)

### 6.1 Binding Kinetics Detection of the Bispecific Antibody of the Present Invention Targeting Recombinant Human EGFR-His

EGFR-His (ACRO, Cat No. EGR-H5222) was serially diluted from 100 nM using PBST by 2-fold serial dilution to create 7 concentration points, with a zero-concentration reference well also set up. The anti-c-Met/EGFR bispecific antibody BT461-DB and the control antibody BT461-KIH (JH021/B/2024015) were diluted to 5 µg/mL respectively. The operating conditions for the molecular interaction instrument (Fortebio, manufacturer: Sartorius, model: Octet R8) were set as follows: temperature 30°C, Shake speed 1000 rpm. Pre-coated AHC2 probes (Sartorius, Cat No. 18-5142) were used to capture the antibody, with a capture time of 180s; then bound to the serially diluted EGFR-HIS samples, with a binding time of 120s; the dissociation time was 300s; regeneration was performed 3 times using regeneration buffer (10 mM glycine, pH 1.7), 30s each time. The test was run using the ForteBio Octet System. After obtaining the sensorgram data, the association constant (ka) and dissociation constant (kd) were analyzed using Octet BLI Analysis software, fitting the ideal binding and dissociation curves as shown in FIG.s 5A and 5B. The equilibrium dissociation constant KD (kd/ka) between the antibody and antigen was calculated, and the results are shown in Table 18.

### 6.2 Binding Kinetics Detection of the Bispecific Antibody of the Present Invention Targeting Recombinant Human c-Met-His

Same as Example 6.1, the association constant (ka) and dissociation constant (kd) of BT461-DB and BT461-KIH with c-Met-HIS (Acrobiosystems, c-Met-H5227) were analyzed using Octet BLI Analysis software, fitting the ideal binding and dissociation curves as shown in FIG.s 5C and 5D. The equilibrium dissociation constant KD (kd/ka) between the antibody and antigen was calculated.

**Table 18. c-Met-HIS affinity and EGFR-HIS affinity**

| Antibody affinity detected by BLI | | | | | | | |
|---|---|---|---|---|---|---|---|
| HGFR-HIS affinity (AHC2 sensor) | | | | EGFR-HIS Affinity (AHC2 sensor) | | | |
| Sample ID | KD (M) | kon(1/Ms) | kdis(1/s) | Sample ID | KD (M) | kon(1/Ms) | kdis(1/s) |
| BT461 -KIH | 9.29E-10 | 2.78E+05 | 2.58E-04 | BT461 -KIH | 1.99E-08 | 1.47E+05 | 2.92E-03 |
| BT461-DB | 7.66E-10 | 2.78E+05 | 2.13E-04 | BT461-DB | 2.19E-08 | 1.51E+05 | 3.30E-03 |

As shown in Table 18, the affinities of BT461-DB and BT461-KIH for c-Met-his are 0.766 nM and 0.929 nM, respectively, which are essentially consistent. Those similarly consistent are the affinities of BT461-DB and BT461-KIH for EGFR-HIS, which are 21.93 nM and 19.85 nM, respectively. The affinities of the two arms of the BT461-DB antibody differ by over 20-fold.

### Example 7. Evaluation of the Affinity of the FC of Antibody BT461-DB to Receptor Proteins

### 7.1 Detection of the Binding Affinity of BT461-DB Antibody to FcγRI (CD64)

The affinity detection for the binding of JH021 antibody to Human FcγRI (CD64) (brand: ACRO, Cat No.: FCA-H52H1, batch No.: RC163P1-2387F1-1GU) based on Bio-layer Interferometry (BLI) technology: The JH021 antibody was serially diluted using PBST, starting from a concentration of 250 nM, followed by 2-fold serial dilution to create 7 concentration points, with a zero-concentration control well also set up. The FcγRI (CD64) receptor protein was diluted to 5 µg/mL using Loading Buffer (1× PBS, pH7.4, with 0.02% Tween-20, 0.1% BSA) and immobilized onto HIS1K sensors (brand: Sartorius, Cat No.: 18-5120, batch No.: 2301012611), with a threshold of 0.5 nm and an immobilization time of 180s. The operating conditions for the molecular interaction instrument ForteBio's Octet System (brand: Sartorius, model: Octet R8) were set as follows: temperature 30°C, Shake speed 1000 rpm. The immobilized HIS1K probes were used to bind the serially diluted samples, with a binding time of 120s and a dissociation time of 300s; regeneration was performed for 30s using regeneration buffer (10 mM glycine, pH 1.7). The test was run using ForteBio's Octet System. After obtaining the sensorgram data, the association constant (ka) and dissociation constant (kd) were analyzed using Octet BLI Analysis 12.2 software, fitting the ideal binding and dissociation curves. The affinity constant KD (kd/ka) between the antibody and antigen was calculated. The results, as shown in Table 19, are as follows: The binding affinities of BT461-DB and BT461-KIH to FcγRI (CD64) are 2.28E-07 and 2.33E-07, respectively, showing little difference.

### 7.2 Detection of the Binding Affinity of Antibody to FcγRIIIA (CD16a-F176)

The affinity detection for the binding of JH021 antibody to Human FcγRIIIA(CD16a-F176) (brand: ACRO, Cat No.: CDA-H5220, batch No.: 2415-226YF1-1J9) based on Bio-layer Interferometry (BLI) technology: The JH021 antibody was serially diluted using PBST, starting from a concentration of 5000 nM, followed by 2-fold serial dilution to create 7 concentration points, with a zero-concentration control well also set up. The FcγRIIIA (CD16a-F176) receptor protein was diluted to 5 µg/mL using Loading Buffer (1× PBS, pH7.4, with 0.02% Tween-20, 0.1% BSA) and immobilized onto HIS1K sensors (brand: Sartorius, Cat No.: 18-5120, batch No.: 2301012611), with a threshold of 0.5 nm and an immobilization time of 180s. The operating conditions for the molecular interaction instrument ForteBio's Octet System (brand: Sartorius, model: Octet R8) were set as follows: temperature 30°C, Shake speed 1000 rpm. The immobilized HIS1K probes were used to bind the serially diluted samples, with a binding time of 120s and a dissociation time of 300s; regeneration was performed for 30s using regeneration buffer (10 mM glycine, pH 1.7). The test was run using ForteBio's Octet System. After obtaining the sensorgram data, the association constant (ka) and dissociation constant (kd) were analyzed using Octet BLI Analysis 12.2 software, fitting the ideal binding and dissociation curves. The affinity constant KD (kd/ka) between the antibody and antigen was calculated. The results, as shown in Table 19, are as follows: The binding affinities of BT461-DB and BT461-KIH to FcγRIIIA (CD16a-F176) are 3.30E-07 and 2.68E-07, respectively, showing little difference.

### 7.3 Detection of the Binding Affinity of Antibody to FcγRIIIA (CD16a-V176)

The affinity detection for the binding of JH021 antibody to Human FcγRIIIA(CD16a-V176) (brand: ACRO, Cat No.: CD8-H52H4, batch No.: 1824-21BCF1-196) based on Bio-layer Interferometry (BLI) technology: The JH021 antibody was serially diluted using PBST, starting from a concentration of 2500 nM, followed by 2-fold serial dilution to create 7 concentration points, with a zero-concentration control well also set up. The FcγRIIIA (CD16a-V176) receptor protein was diluted to 10 µg/mL using Loading Buffer (1× PBS, pH7.4, with 0.02% Tween-20, 0.1% BSA) and immobilized onto HIS1K sensors (brand: Sartorius, Cat No.: 18-5120, batch No.: 2301012611), with a threshold of 0.5 nm and an immobilization time of 180s. The operating conditions for the molecular interaction instrument ForteBio's Octet System (brand: Sartorius, model: Octet R8) were set as follows: temperature 30°C, Shake speed 1000 rpm. The immobilized HIS1K probes were used to bind the serially diluted samples, with a binding time of 120s and a dissociation time of 300s; regeneration was performed for 30s using regeneration buffer (10 mM glycine, pH 1.7). The test was run using ForteBio's Octet System. After obtaining the sensorgram data, the association constant (ka) and dissociation constant (kd) were analyzed using Octet BLI Analysis 12.2 software, fitting the ideal binding and dissociation curves. The affinity constant KD (kd/ka) between the antibody and antigen was calculated. The results, as shown in Table 19, are as follows: The binding affinities of BT461-DB and BT461-KIH to FcγRIIIA (CD16a-V176) are 2.65E-08 and 2.26E-08, respectively, showing little difference.

### 7.4 Detection of the Binding Affinity of Antibody to FcγRIIIB (CD16b)

The affinity detection for the binding of JH021 antibody to Human FcγRIIIB (CD16b) (brand: ACRO, Cat No.: CDB-H5227, batch No.: 2417-228KF1-17G) based on Bio-layer Interferometry (BLI) technology: The JH021 antibody was serially diluted using PBST, starting from a concentration of 5000 nM, followed by 2-fold serial dilution to create 7 concentration points, with a zero-concentration control well also set up. The FcγRIIIB (CD16b) receptor protein was diluted to 5 µg/mL using Loading Buffer (1× PBS, pH7.4, with 0.02% Tween-20, 0.1% BSA) and immobilized onto HIS1K sensors (brand: Sartorius, Cat No.: 18-5120, batch No.: 2301012611), with a threshold of 0.5 nm and an immobilization time of 180s. The operating conditions for the molecular interaction instrument ForteBio's Octet System (brand: Sartorius, model: Octet R8) were set as follows: temperature 30°C, Shake speed 1000 rpm. The immobilized HIS1K probes were used to bind the serially diluted samples, with a binding time of 120s and a dissociation time of 300s; regeneration was performed for 30s using regeneration buffer (10 mM glycine, pH 1.7). The test was run using ForteBio's Octet System. After obtaining the sensorgram data, the association constant (ka) and dissociation constant (kd) were analyzed using Octet BLI Analysis 12.2 software, fitting the ideal binding and dissociation curves. The affinity constant KD (kd/ka) between the antibody and antigen was calculated. The results, as shown in Table 19, are as follows: The binding affinities of BT461-DB and BT461-KIH to FcγRIIIB (CD16b) are 1.44E-05 and 1.42E-05, respectively, which are essentially consistent.

### 7.5 Detection of the Binding Affinity of Antibody to FcγRIIA (CD32a)

The affinity detection for the binding of JH021 antibody to Human FcγRIIA (CD32a) (brand: ACRO, Cat No.: CDA-H5221, batch No.: 1822-216TF1-139) based on Bio-layer Interferometry (BLI) technology: FcγRIIA (CD32a) was serially diluted using PBST, starting from a concentration of 2000 nM, followed by 2-fold serial dilution to create 7 concentration points, with a zero-concentration control well also set up. The JH021 antibody receptor protein was diluted to 20 µg/mL using Loading Buffer (1× PBS, pH7.4, with 0.02% Tween-20, 0.1% BSA) and immobilized onto ProA sensors (brand: Sartorius, Cat No.: 18-5010, batch No.: 2302000611), with a threshold of 2.0 nm and an immobilization time of 180s. The operating conditions for the molecular interaction instrument ForteBio's Octet System (brand: Sartorius, model: Octet R8) were set as follows: temperature 30°C, Shake speed 1000 rpm. The immobilized ProA probes were used to bind the serially diluted samples, with a binding time of 120s and a dissociation time of 300s; regeneration was performed for 30s using regeneration buffer (10 mM glycine, pH 1.7). The test was run using ForteBio's Octet System. After obtaining the sensorgram data, the association constant (ka) and dissociation constant (kd) were analyzed using Octet BLI Analysis 12.2 software, fitting the ideal binding and dissociation curves. The affinity constant KD (kd/ka) between the antibody and antigen was calculated. The results, as shown in Table 19, are as follows: The binding affinities of BT461-DB and BT461-KIH to FcγRIIA (CD32a) are 1.52E-06 and 1.53E-06, respectively, which are essentially consistent.

### 7.6 Detection of the Binding Affinity of Antibody to FcγRIIB/C (CD32b/c)

The affinity detection for the binding of JH021 antibody to Human FcγRIIB/C (CD32b/c) (brand: ACRO, Cat No.: CDB-H5228, batch No.: 478-234AF1-1AJ) based on Bio-layer Interferometry (BLI) technology: The JH021 antibody was serially diluted using PBST, starting from a concentration of 5000 nM, followed by 2-fold serial dilution to create 7 concentration points, with a zero-concentration control well also set up. The FcγRIIB/C (CD32b/c) receptor protein was diluted to 5 µg/mL using Loading Buffer (1× PBS, pH7.4, with 0.02% Tween-20, 0.1% BSA) and immobilized onto HIS1K sensors (brand: Sartorius, Cat No.: 18-5120, batch No.: 2301012611), with a threshold of 0.5 nm and an immobilization time of 180s. The operating conditions for the molecular interaction instrument ForteBio's Octet System (brand: Sartorius, model: Octet R8) were set as follows: temperature 30°C, Shake speed 1000 rpm. The immobilized HIS1K probes were used to bind the serially diluted samples, with a binding time of 120s and a dissociation time of 300s; regeneration was performed for 30s using regeneration buffer (10 mM glycine, pH 1.7). The test was run using ForteBio's Octet System. After obtaining the sensorgram data, the association constant (ka) and dissociation constant (kd) were analyzed using Octet BLI Analysis 12.2 software, fitting the ideal binding and dissociation curves. The affinity constant KD (kd/ka) between the antibody and antigen was calculated. The results, as shown in Table 19, are as follows: The binding affinities of BT461-DB and BT461-KIH to FcγRIIB/C (CD32b/c) are 1.65E-06 and 1.16E-06, respectively, which are essentially consistent.

### 7.7 Detection of the Binding Affinity of Antibody to FcRn

The affinity detection for the binding of JH021 antibody to Human FcRn (brand: ACRO, Cat No.: FCM-H8286, batch No.: BLA476-21CFF1-12V) based on Bio-layer Interferometry (BLI) technology: The JH021 antibody was serially diluted using PBST, starting from a concentration of 1000 nM, followed by 2-fold serial dilution to create 7 concentration points, with a zero-concentration control well also set up. The FcRn receptor protein was diluted to 2 µg/mL using Loading Buffer (1× PBS, pH7.4, with 0.02% Tween-20, 0.1% BSA) and immobilized onto SA sensors (brand: Sartorius, Cat No.: 18-5019, batch No.: 2309011711), with a threshold of 2.5 nm and an immobilization time of 180s. The operating conditions for the molecular interaction instrument ForteBio's Octet System (brand: Sartorius, model: Octet R8) were set as follows: temperature 30°C, Shake speed 1000 rpm. The immobilized SA probes were used to bind the serially diluted samples, with a binding time of 120s and a dissociation time of 300s; regeneration was performed for 30s using regeneration buffer (10 mM glycine, pH 1.7). The test was run using ForteBio's Octet System. After obtaining the sensorgram data, the association constant (ka) and dissociation constant (kd) were analyzed using Octet BLI Analysis 12.2 software, fitting the ideal binding and dissociation curves. The affinity constant KD (kd/ka) between the antibody and antigen was calculated. The results, as shown in Table 19, are as follows: The binding affinities of BT461-DB and BT461-KIH to FcRn are 2.28E-07 and 2.33E-07, respectively, which are essentially consistent.

### 7.8 Detection of the Binding Affinity of Antibody to C1q

The affinity detection for the binding of JH021 antibody to Human C1q (brand: Abcam, Cat No.: AB282858, batch No.: 1034803-2) based on Bio-layer Interferometry (BLI) technology: C1q was serially diluted using PBST, starting from a concentration of 100 nM, followed by 2-fold serial dilution to create 7 concentration points, with a zero-concentration control well also set up. The JH021 antibody receptor protein was diluted to 20 µg/mL using Loading Buffer (1× PBS, pH7.4, with 0.02% Tween-20, 0.1% BSA) and immobilized onto FAB2G sensors (brand: Sartorius, Cat No.: 18-5125, batch No.: 2212007211), with a threshold of 2.5 nm and an immobilization time of 180s. The operating conditions for the molecular interaction instrument ForteBio's Octet System (brand: Sartorius, model: Octet R8) were set as follows: temperature 30°C, Shake speed 1000 rpm. The immobilized FAB2G probes were used to bind the serially diluted samples, with a binding time of 120s and a dissociation time of 300s; regeneration was performed for 30s using regeneration buffer (10 mM glycine, pH 1.7). The test was run using ForteBio's Octet System. After obtaining the sensorgram data, the association constant (ka) and dissociation constant (kd) were analyzed using Octet BLI Analysis 12.2 software, fitting the ideal binding and dissociation curves. The affinity constant KD (kd/ka) between the antibody and antigen was calculated. The results, as shown in Table 19, are as follows: The binding affinities of BT461-DB and BT461-KIH to C1q are 1.12E-08 and 1.62E-08, respectively, which are essentially consistent.

**Table 19. Detection Results of the Affinity of Antibody BT461-DB to FC Receptor Proteins**

| FC Affinity Evaluation KD (M) | | | | | | | | |
|---|---|---|---|---|---|---|---|---|
| Loading Sample ID | CD16a(V176) | CD16a(F176) | CD16 b | CD32a | CD32 b/c | CD64-His | Bio-FcRn | C1Q |
| BT461-DB | 2.65E-08 | 3.30E-07 | 1.44E-05 | 1.52E-06 | 1.65E-06 | 8.97E-09 | 2.28E-07 | 1.12E-08 |
| BT461-KIH | 2.26E-08 | 2.68E-07 | 1.42E-05 | 1.53E-06 | 1.16E-06 | 7.63E-09 | 2.33E-07 | 1.62E-08 |

### Example 8. Antibody BT461-DB Inhibits EGFR Autophosphorylation and c-Met Autophosphorylation

To investigate whether the anti-c-Met/EGFR bispecific antibody can inhibit the c-Met and EGFR signaling pathways, we tested the changes in EGFR and c-Met autophosphorylation levels respectively.

### Western blot analysis

Non-small cell lung cancer A549 cells were seeded in a 6-well plate (4×10⁵ cells/well). After adherence, control and experimental groups were set up on the 6-well plate. Under the culture medium condition comprising 1% serum with 100 ng/mL EGF-mFC (Acrobiosystems) + 100 ng/mL HGF-his (Acrobiosystems), IgG1, amivantamab, BT461-DB, and BT461-KIH at a concentration of 250 µg/mL were added to the negative control group, control group, and experimental groups (BT461-DB, BT461-KIH) respectively. After reacting for 15 minutes, RIPA lysis buffer (150 µL, Beyotime) was added, and lysis was performed at 0°C (on ice) for 30 mins. Loading buffer (comprising the reducing agent β-mercaptoethanol (Life Technologies)) was added to each well, and the proteins were denatured by boiling at 95°C for 10 minutes. Using Tris-SDS as the electrophoresis buffer, the denatured proteins were subjected to electrophoresis (the denatured proteins were loaded into the wells of a 10% PAGE precast gel (Genscript)). After the electrophoresis test was completed, the proteins were transferred onto a PVDF membrane using transfer buffer with 20% methanol, and then the PVDF membrane was blocked with 5% skimmed milk at room temperature for 1 hour. Anti-c-Met antibody (Invitrogen, RJ243364), or anti-EGFR antibody (ABCAM, ab52894), or anti-GAPDH antibody (Solarbio, k200057m), or anti-p-c-Met antibody (Cellsignaling, #3077S), or anti-p-EGFR antibody (R&D, AF1095) was added and incubated overnight at 4°C, followed by washing with TBST (3 times, 10 mins each time). At room temperature, goat anti-mouse/rabbit IgG (H+L) HRP-conjugated secondary antibody (diluted 1:3000) was added to each well, incubated for 1h, and washed with TBST (3 times, 10 mins each time). At room temperature, reaction was performed with ECL substrate (Sangon, l115AA0118) for 1min, and development was carried out using a chemiluminescence system.

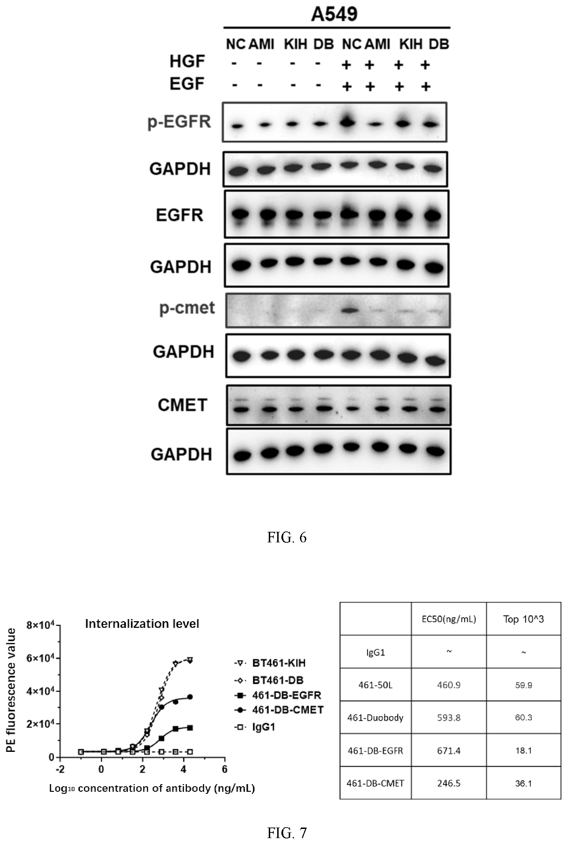

The development results are shown in FIG. 6. Stimulation with HGF-his (100 µg/mL) and EGF-mfc (100 µg/mL) for 15min activated the phosphorylation of EGFR and c-Met in A549 cells; the addition of BT461-DB or BT461-KIH on this basis significantly inhibited the intracellular p-c-Met (Y1234/1235) phosphorylation level and slightly inhibited the p-EGFR (Y1173) phosphorylation level.

### Example 9. Antibody BT461 Mediates Target Protein Internalization and Degradation

### Using pHAb Probes to Plot the Internalization Curve of Gradient Antibody Concentrations

100 µg of the secondary antibody Goat-anti-human-IgG-Fc-Secondary-Antibody was subjected to buffer exchange using an ultrafiltration tube. 500 microliters of 10 mM sodium bicarbonate (pH 8.5) was added, followed by centrifugation at 15000 rpm, 4°C, for 10 mins, repeated twice. 25 microliters of DMSO-water Mix (1:1) solution was added to 250 µg of pHAb Reactive Dyes (Promega, stored at - 80°C) to dissolve, obtaining a 10 mg/mL dye stock solution. The pHAb reactive dye was conjugated to the antibody at an antibody-to-dye ratio of 100 µg : 1.2 µL, with conjugation performed on a shaker at room temperature for 60 minutes. A desalting column was used to remove unreacted dye. The column was balanced by centrifugation at 900 g for 2 mins. The dye-antibody conjugation mix was dropped onto the center of the desalting column's slanted surface, and centrifuged with the slanted surface facing outward at 900 g for 2 mins. After collection, the antibody concentration and dye-to-antibody ratio were tested.

MKN45 cells were counted, and 3E5 cells were added to each well of a U-bottom 96-well plate, centrifuged at 1000 rpm for 2 min, and washed twice with PBS. Antibodies BT461-DB, BT461-KIH, BT461-DB-EGFR, BT461-DB-c-Met, and IgG1 were added to the wells and serially diluted, starting from a concentration of 20 µg/mL, with 5-fold dilution across 7 points. 50 µL of antibody was added to each well, along with a blank control; incubation was performed at 4°C for 30 min. Centrifugation was performed at 1000 rpm for 2 min, the liquid in the wells was discarded, and the wells were washed with PBS, 200 µL per wash, twice, with operations performed on ice. Dye-labeled secondary antibody (diluted 1:200) was added to the wells at 50 µL per well. One plate was placed in a 37°C incubator for incubation, and the other plate was placed in a 4°C refrigerator for incubation. After 4 hours, the wells were washed twice with PBS and resuspended in 200 µL of PBS. Flow cytometer detection was performed (using PE fluorescence excitation and detection).

The results are shown in FIG. 7. Antibody BT461-DB promotes target protein internalization, and its internalization efficiency is consistent with that of BT461-KIH. Furthermore, the internalization level mediated by the anti-c-Met antibody arm > the internalization level mediated by the anti-EGFR antibody arm.

### Example 10. Evaluation of ADCC Level Mediated by BT461-DB Antibody

The ADCC Reporter Bioassay method was used to test the ADCC biological activity of the antibodies. The ADCC reporter system used in this example was ADCC Reporter Bioassay (Suzhou Ruian, RA-CK01), and the fluorescent substrate was Bio-Turbo-One-Step Firefly Luciferase Assay Kit (Suzhou Ruian, RA-GL03).

### 10.1 Target Cells are CHOK1-EGFR

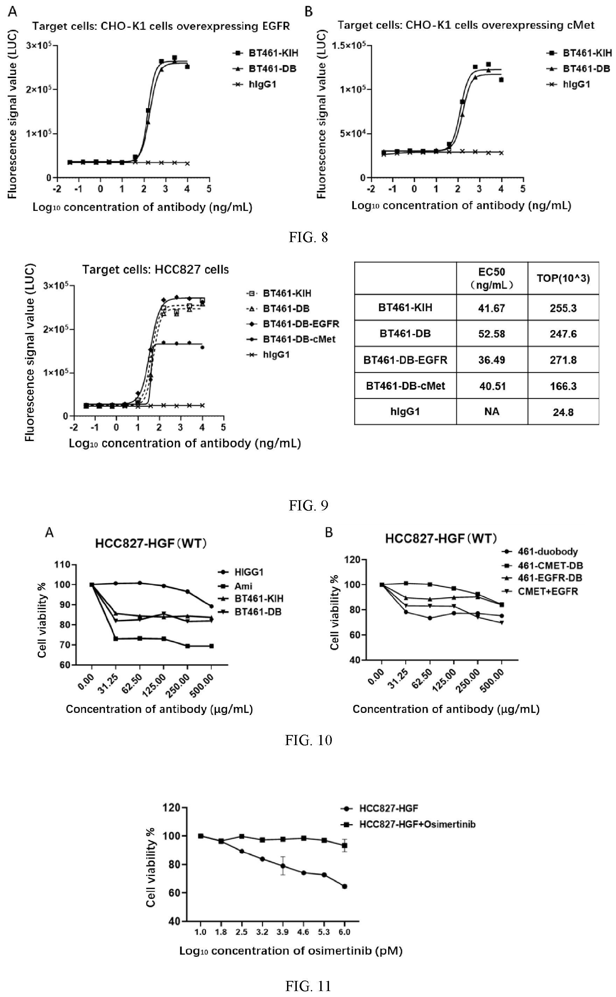

CHOK1-EGFR cells (overexpressing EGFR membrane protein) in the logarithmic growth phase were taken as target cells. They were digested, counted, and resuspended in culture medium (Gibco, F12K+10% FBS). The obtained cell suspension had a density of 2.5E5/mL. They were seeded into a 96-well plate (96-well opaque flat-bottom white plate, Costar, 3917) at 100 µL per well and cultured for 6 hours to allow adherence. After cell adherence, the medium was aspirated, and the cells were washed once with PBS. The corresponding antibodies were diluted to the corresponding gradient concentrations using 1640+1% FBS medium according to the plate layout of the experimental and control groups and added to the corresponding wells, 50 µL per well. Co-incubation was performed at 37°C, 5% CO2 for 45 min. ADCC reporter cells were collected and resuspended in phenol red-free 1640+1% FBS medium (1E6 cells/mL), at 25000 cells/well, 25 µL per well, resulting in an effector cell:target cell ratio = 1:1. After placing in the incubator for 6 h, the plate was taken out and allowed to equilibrate to room temperature. 75 µL of luciferase substrate was added to each well, incubated for 3 min, and the CTG reading was tested using a microplate reader (PerkinElmer). The obtained results are shown in FIG. 8A and Table 20.

As can be seen from the data in FIG. 8A, both antibodies BT461-DB and BT461-KIH mediated antibody-dependent cellular cytotoxicity (ADCC) against CHOK1-EGFR target cells, and the ADCC levels were similar.

### 10.2 Target Cells are CHOK1-c-Met

CHOK1-c-Met cells (overexpressing c-Met membrane protein) in the logarithmic growth phase were taken as target cells. They were digested, counted, and resuspended in culture medium (Gibco, F12K+10% FBS). The obtained cell suspension had a density of 2.5E5/mL. They were seeded into a 96-well plate (96-well opaque flat-bottom white plate, Costar, 3917) at 100 µL per well and cultured for 6 hours to allow adherence. After cell adherence, the medium was aspirated, and the cells were washed once with PBS. The corresponding antibodies were diluted to the corresponding gradient concentrations using 1640+1% FBS medium according to the plate layout of the experimental and control groups and added to the corresponding wells, 50 µL per well. Co-incubation was performed at 37°C, 5% CO2 for 45 min. ADCC reporter cells were collected and resuspended in phenol red-free 1640+1% FBS medium (1E6 cells/mL), at 25000 cells/well, 25 µL per well, resulting in an effector cell:target cell ratio = 1:1. After placing in the incubator for 6 h, the plate was taken out and allowed to equilibrate to room temperature. 75 µL of luciferase substrate was added to each well, incubated for 3 min, and the CTG reading was tested using a microplate reader (PerkinElmer). The obtained results are shown in FIG. 8B and Table 20.

As can be seen from the data in FIG. 8B, both antibodies BT461-DB and BT461-KIH mediated antibody-dependent cellular cytotoxicity (ADCC) against CHOK1-c-Met target cells, and the ADCC levels were similar.

**Table 20. EC50 of Antibody-Mediated ADCC Killing Curves**

| EC50 (ng/mL) | BT461-DB | BT461-KIH | hIgG1 |
|---|---|---|---|
| CHOK1-c-Met | 162.0 | 126.5 | NA |
| CHOK1-EGFR | 185.2 | 153.4 | NA |

### 10.3 Target Cells are Non-Small Cell Lung Cancer Cell Strain HCC827

HCC827 tumor cells in the logarithmic growth phase were taken as target cells. They were digested, counted, and resuspended in culture medium (Gibco, RPMI1640+10% FBS). The obtained cell suspension had a density of 2.5E5/mL. They were seeded into a 96-well plate (96-well opaque flat-bottom white plate, Costar, 3917) at 100 µL per well and cultured for 6 hours to allow adherence. After cell adherence, the medium was aspirated, and the cells were washed once with PBS. The corresponding antibodies were diluted to the corresponding gradient concentrations using 1640+1% FBS medium according to the plate layout of the experimental and control groups and added to the corresponding wells, 50 µL per well. Co-incubation was performed at 37°C, 5% CO2 for 45 min. ADCC reporter cells were collected and resuspended in phenol red-free 1640+1% FBS medium (1E6 cells/mL), at 25000 cells/well, 25 µL per well, resulting in an effector cell:target cell ratio = 1:1. After placing in the incubator for 6 h, the plate was taken out and allowed to equilibrate to room temperature. 75 µL of luciferase substrate was added to each well, incubated for 3 min, and the CTG reading was tested using a microplate reader (PerkinElmer). The obtained results are shown in FIG. 9.

As can be seen from the data in FIG. 9, both antibodies BT461-DB and BT461-KIH mediated antibody-dependent cellular cytotoxicity (ADCC) against HCC827 tumor cells, and the ADCC levels were similar, with EC50 values of 52.58 ng/mL and 41.67 ng/mL respectively, and maximum killing released fluorescence of 247.6*10^3 and 255.3*10^3 respectively. Furthermore, the experimental results also show that the ADCC mediated by 461-DB primarily depends on the BT461-DB-EGFR antibody arm.

### Example 11. BT461-DB Antibody Inhibits Tumor Cell Proliferation In Vitro

This example selected the non-small cell lung cancer EGFR mutant cell HCC827 (Procell #CL-0094, EGFR exon 19 deletion), which also overexpresses HGF (via lentiviral transfection, enabling autocrine expression after expression). The CellCounting-Lite^{®}3D kit (Vazyme, DD1102) was used to test the effect of the antibody of the present invention on the proliferation of these cell lines.

Tumor cells in the logarithmic growth phase were taken, digested, and seeded into a 96-well plate (2000 cells/well), and cultured in complete medium overnight. After removing the medium from the 96-well plate, 100 µL of anti-c-Met/EGFR antibodies BT461-DB, BT461-KIH, and control IgG1 (initial concentration 500 µg/mL) diluted in cell complete medium were added and serially diluted 2-fold to create 5 concentration gradients. After culturing in an incubator under conditions of 5% CO2 atmosphere and 37°C for 5 days, an equal volume of CellCounting-Lite^{®}3D reagent (Vazyme, Cat No. DD1102) was added to each well, mixed by shaking for 5 min until the cell clusters were completely lysed. After incubating for 25 min, 100 µL of the mixture was transferred to a white opaque plate and detected using a TR-FRET microplate reader (PerkinElmer, model, Cat No.).

The results are as shown in FIG. 10A, demonstrating that the antibody BT461-DB of the present invention can significantly inhibit the proliferation of tumor cells HCC827-HGF (expressing HGF) at multiple gradient concentrations, and its inhibitory level on HCC827-HGF cell proliferation is comparable to that of BT461-KIH.

### Example 12. The In Vitro Efficacy of the BT461-DB Bispecific Antibody Shows a Synergistic Effect

The experimental method was the same as in Example 11. HCC827-HGF tumor cells in the logarithmic growth phase were taken, digested, and seeded into a 96-well plate (2000 cells/well), and cultured in complete medium overnight. After removing the medium from the 96-well plate, 100 µL of antibodies BT461-DB, BT461-DB-EGFR, BT461-DB-c-Met, and an equal-dose combination of BT461-DB-EGFR and BT461-DB-c-Met diluted in cell complete medium were added. The initial concentration was 500 µg/mL, followed by 2-fold serial dilution to create 5 concentration gradients. After culturing in an incubator under conditions of 5% CO2 atmosphere and 37°C for 5 days, an equal volume of CellCounting-Lite^{®}3D reagent (Vazyme, Cat No. DD1102) was added to each well, mixed by shaking for 5 min until the cell clusters were completely lysed. After incubating for 25 min, 100 µL of the mixture was transferred to a white opaque plate and detected using a TR-FRET microplate reader (PerkinElmer, model, Cat No.).

The results are as shown in FIG. 10B, in which the efficacy of the antibody 461-DB of the present invention > combination > 461-DB-EGFR > 461-DB-c-Met, demonstrating that the anti-EGFR/c-Met dual-target antibody has a synergistic effect in inhibiting tumor cell proliferation *in vitro.*

### Example 13. BT461-DB Antibody Inhibits the Proliferation of Cell Strain with Acquired Resistance to Osimertinib In Vitro

### 13.1 Construction of the HCC827-HGF-OSI Osimertinib-Resistant Cell Strain

This example obtained the HCC827-HGF-OSI osimertinib-resistant cell strain through gradient dose escalation.

HCC827-HGF cells were thawed and cultured in 1640+10% FBS+1% double-antibiotic medium, stably passaged for 2-3 generations to stabilize cell properties.

Osimertinib induction: Initially, 100 nM osimertinib was administered, with continuous induction for 2 months; then 200 nM osimertinib was administered, with continuous induction for 1 month and cell proliferation level testing; followed by 400 nM osimertinib administration with continuous induction for 1 month and cell proliferation level testing; finally, 600 nM osimertinib was administered, with continuous induction for 1 month and cell proliferation level testing.

TKI resistance experiment:
(1) HCC827-HGF-OSI cell strain and non-resistant HCC827-HGF cells in the logarithmic growth phase were taken, digested, and resuspended in complete medium. The cell concentration was adjusted to 4E4/mL. A 96-well plate (3599, Corning) was used, and 50 µL of cell suspension was added to each well, allowing the cells and osimertinib to co-incubate in an incubator for 3 days;
(2) After 3 days, the cells were removed from the incubator and equilibrated to room temperature;
(3) The detection solution was thawed in advance from -20°C and equilibrated to room temperature. 100 µL of detection reagent was added to each well, mixed on a shaker at 300 rpm/min for 5 min, and then left at room temperature for 25 min;
(4) Detection: Using a multi-channel pipette, the liquid in each well was mixed by pipetting up and down, and 100 µL was taken from each well into a new OptiPlate standard non-bottom-reading microplate. The CTG mode was selected for reading.

The results, as shown in FIG. 11, demonstrating that HCC827-HGF-OSI acquired tolerance to osimertinib concentrations below 1 µM.

### 13.2 In Vitro Efficacy of BT461-DB on the HCC827-HGF-OSI Osimertinib-Resistant Cell Strain

The test method was similar to Example 11. Tumor cells in the logarithmic growth phase were taken, digested, and seeded into a 96-well plate (2000 cells/well), and cultured in complete medium overnight. After removing the medium from the 96-well plate, 100 µL of anti-c-Met/EGFR antibodies BT461-DB, BT461-KIH, and control IgG1 (initial concentration 500 µg/mL) diluted in cell complete medium were added and serially diluted 2-fold to create 5 concentration gradients. After culturing in an incubator under conditions of 5% CO2 atmosphere and 37°C for 5 days, an equal volume of CellCounting-Lite^{®}3D reagent (Vazyme, Cat No. DD1102) was added to each well, mixed by shaking for 5 min until the cell clusters were completely lysed. After incubating for 25 min, 100 µL of the mixture was transferred to a white opaque plate and detected using a TR-FRET microplate reader (PerkinElmer, model, Cat No.).

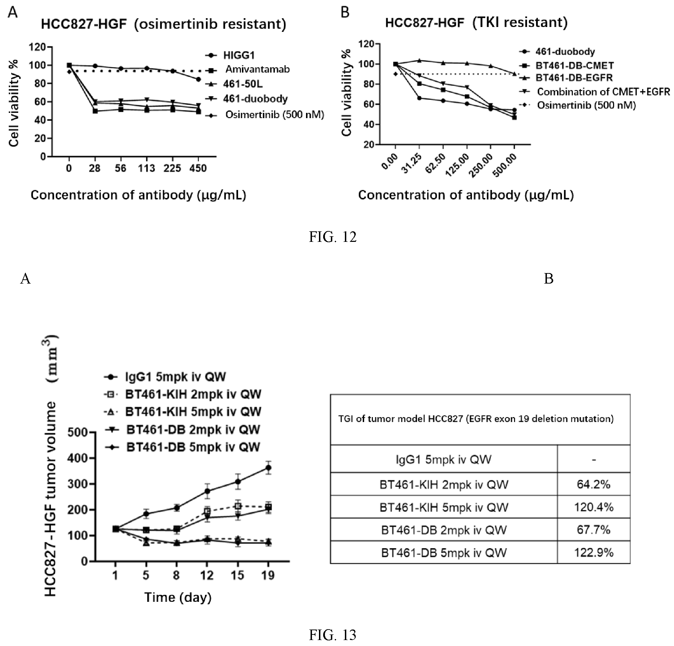

The results, as shown in FIG. 12A, demonstrating that the antibody BT461-DB of the present invention can significantly inhibit the proliferation of tumor cells HCC827-HGF-osimertinib-resistant cells at multiple gradient concentrations, and its inhibitory level on HCC827-HGF cell proliferation is comparable to that of BT461-KIH and amivantamab.

### 13.3 Evaluation of the Synergistic Effect of BT461-DB on Inhibiting the Proliferation of Osimertinib-Resistant Cells

The experimental method was the same as in Example 12. HCC827-HGF-OSI tumor cells in the logarithmic growth phase were taken, digested, and seeded into a 96-well plate (2000 cells/well), and cultured in complete medium overnight. After removing the medium from the 96-well plate, 100 µL of antibodies BT461-DB, BT461-DB-EGFR, BT461-DB-c-Met, and an equal-dose combination of BT461-DB-EGFR and BT461-DB-c-Met diluted in cell complete medium were added. The initial concentration was 500 µg/mL, followed by 2-fold serial dilution to create 5 concentration gradients. After culturing in an incubator under conditions of 5% CO2 atmosphere and 37°C for 5 days, an equal volume of CellCounting-Lite^{®}3D reagent (Vazyme, Cat No. DD1102) was added to each well, mixed by shaking for 5 min until the cell clusters were completely lysed. After incubating for 25 min, 100 µL of the mixture was transferred to a white opaque plate and detected using a TR-FRET microplate reader (PerkinElmer, model, Cat No.).

The results, as shown in FIG. 12B, in which the 461-EGFR monoclonal antibody has essentially no efficacy against osimertinib-resistant cells, while the efficacy of the antibody 461-DB of the present invention > 461-DB-c-Met > combination > 461-DB-EGFR, demonstrating that the anti-EGFR/c-Met dual-target antibody has a synergistic effect in inhibiting the proliferation of osimertinib-resistant tumor cells *in vitro.*

### Example 14. Research on the In Vivo Tumor Therapeutic Effects of the Bispecific Antibody Molecule

The *in vivo* anti-tumor activity of the anti-bispecific antibody was evaluated in a mouse xenograft model (NSG, Vital River, 6-week-old, female) of human non-small cell lung cancer HCC827-HGF cells.

Using RPMI1640+10% FBS as the complete medium, human HCC827-HGF cells were propagated *in vitro* as a monolayer culture. Cells in the logarithmic growth phase were digested and resuspended in serum-free medium/Matrigel (1:1) to obtain a suspension. Cells were inoculated subcutaneously (s.c.) on the right back of mice at 5×10⁶ cells per mouse for tumor formation. Ten days after implantation, mice were divided into 5 groups based on tumor volume, with an average tumor volume of approximately 80-100mm³ per group, and drug administering was initiated. IgG1 or antibody molecules were administered intravenously (I.V.) once a week for a total of three weeks.

The experimental results are as shown in FIG. 13. In the HCC827-HGF tumor formation model, the anti-tumor efficacy of BT461-DB and BT461-KIH was dose-dependent. At an administering dose of 2 mg/kg, the TGI (tumor growth inhibition ratio) of BT461-DB and BT461-KIH was 67.7% and 64.2%, respectively. At a administering dose of 5 mg/kg, the TGI (tumor growth inhibition ratio) of BT461-DB and BT461-KIH was 122.9% and 120.4%, respectively.

## Claims

1. A bispecific antibody comprising an antigen-binding arm A that specifically binds to human epidermal growth factor receptor (EGFR) and antagonizes the binding of epidermal growth factor (EGF) to EGFR, and an antigen-binding arm B that specifically binds to hepatocyte growth factor receptor (c-Met) and antagonizes the binding of hepatocyte growth factor (HGF) to c-Met, wherein the binding arm A comprises light chain A (LCA) and heavy chain A (HCA), and the binding arm B comprises light chain B (LCB) and heavy chain B (HCB), wherein the LCA, HCA, LCB and HCB comprise the amino acid sequences shown in SEQ ID NOs: 1, 2, 3 and 4, respectively.

2. The bispecific antibody according to claim 1, wherein the bispecific antibody has one or more of the following functions:
(1) being capable of inhibiting EGFR autophosphorylation mediated by ligand EGF and c-Met autophosphorylation mediated by ligand HGF;
(2) being capable of inhibiting growth and proliferation of EGFR and/or c-Met double-positive human tumor cells, wherein the effect of said inhibiting proliferation of EGFR and c-Met double-positive tumor cells is stronger than combination of EGFR monoclonal antibody and c-Met monoclonal antibody;
(3) being capable of mediating antibody-dependent cytotoxicity (ADCC);
(4) promoting internalization of target protein, and the internalization level mediated by the bispecific antibody is higher than the internalization level mediated by the c-Met specific binding arm B or EGFR specific binding arm A;
(5) being capable of inhibiting proliferation of cell strains with acquired resistance to osimertinib.

3. The bispecific antibody according to claim 1 or 2, wherein the bispecific antibody has a significantly higher affinity for c-Met than for EGFR.

4. The bispecific antibody according to claims 1-3, wherein the binding arm A comprises a light chain variable region A (VLA) and a heavy chain variable region A (VHA), and the binding arm B comprises a light chain variable region B (VLB) and a heavy chain variable region B (VHB), wherein the VHA and VLA of the binding arm A are derived from an IgG polypeptide A (IgG (A)) comprising a homodimeric structure of VHA and VLA, and the VHB and VLB of the binding arm B are derived from an IgG polypeptide B (IgG(B)) comprising a homodimeric structure of VHB and VLB; wherein the VHA comprises VHA CDR1, VHA CDR2, and VHA CDR3 of the amino acid sequences shown in SEQ ID NOs: 19, 20, and 21, respectively, the VLA comprises VLA CDR1, VLA CDR2, and VLA CDR3 of the amino acid sequences shown in SEQ ID NOs: 22, 23, and 24, respectively, the VHB comprises VHB CDR1, VHB CDR2, and VHB CDR3 of the amino acid sequences shown in SEQ ID NOs: 25, 26, and 27, respectively, and the VLB comprises VLB CDR1, VLB CDR2, and VLB CDR3 of the amino acid sequences shown in SEQ ID NOs: 28, 29, and 30, respectively.

5. The bispecific antibody according to claim 4, wherein the VHA, VLA, VHB and VLB comprise the amino acid sequences shown in SEQ ID NOs: 5, 6, 7 and 8, respectively.

6. The bispecific antibody according to claims 1-5, wherein the binding arm A further comprises a heavy chain constant region A (CHA) and a light chain constant region A (CLA), and the binding arm B further comprises a heavy chain constant region B (CHB) and a light chain constant region B (CLB), wherein the CHA and the CHB are both IgG1 isotype heavy chain constant regions.

7. The bispecific antibody according to claims 1-6, wherein the antigen CHA comprises an Fc(A) region, the Fc(A) region comprises a CH3(A) region; the CHB comprises an Fc(B) region, the Fc(B) region comprises a CH3(B) region, the CH3(A) region and the CH3(B) region can form a heterodimer through interaction, the heterodimeric interaction is stronger than each of the homodimeric interaction of the CH3(A) region and the CH3(B) region, or optionally, wherein the CH3(A) of antigen binding arm A and the CH3(B) of antigen binding arm B both comprise amino acid mutations that enhance and promote the formation of heterodimer;
or optionally, wherein the CH3(A) comprises an amino acid mutation as shown in F405L, and the CH3 (B) comprises an amino acid mutation as shown in K409R; or further optionally, both the Fc(A) and the Fc(B) comprise a combination of amino acid mutations that reduce immunogenicity.

8. The bispecific antibody according to claims 1-7, wherein the Fc(A) comprises the amino acid sequence shown in SEQ ID NO: 9 and the Fc(B) comprises the amino acid sequence shown in SEQ ID NO: 10.

9. The bispecific antibody according to claims 1-8, wherein the CHA, CLA, CHB and CLB comprise the amino acid sequences as shown SEQ ID NOs: 11, 12, 13 and 14, respectively.

10. A composition of encoding nucleic acid comprising the polynucleotide sequences as shown in SEQ ID NOs: 15, 16, 17 and 18 capable of respectively encoding HCA, HCB, LCA and LCB of the bispecific antibody according claim 1.

11. A pharmaceutical composition comprising the bispecific antibody according to any one of claims 1-8, and a pharmaceutically acceptable carrier or diluent.

12. The pharmaceutical composition according to claim 11, wherein the pharmaceutical composition further comprises a second therapeutic agent or therapeutic regimen, the second therapeutic agent or therapeutic regimen comprises chemotherapeutic drugs, DNA alkylating agents, immunomodulators, proteasome inhibitors, histone deacetylase inhibitors, radiation therapy, stem cell transplantation, variant bispecific antibodies interacting with various tumor cell surface antigens and T cell or immune cell antigens, antibody drug conjugates, bispecific antibodies conjugated to an antitumor agent, PD-1, PD-L1 or CTLA-4 checkpoint inhibitors or a combination thereof.

13. Use of the bispecific antibody according to claims 1-9 or the pharmaceutical composition according to claim 11 or 12 in the manufacture of a therapeutic medicament used for the treatment of a disease or tumor involving EGFR and c-Met abnormal cells and a therapeutic medicament used for inhibiting the growth or metastasis of tumor or cancer cells expressing EGFR and/or c-Met in a patient;
wherein the EGFR and c-Met abnormal cells comprise EGFR activating mutation, EGFR gene amplification, up-regulation of HGF expression level, c-Met activating mutation, c-Met gene amplification, or KRAS mutant association.

14. Use of the bispecific antibody according to claims 1-9 or the pharmaceutical composition according to claim 11 or 12 in the manufacture of a medicament used for the treatment of a disease resistant to osimertinib.

15. The use according to claim 13 or 14, wherein the tumor or cancer is one of epithelial cell cancer, breast cancer, ovarian cancer, lung adenocarcinoma, small cell lung cancer, colorectal cancer, anal cancer, prostate cancer, bladder cancer, pharyngeal cancer, nasal cancer, pancreatic cancer, skin cancer, tongue cancer, esophageal cancer, vaginal cancer, cervical cancer, splenic cancer, testicular cancer, gastric cancer, thymic cancer, thyroid cancer, hepatocellular carcinoma, or sporadic or hereditary papillary renal cell carcinoma; or optionally, the cancer is one of non-small cell lung cancer, pancreatic cancer, gastric cancer, colon cancer, and liver cancer.
